# EUROPEAN PATENT SPECIFICATION

(11) **EP 2 970 487 B1**
(45) Date of publication and mention of the grant of the patent: **11.03.2020**
(21) Application number: 14726015.2
(22) Date of filing: 11.03.2014
(51) Int. Cl.: A61K 38/00, C07K 16/40, C07K 14/245, C07K 16/28, C07K 14/25, A61K 39/00, C07K 16/08, C07K 16/20, C07K 16/30, C07K 16/32

(54) **CYTOTOXIC PROTEINS COMPRISING CELL-TARGETING BINDING REGIONS AND SHIGA TOXIN A SUBUNIT REGIONS FOR SELECTIVE KILLING OF SPECIFIC CELL TYPES**
ZYTOTOXISCHE PROTEINE MIT ZELLGERICHTETEN BINDUNGSREGIONEN UND SHIGATOXIN-A-UNTEREINHEITSREGIONEN ZUR SELEKTIVEN TÖTUNG VON SPEZIFISCHEN ZELLARTEN
PROTÉINES CYTOTOXIQUES COMPRENANT DES RÉGIONS LIANTES CIBLANT DES CELLULES ET DES RÉGIONS SOUS-UNITÉS DE LA SHIGA-TOXINE A DESTINÉES À ÉLIMINER SÉLECTIVEMENT DES TYPES DE CELLULES SPÉCIFIQUES

(30) Priority: 12.03.2013 US 201361777130 P
(43) Date of publication of application: 20.01.2016
(60) Divisional application: 20154062.2
(73) Proprietor: Molecular Templates, Inc., Austin, TX 78729 (US)
(72) Inventor: POMA, Eric, New York New York 10014 (US); WILLERT, Erin, Round Rock Texas 78681 (US); KIM, Jason, Austin Texas 78759 (US); HIGGINS, Jack, Georgetown Texas 78628 (US)
(74) Representative: Griffin, Philippa Jane
(86) International application number: PCT/US2014/023231
(87) International publication number: WO 2014/164693

(56) References cited:
- WO-A1-2016/196344
- DANIEL A VALLERA ET AL: "Bioengineering a Unique Deimmunized Bispecific Targeted Toxin That Simultaneously Recognizes Human CD22 and CD19 Receptors in a Mouse Model of B-Cell Metastases", MOLECULAR CANCER THERAPEUTICS, AMERICAN ASSOCIATION OF CANCER RESEARCH, US , vol. 9, no. 6 9 June 2010 (2010-06-09), pages 1872-1883, XP002679892, ISSN: 1535-7163, DOI: 10.1158/1535-7163.MCT-10-0203 Retrieved from the Internet: URL:http://mct.aacrjournals.org/content/9/ 6/1872 [retrieved on 2010-06-08]
- Cizeau et al: "8th Fabisch-Symposium, 3rd Targeted Tumor Therapies, Berlin 2012", , 21 March 2012 (2012-03-21), XP055140853, Retrieved from the Internet: URL:http://www.charite.de/fabisch/Abstract sText.html [retrieved on 2014-09-17]
- Maria L. Torgersen ET AL: "The A-subunit of surface-bound Shiga toxin stimulates clathrin-dependent uptake of the toxin : Endocytosis of Shiga toxin", FEBS JOURNAL, vol. 272, no. 16, 3 August 2005 (2005-08-03), pages 4103-4113, XP055379368, GB ISSN: 1742-464X, DOI: 10.1111/j.1742-4658.2005.04835.x
- GLENNIE ET AL: "Mechanisms of killing by anti-CD20 monoclonal antibodies", MOLECULAR IMMUNOLOGY, PERGAMON, GB, vol. 44, no. 16, 1 September 2007 (2007-09-01), pages 3823-3837, XP022227562, ISSN: 0161-5890, DOI: 10.1016/J.MOLIMM.2007.06.151
- HADDAD J E ET AL: "Minimum Domain of the Shiga Toxin A Subunit Required for Enzymatic Activity", J. BACTERIO,, vol. 175, no. 16, 1 August 1993 (1993-08-01), pages 4970-4978, XP008109062,
- LAPOINTE P ET AL: "A role for the protease-sensitive loop region of Shiga-like toxin 1 in the retrotranslocation of its A1 domain from the endoplasmic reticulum lumen", JOURNAL OF BIOLOGICAL CHEMISTRY, AMERICAN SOCIETY FOR BIOCHEMISTRY AND MOLECULAR BIOLOGY, US, vol. 280, no. 24, 17 June 2005 (2005-06-17), pages 23310-23318, XP002537087, ISSN: 0021-9258, DOI: 10.1074/JBC.M414193200 [retrieved on 2005-04-07]
- Anonymous: "Abstract 1483: MT-3724, an engineered toxin body targeting CD20 for non-Hodgkin's lymphoma | Cancer Research", , 1 January 2016 (2016-01-01), XP055408114, Retrieved from the Internet: URL:http://cancerres.aacrjournals.org/cont ent/76/14_Supplement/1483 [retrieved on 2017-09-19]
- Jenna Mckenzie ET AL: "Passage through the Golgi is necessary for Shiga toxin B?subunit to reach the endoplasmic reticulum : Shiga toxin in the Golgi", FEBS JOURNAL, vol. 276, no. 6, 1 March 2009 (2009-03-01) , pages 1581-1595, XP055408115, GB ISSN: 1742-464X, DOI: 10.1111/j.1742-4658.2009.06890.x
- Barbara Windschiegl ET AL: "Lipid Reorganization Induced by Shiga Toxin Clustering on Planar Membranes", PLoS ONE, vol. 4, no. 7, 16 July 2009 (2009-07-16), page e6238, XP055408116, DOI: 10.1371/journal.pone.0006238
- M. V. Bujny ET AL: "The retromer component sorting nexin-1 is required for efficient retrograde transport of Shiga toxin from early endosome to the trans Golgi network", JOURNAL OF CELL SCIENCE, vol. 120, no. 12, 22 May 2007 (2007-05-22) , pages 2010-2021, XP055408117, GB ISSN: 0021-9533, DOI: 10.1242/jcs.003111
- Y. Shiba ET AL: "AGAP2 regulates retrograde transport between early endosomes and the TGN", Journal of Cell Science, vol. 123, no. 14, 15 June 2010 (2010-06-15), pages 2381-2390, XP055119261, ISSN: 0021-9533, DOI: 10.1242/jcs.057778
- M. Maak ET AL: "Tumor-Specific Targeting of Pancreatic Cancer with Shiga Toxin B-Subunit", MOLECULAR CANCER THERAPEUTICS, vol. 10, no. 10, 1 October 2011 (2011-10-01), pages 1918-1928, XP055408119, US ISSN: 1535-7163, DOI: 10.1158/1535-7163.MCT-11-0006
- Frédéric Mallard ET AL: "Direct Pathway from Early/Recycling Endosomes to the Golgi Apparatus Revealed through the Study of Shiga Toxin B-fragment Transport", The journal of cell biology : JCB, vol. 143, no. 4, 16 November 1998 (1998-11-16), pages 973-990, XP055408120, US ISSN: 0021-9525, DOI: 10.1083/jcb.143.4.973
- BOLOGNESI A ET AL: "A COMPARISON OF ANTI-LYMPHOCYTE IMMUNOTOXINS CONTAINING DIFFERENT RIBOSOME-INACTIVATING PROTEINS AND ANTIBODIES", CLINICAL AND EXPERIMENTAL IMMUNOLOGY, WILEY-BLACKWELL PUBLISHING LTD, GB, vol. 89, 1 January 1992 (1992-01-01), pages 341-346, XP000606023, ISSN: 0009-9104
- C. M. Pirie ET AL: "Convergent Potency of Internalized Gelonin Immunotoxins across Varied Cell Lines, Antigens, and Targeting Moieties", Journal of Biological Chemistry, vol. 286, no. 6, 7 December 2010 (2010-12-07), pages 4165-4172, XP055088620, ISSN: 0021-9258, DOI: 10.1074/jbc.M110.186973

## Description

### FIELD OF THE INVENTION

The present invention relates to cytotoxic proteins comprising immunoglobulin-type binding regions and Shiga toxin effector regions derived from A Subunits of members of the Shiga toxin family, as defined in the claims. The cytotoxic proteins of this invention have uses for the selective killing of specific cell types and as therapeutics for the treatment of a variety of diseases, including cancers, immune disorders, and microbial infections.

### BACKGROUND

Synthetically engineered compositions based on naturally occurring toxins have been designed in attempts to create new therapeutics exhibiting targeted cytotoxicity after administration to a patient (Pastan I, et al., Annu Rev Med 58: 221-37 (2007)). Naturally occurring toxins or truncated versions have been fused to immunoglobulin domains or receptor ligands by recombinant protein engineering (Chaudhary V et al., Nature 339: 394-97 (1989); Strom T et al., Semin Immunol 2: 467-79 (1990); Pastan I et al., Annu Rev Biochem 61: 331-54 (1992); Foss F et al., Curr Top Microbiol Immunol 234: 63-81 (1998)). The aim of such molecular engineering is to design chimeric molecules with the dual functionality of: 1) delivering toxins to specific cell types or places within an organism after systemic administration and 2) effectuating a targeted cytotoxicity to specific cells using potent cytotoxicity mechanisms effective in eukaryotic cells.

The Shiga toxin family of related protein toxins, notably toxins isolated from *S*. *dysenteriae* and *E. coli,* is composed of various naturally occurring toxins that are structurally and functionally related (Johannes L, Römer W, Nat Rev Microbiol 8: 105-16 (2010)). For example, the Shiga toxin family encompasses true Shiga toxin (Stx) isolated from *S*. *dysenteriae* serotype 1, Shiga-like toxin 1 variants (SLT1 or Stx1 or SLT-1 or Slt-I) isolated from serotypes of enterohemorrhagic *E. coli,* and Shiga-like toxin 2 variants (SLT2 or Stx2 or SLT-2) isolated from serotypes of enterohemorrhagic *E. coli.* SLT1 differs by only one residue from Stx, and both have been referred to as Verocytotoxins or Verotoxins (VTs) (O'Brien A et al., Curr Top Microbiol Immunol 180: 65-94 (1992)). Members of the Shiga toxin family share the same overall structure and mechanism of action (Engedal, N et al., Microbial Biotech 4: 32-46 (2011)). Members of the Shiga toxin family contain targeting domains that preferentially bind a specific glycosphingolipid present on the surface of some host cells and an enzymatic domain capable of permanently inactivating ribosomes once inside a cell (Johannes, Nat Rev Microbiol 8: 105-16 (2010)).

Members of the Shiga toxin family are employed by bacteria as virulence factors during infection of a host (Johannes, Nat Rev Microbiol 8: 105-16 (2010)). In an infected host, Shiga toxins are cytotoxic because of the toxins' potent ability to inhibit protein synthesis and to trigger apoptotic cell death (Johannes, Nat Rev Microbiol 8: 105-16 (2010)). The potent cytotoxic effects of Shiga toxins on host cells can result in hemorrhagic colitis and hemolytic uremic syndrome (Johannes, Nat Rev Microbiol 8: 105-16 (2010)).

Members of the Shiga toxin family share a common, multimeric, protein structure characterized by an A(B)₅ arrangement of Shiga protein subunits (Johannes, Nat Rev Microbiol 8: 105-16 (2010)). Each Shiga toxin is composed of two protein subunits, A and B, that associate in an A(B)₅ arrangement to form a holotoxin protein complex. The Shiga toxin A Subunit is a 32 kilodalton monomer that contains an enzymatic domain, and the Shiga toxin B Subunit is a 7.7 kilodalton subunit that associates with four other Shiga toxin B Subunits to form a pentamer of Shiga toxin B Subunits. The pentamer of B subunits associates with one A subunit to form the Shiga holotoxin, which is about 70 kilodaltons (O'Brien A, Holmes, R, Microbiol Rev 51: 206-20 (1987)).

Members of the Shiga toxin family share a common process for the intoxication of a host cell that can be divided into five main phases: cell surface binding, endocytosis, retrograde subcellular movement to the endoplasmic reticulum, translocation from the endoplasmic reticulum to the cytosol, and the enzymatic inactivation of ribosomes in the cytosol. First, Shiga holotoxins are directed to the cellular surfaces of specific host cells by the B subunit's ability to specifically bind the glycosphingolipid globotriaosylceramide Gb3, also known as CD77, present on the exoplasmic membrane leaflet (Ling, H et al, Biochemistry 37: 1777-88 (1998); Thorpe C et al., Infect Immun 67: 5985-93 (1999); Soltyk A et al., J Biol Chem 277: 5351-59 (2002)). Second, Shiga holotoxins exploit the host cell's endocytotic machinery to enter into the host cell, where the holotoxins are initially contained within the endosomes (Sandvig K et al., J Cell Biol 108: 1331-43 (1989); Sandvig K et al., Histochem Cell Biol 117: 131-141 (2002)). Third, Shiga holotoxins exploit the host cell's intracellular-transport machinery to reach the endoplasmic reticulum and gain access to the cytosol (Nichols B et al., J Cell Biol 153: 529-41 (2001); Lauvrak S et al., J Cell Sci 117: 2321-31 (2004); Saint-Pol A et al., Dev. Cell 6: 525-38 (2004)). Fourth, enzymatically active fragments of the Shiga holotoxins retrotranslocate from the lumen of the endoplasmic reticulum to the cytosol (Yu M, Haslam D, Infect Immun 73: 2524-32 (2005); LaPointe P et al., J Biol Chem 280: 23310-18 (2005); Falguieres T, Johannes L, Biol Cell 98: 125-34 (2006); Tam P, Lingwood C, Microbiology 153: 2700-10 (2007)). Fifth, the cytosolic fraction of Shiga toxin enzymatically active fragments causes cytotoxicity by inactivating host-cell ribosomes (Tam, Microbiology 153: 2700-10 (2007)).

During the Shiga toxin intoxication process, the Shiga toxin A Subunit is proteolytically cleaved between a conserved arginine residue and a methionine residue (*e.g*. Arg251-Met252 in StxA) by furin, a host cell endoprotease (Garred Ø et al., J Biol Chem 270: 10817-21 (1995)). The amino-terminal fragment of the furin-cleaved, Shiga-toxin A Subunit is called the Shiga toxin "A1 fragment" (or Stxn-A1, SLTn-A1, SLT-nA1). The Shiga toxin A1 fragment is a 28 kilodalton protein that contains the catalytic domain of the Shiga toxin (Fraser M et al., Nat Struct Biol 1: 59-64 (1994)). The mechanism of Shiga toxin cytotoxicity to host cells is predominantly through the A1 fragment's potent catalytic inactivation of eukaryotic ribosomes and cell-wide inhibition of protein synthesis (Johannes, Nat Rev Microbiol 8: 105-16 (2010)).

The Shiga toxin A1 fragment inhibits protein translation by its potent depurination activity towards a universally conserved adenine nucleobase at position 4,324 in the alpha-sarcin-ricin loop of the 28S ribosomal RNA of the eukaryotic ribosome (Johannes, Nat Rev Microbiol 8: 105-16 (2010)). After a threshold number of ribosomes is inactivated, the host cell is predicted to experience sufficient reduction in protein synthesis to induce cell death via apoptosis (Iordanov M et al., Mol Cell Biol 17: 3373-81 (1997); Smith W et al., Infect Immun 71: 1497-504 (2003); Lee S et al., Cell Microbiol 10: 770-80 (2008); Tesh V, Future Microbiol 5: 431-53 (2010)).

A ribosome-inactivating A-B toxin can permanently cripple one ribosome after another within the same cell at a rate of approximately 1,500 ribosomes per minute (Endo Y, Tsurugi K, Eur J Biochem 171: 45-50 (1988); Endo Y et al., J Biol Chem 263: 8735-9 (1988)). The potency of A-B toxins is reported to be extremely high, such that as little as one toxin molecule can kill a cell (Yamaizumi M et al., Cell 15: 245-50 (1978); Antignani A, Fitzgerald D, Toxins 5: 1486-502 (2013)). It is believed that a single molecule of A-B toxin can irreversibly inactivate 300 ribosomes in 35 minutes and is sufficient to kill a cancer cell (Weldon J, Pastan I, FEBS Journal 278: 4683-700 (2011)). This level of cytotoxic potency is further predicted for Shiga toxin A Subunit, for which it has been suggested that one molecule translocated into the cytosol would be sufficient to kill a cell (Tam, Microbiology 153: 2700-10 (2007)).

Holotoxins of the Shiga toxin family are predicted to be too toxic for untargeted use as a therapeutic (Jain, R, Tumor physiology and antibody delivery, Front Radiat Ther Oncol 24: 32-46 (1990)). However, members of the Shiga toxin family have the potential to be synthetically engineered for therapeutic applications by rational alterations to the toxin's structure, characteristics, and biological activities (Johannes, Nat Rev Microbiol 8: 105-16 (2010); Engedal, Microbial Biotech 4: 32-46 (2011)). Shiga holotoxins have a bipartite structure composed of two non-covalently attached, modular parts: an A-moiety containing the enzymatically active A1 fragment and a B-moiety containing binding sites to the cell-surface target Gb3. Because the Shiga toxin subunits are modular, it has been hypothesized that therapeutic compositions may be created based on the separate structures and functions of the A and B moieties (U.S. application 20090156417 A1; Johannes, Nat Rev Microbiol 8: 105-16 (2010); Engedal, Microbial Biotech 4: 32-46 (2011); E.P. application 2402367 A1; U.S. application 20130196928 A1).

The A-moiety of members of the Shiga toxin family is stable, enzymatically active, and cytotoxic independent of any B-moiety (Engedal, Microbial Biotech 4: 32-46 (2011)). The Shiga toxin 1 A Subunit is catalytically active, capable of enzymatically inactivating ribosomes *in vitro,* and cytotoxic even if truncated or fused to other protein domains (Haddad J et al., J Bacteriol 175: 4970-8 (1993); Al-Jaufy A et al., Infect Immun 62: 956-60 (1994); Al-Jaufy A et al., Infect Immun 63: 3073-8 (1995); LaPointe, J Biol Chem 280: 23310-18 (2005); Di R et al., Toxicon 57: 535-39 (2011)). Shiga-like toxin 1 A Subunit truncations are catalytically active, capable of enzymatically inactivating ribosomes *in vitro,* and cytotoxic when expressed within a cell (LaPointe, J Biol Chem 280: 23310-18 (2005)).

The idea of linking a toxin to a targeting domain to make a chimeric molecule that selectively kills cancer cells is not new (Strebhardt K, Ullrich A, Nat Rev Cancer 8: 473-80 (2008)). For example, since the 1970s immunotoxins have been developed using three, primary, toxin candidates: the bacterial diphtheria toxin, the bacterial *Pseudomonas* exotoxin, and plant toxins exemplified by ricin (Antignani, Toxins 5: 1486-502 (2013)). By 2013, however, these three toxins remained "among the top choices for immunotoxin development" (Antignani, Toxins 5: 1486-502 (2013)). To date, no one has described a cytotoxic protein comprising an amino acid sequence derived from a Shiga-toxin combined with a cell-targeting, immunoglobulin-derived binding region that was capable of specifically and selectively killing a targeted cell type in a vertebrate or even a laboratory, cell-culture environment. It would be desirable to have cytotoxic proteins comprising Shiga toxin regions capable of targeted cell killing for the development of effective therapeutic molecules and for use as therapeutic molecules for the treatment of a variety of diseases. Vallera et al. (2010) describes bioengineering of a unique de-immunized bispecific targeted toxin that simultaneously recognizes human CD22 and CD19 receptors in a mouse model of B-cell metastases. Cizeau et al. (2012) describes engineering and biologic characterization of deBouganin linked with anti-EpCAM Fab fragment, and its application in oncology.

### SUMMARY OF THE INVENTION

The present invention provides various cytotoxic proteins for the selective killing of specific cell types, the proteins comprising 1) immunoglobulin-type binding regions, as defined in the claims, such as from immunoglobulins, and 2) Shiga toxin effector regions, such as truncations of SLT-1A, as defined in the claims. The linking of binding regions with Shiga-toxin-Subunit-A-derived polypeptides enabled the engineering of cytotoxic Shiga-toxin based molecules capable of being targeted to specific cell-types. The cytotoxic proteins of the invention have uses for targeted cell-killing and as therapeutics for the treatment of a variety of diseases, disorders, and conditions, including cancers, immune disorders, and microbial infections.

A cytotoxic protein of the invention comprises (a) an immunoglobulin-type binding region comprising one or more polypeptides and capable of specifically binding at least one extracellular target biomolecule, as defined in the claims, and (b) a Shiga toxin effector region comprising a polypeptide derived from the amino acid sequence of the A Subunit of at least one member of the Shiga toxin family, as defined in the claims.

For certain embodiments of the cytotoxic proteins of the present invention, the immunoglobulin-type binding region comprises a polypeptide selected from the group consisting of a: single-domain antibody fragment, single-chain variable fragment, antibody variable fragment, antigen-binding fragment, and Fd fragment.

As defined in the claims, upon administration of the cytotoxic protein of the invention to a cell physically coupled with an extracellular target biomolecule of the immunoglobulin-type binding region of the cytotoxic protein, the cytotoxic protein is capable of causing death of the cell.

In certain embodiments, upon administration of the cytotoxic protein to two different populations of cell types with respect to the presence of an extracellular target biomolecule, the cytotoxic protein is capable of causing cell death to the cell-types physically coupled with an extracellular target biomolecule of its immunoglobulin-type region at a CD₅₀ at least three times or less than the CD₅₀ to cell types which are not physically coupled with an extracellular target biomolecule of its immunoglobulin-type binding region.

In certain embodiments, the immunoglobulin-type binding region is designed or selected by its ability to bind an extracellular target biomolecule selected from the group consisting of: CD20, CD22, CD40, CD79, CD25, CD30, HER2/neu/ErbB2, EGFR, EpCAM, EphB2, prostate-specific membrane antigen, Cripto, endoglin, fibroblast activation protein, Lewis-Y, CD19, CD21, CS1/ SLAMF7, CD33, CD52, , gpA33, Mucins, TAG-72, carbonic anhydrase IX, folate binding protein, ganglioside GD2, ganglioside GD3, ganglioside GM2, ganglioside Lewis-Y2, VEGFR, Alpha Vbeta3, Alpha5beta1, ErbB1/EGFR, Erb3, c-MET, IGF1R, EphA3, TRAIL-R1, TRAIL-R2, RANKL, Tenascin, CD64, and mesothelin, BRCA1, MART-1/MelanA, gp100, tyrosinase, TRP-1, TRP-2, MAGE-1, MAGE-3, GAGE-1/2, BAGE, RAGE, NY-ESO-1, CDK-4, beta-catenin, MUM-1, caspase-8, KIAA0205, HPVE6, SART-1, PRAME, carcinoembryonic antigen, prostate specific antigen, prostate stem cell antigen, human aspartyl (asparaginyl) beta-hydroxylase, EphA2, HER3/ErbB-3, MUC1, tyrosinase associated antigen, HPV-E7, Epstein-Barr Virus antigens, Bcr-Abl, alpha-fetoprotein antigen, 17-A1, bladder tumor antigen, CD38, CD15, CD23, CD52, CD53, CD88, CD129, CD183, CD191, CD193, CD244, CD294, CD305; C3AR, FceRIa, galectin-9, mrp-14, siglec-8, siglec-10, CD49d, CD13, Cd44, Cd54, CD63, CD69, CD123, CD193, TLR4, IgE, CD107a, CD203c, CD14, CD15, CD33, CD64, CD68, CD80, CD86, CD105, CD115, F4/80, ILT-3, Galectin-3, CD11a-c, GITRL, MHC Class II, CD284-TLR4, CD107-Mac3, CD195-CCR5, HLA-DR, CD16/32, CD282-TLR2, CD11c, CD123, and any immunogenic fragment of any of the foregoing.

For certain embodiments, the cytotoxic proteins comprise the Shiga toxin effector region comprising or consisting of amino acids 75 to 251 of SEQ ID NO:1, SEQ ID NO:2, or SEQ ID NO:3. Further embodiments are cytotoxic proteins in which the Shiga toxin effector region comprises or consists of amino acids 1 to 241 SEQ ID NO:1, SEQ ID NO:2, or SEQ ID NO:3; amino acids 1 to 251 of SEQ ID NO:1, SEQ ID NO:2, or SEQ ID NO:3; and/or amino acids 1 to 261 of SEQ ID NO:1, SEQ ID NO:2, or SEQ ID NO:3.

In certain embodiments, the cytotoxic proteins comprise the immunoglobulin-type binding region comprising or consisting of the amino acids 269-508 of SEQ ID NO:4. Further embodiments are the cytotoxic proteins comprising or consisting of SEQ ID NO:4.

In certain embodiments, the cytotoxic proteins comprise the immunoglobulin-type binding region comprising or consisting of amino acids 1-119 of SEQ ID NO:5. Further embodiments are the cytotoxic proteins comprising or consisting of SEQ ID NO:5.

In certain embodiments, the cytotoxic proteins comprise Shiga toxin effector regions which comprise a mutation relative to a naturally occurring A Subunit of a member of the Shiga toxin family which changes the enzymatic activity of the Shiga toxin effector region while retaining a cytotoxic activity, the mutation selected from at least one amino acid residue deletion or substitution, as defined in the claims.

The invention also includes pharmaceutical compositions comprising a cytotoxic protein of the invention and at least one pharmaceutically acceptable excipient or carrier, as defined in the claims; and the use of such a cytotoxic protein in methods of the invention as further described herein, and as defined in the claims.

Beyond the cytotoxic proteins of the present invention, the polynucleotides which are capable of encoding a cytotoxic protein of the invention are within the scope of the present invention, as well as expression vectors which comprise a polynucleotide of the invention and transformed host cells comprising an expression vector of the invention, as defined in the claims.

Additionally, the present invention provides *in vitro* methods of killing cell(s) comprising the step of contacting a cell(s) with a cytotoxic protein of the invention, as defined in the claims

Also, the present invention provides for treating diseases, disorders, and conditions in a patient in need thereof using a therapeutically effective amount of a cytotoxic protein or a pharmaceutical composition of the invention, as defined in the claims. In certain embodiments, the disease, disorder, or condition is selected from any one of the following: a cancer, tumor, immune disorder, or microbial infection. In certain embodiments, the cancer to be treated is selected from the group consisting of: bone cancer, breast cancer, central/peripheral nervous system cancer, gastrointestinal cancer, germ cell cancer, glandular cancer, head-neck cancer, hematological cancer, kidney-urinary tract cancer, liver cancer, lung/pleura cancer, prostate cancer, sarcoma, skin cancer, and uterine cancer. In certain embodiments, the immune disorder to be treated is an immune disorder associated with a disease selected from the group consisting of: amyloidosis, ankylosing spondylitis, asthma, Crohn's disease, diabetes, graft rejection, graft versus host disease, Hashimoto's thyroiditis, hemolytic uremic syndrome, HIV-related disease, lupus erythematosis, multiple sclerosis, polyarteritis, psoriasis, psoriatic arthritis, rheumatoid arthritis, scleroderma, septic shock, Sjögren's syndrome, ulcerative colitis, and vasculitis.

Among certain embodiments of the present invention is a cytotoxic protein or a pharmaceutical composition comprising said protein for use in the treatment or prevention of a cancer, immune disorder, or microbial infection, as defined in the claims.

These and other features, aspects and advantages of the present invention will become better understood with regard to the following description, appended claims, and accompanying figures. The aforementioned elements of the invention could be combined or removed freely in order to make other embodiments of the invention within the scope of the claims, without any statement to object to such combination or removal hereinafter.

### BRIEF DESCRIPTION OF THE DRAWINGS AND FIGURES

**Figure 1** shows the general architecture of exemplary cytotoxic proteins.
**Figure 2** graphically shows "αCD38-scFv fused with SLT-1A" was specifically cytotoxic to CD38+ cells as compared to CD38- cells. The percent viability of cells was plotted over the logarithm to base 10 of the cytotoxic protein concentration.
**Figure 3** graphically shows "αHER2-V_{HH} fused with SLT-1A" was specifically cytotoxic to HER2+ cells as compared to HER2- cells. The percent viability of cells was plotted over the logarithm to base 10 of the cytotoxic protein concentration.
**Figure 4** graphically shows the change in total body luminescence with the administration of αCD20scFv fused with SLT-1A versions 1 and 2 in a disseminated Raji-luc xenograft model.
**Figure 5** graphically shows the increased survival of Raji-luc xenograft model mice with the administration of αCD20scFv fused with SLT-1A versions 1 and 2.
**Figure 6** graphically shows the change in tumor volume with the administration of αCD20scFv fused with SLT-1A versions 1 and 2 in a Raji subcutaneous xenograft model.
**Figure 7** shows B-cell depletion in a non-human primate study with the administration of αCD20scFv::SLT-1A version 1. Specifically, the subset of CD20+ B-cells that expressed CD21 were analyzed.
**Figure 8** shows B-cell depletion in a non-human primate study with the administration of αCD20scFv::SLT-1A version 1. Specifically, the subset of CD20+ B cells that did not express CD21 were analyzed.

### DETAILED DESCRIPTION

The present invention is described more fully hereinafter using illustrative, non-limiting embodiments, and references to the accompanying figures. This invention may, however, be embodied in many different forms within the scope of the invention and should not be construed as to be limited to the embodiments set forth below. Rather, these embodiments are provided so that this disclosure is thorough and conveys the scope of the invention to those skilled in the art.

In order that the present invention may be more readily understood, certain terms are defined below. Additional definitions may be found within the detailed description of the invention.

As used in the specification and the appended claims, the terms "a," "an" and "the" include both singular and the plural referents unless the context clearly dictates otherwise.

As used in the specification and the appended claims, the term "and/or" when referring to two species, A and B, means at least one of A and B. As used in the specification and the appended claims, the term "and/or" when referring to greater than two species, such as A, B, and C, means at least one of A, B, or C, or at least one of any combination of A, B, or C (with each species in singular or multiple possibility).

Throughout this specification, the word "comprise" or variations such as "comprises" or "comprising" will be understood to imply the inclusion of a stated integer (or components) or group of integers (or components), but not the exclusion of any other integer (or components) or group of integers (or components).

Throughout this specification, the term "including" is used to mean "including but not limited to." "Including" and "including but not limited to" are used interchangeably.

The term "amino acid residue" or "amino acid" includes reference to an amino acid that is incorporated into a protein, polypeptide, or peptide. The term "polypeptide" includes any polymer of amino acids or amino acid residues. The term "polypeptide sequence" refers to a series of amino acids or amino acid residues which physically comprise a polypeptide. A "protein" is a macromolecule comprising one or more polypeptides chains. A "peptide" is a small polypeptide of sizes less than a total of 15-20 amino acid residues.

The terms "amino acid," "amino acid residue" or polypeptide sequence includes naturally occurring amino acids and, unless otherwise limited, also includes known analogs of natural amino acids that can function in a similar manner as naturally occurring amino acids. The amino acids referred to herein are described by shorthand designations as follows in Table A:

**TABLE A. Amino Acid Nomenclature**

| **Name** | **3-letter** | **1-letter** |
|---|---|---|
| Alanine | Ala | A |
| Arginine | Arg | R |
| Asparagine | Asn | N |
| Aspartic Acid or Aspartate | Asp | D |
| Cysteine | Cys | C |
| Glutamic Acid or Glutamate | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The phrase "conservative substitution" with regard to a polypeptide, refers to a change in the amino acid composition of the polypeptide that does not substantially alter the function and structure of the overall polypeptide (*see* Creighton, Proteins: Structures and Molecular Properties (W. H. Freeman and Company, New York (2nd ed., 1992)).

As used herein, the terms "expressed," "expressing" or "expresses" refers to translation of a polynucleotide or nucleic acid into a polypeptide or protein. The expressed polypeptide or proteins may remain intracellular, become a component of the cell surface membrane or be secreted into an extracellular space.

As used herein, the symbol "α" is shorthand for an immunoglobulin-type binding region is capable of binding to the biomolecule following the symbol. The symbol "α" is used to refer to the functional characteristic of an immunoglobulin-type binding region based on its capability of binding to the biomolecule following the symbol.

The term "selective cytotoxicity" with regard to the cytotoxic activity of a cytotoxic protein refers to the relative levels of cytotoxicity between a targeted cell population and a non-targeted bystander cell population, which can be expressed as a ratio of the half-maximal cytotoxic concentration (CD₅₀) for a targeted cell type over the CD₅₀ for an untargeted cell type to show preferentiality of cell killing of the targeted cell type.

For purposes of the present invention, the term "effector" means providing a biological activity, such as cytotoxicity, biological signaling, enzymatic catalysis, subcellular routing, and/or intermolecular binding resulting in the recruitment of factors and/or allosteric effects.

For purposes of the present invention, the phrase "derived from" means the polypeptide region comprises amino acid sequences originally found in a protein and may now comprise additions, deletions, truncations, or other alterations form the original sequence such that overall function and structure is substantially conserved.

### I. The General Structure of the Cytotoxic Protein

The present invention provides various cytotoxic proteins for the selective killing of specific cell types, the proteins each comprising 1) an immunoglobulin-type binding region for cell targeting, as defined in the claims; and 2) a Shiga toxin effector region, as defined in the claims. The linking of cell targeting immunoglobulin-type binding regions with Shiga-toxin-Subunit-A-derived regions enables the engineering of cell-type specific targeting of the potent Shiga toxin cytotoxicity. Cytotoxic proteins of the invention as defined in the claims comprise Shiga toxin effector regions derived from the A Subunits of members of the Shiga toxin family linked to immunoglobulin-type binding regions which can bind specifically to at least one extracellular target biomolecule in physical association with a cell, such as a target biomolecule expressed on the surface of a cell. This general structure is modular in that any number of diverse immunoglobulin-type binding regions may be linked to Shiga-toxin-Subunit-A derived effector regions to produce variations of the same general structure.

### A. Immunoglobulin-Type Binding Regions

Cytotoxic proteins of the invention comprise an immunoglobulin-type binding region comprising one or more polypeptides capable of selectively and specifically binding an extracellular target biomolecule, as defined in the claims. The term "immunoglobulin-type binding region" as used herein refers to a polypeptide region capable of binding one or more target biomolecules, such as an antigen or epitope. Immunoglobulin-type binding regions are functionally defined by their ability to bind to target molecules. Immunoglobulin-type binding regions are commonly derived from antibody or antibody-like structures; however, alternative scaffolds from other sources are contemplated within the scope of the term.

Immunoglobulin (Ig) proteins have a structural domain known as an Ig domain. Ig domains range in length from about 70-110 amino acid residues and possess a characteristic Ig-fold, in which typically 7 to 9 antiparallel beta strands arrange into two beta sheets which form a sandwich-like structure. The Ig fold is stabilized by hydrophobic amino acid interactions on inner surfaces of the sandwich and highly conserved disulfide bonds between cysteine residues in the strands. Ig domains may be variable (IgV or V-set), constant (IgC or C-set) or intermediate (IgI or I-set). Some Ig domains may be associated with a complementarity determining region (CDR), also referred to as antigen binding region (ABR), that is important for the specificity of antibodies binding to their epitopes. Ig-like domains are also found in non-immunoglobulin proteins and are classified on that basis as members of the Ig superfamily of proteins. The HUGO Gene Nomenclature Committee (HGNC) provides a list of members of the Ig-like domain containing family.

An immunoglobulin-type binding region may be a polypeptide sequence of an antibody or antigen-binding fragment thereof wherein the amino acid sequence has been varied from that of a native antibody or an Ig-like domain of a non-immunoglobulin protein, for example by molecular engineering or library screening. Because of the relevance of recombinant DNA techniques and *in vitro* library screening in the generation of immunoglobulin-type binding regions, antibodies can be redesigned to obtain desired characteristics, such as smaller size, cell entry, or other therapeutic improvements. The possible variations are many and may range from the changing of just one amino acid to the complete redesign of, for example, a variable region. Typically, changes in the variable region will be made in order to improve the antigen-binding characteristics, improve variable region stability, or reduce the potential for immunogenic responses.

There are numerous immunoglobulin-type binding regions contemplated as components of the present invention. In certain embodiments, the immunoglobulin-type binding region is derived from an immunoglobulin binding region capable of binding an extracellular target biomolecule, as defined in the claims. In certain other embodiments of the disclosure, the immunoglobulin-type binding region comprises an engineered polypeptide not derived from any immunoglobulin domain but which functions like an immunoglobulin binding region by providing high-affinity binding to an extracellular target biomolecule. This engineered polypeptide may optionally include polypeptide scaffolds comprising or consisting essentially of complementary determining regions from immunoglobulins as described herein.

There are numerous immunoglobulin-derived binding regions and non-immunoglobulin engineered polypeptides in the prior art that are useful for targeting polypeptides to specific cell-types via their high-affinity binding capabilities. The immunoglobulin-type binding region of the present cytotoxic proteins is selected from the group which includes: single-domain antibody fragments, single-chain variable (scFv) fragments, antibody variable (Fv) fragments, antigen-binding (Fab) fragments, and Fd fragments (*see*, Weiner L, Cell 148: 1081-4 (2012); Ahmad Z et al., Clin Dev Immunol 2012: 980250 (2012), for reviews).

In accordance with certain other embodiments of the disclosure, the immunoglobulin-type binding region includes engineered, alternative scaffolds to immunoglobulin domains that exhibit similar functional characteristics, such as high-affinity and specific binding of target biomolecules, and enables the engineering of improved characteristics, such as greater stability or reduced immunogenicity. For certain embodiments of the cytotoxic proteins disclosed herein, the immunoglobulin-type binding region is selected from the group which includes engineered, fibronectin-derived, 10^{th} fibronectin type III (10Fn3) domain (monobodies, AdNectins™, or AdNexins™); engineered, tenacsin-derived, tenacsin type III domain (Centryns™); engineered, ankyrin repeat motif containing polypeptide (DARPins™); engineered, low-density-lipoprotein-receptor-derived, A domain (LDLR-A) (Avimers™); lipocalin (anticalins); engineered, protease inhibitor-derived, Kunitz domain; engineered, Protein-A-derived, Z domain (Affibodies™); engineered, gamma-B crystallin-derived scaffold or engineered, ubiquitin-derived scaffold (Affilins); Sac7d-derived polypeptides (Nanoffitins® or affitins); engineered, Fyn-derived, SH2 domain (Fynomers®); and engineered antibody mimic and any genetically manipulated counterparts of the foregoing that retains its binding functionality (Wörn A, Plückthun A, J Mol Biol 305: 989-1010 (2001); Xu L et al., Chem Biol 9: 933-42 (2002); Wikman M et al., Protein Eng Des Sel 17: 455-62 (2004); Binz H et al., Nat Biotechnol 23: 1257-68 (2005); Holliger P, Hudson P, Nat Biotechnol 23: 1126-36 (2005); Gill D, Damle N, Curr Opin Biotech 17: 653-8 (2006); Koide A, Koide S, Methods Mol Biol 352: 95-109 (2007)).

Any of the above immunoglobulin-type binding regions may be used as a component of the present invention so long as the binding region component has a dissociation constant of 10⁻⁵ to 10⁻¹² moles/liter, preferably less than 200 nM, towards an extracellular target biomolecule as described herein.

This system is modular, in that various, diverse immunoglobulin-type binding regions may be used with the same Shiga toxin effector region to provide for diverse targeting of various extracellular target biomolecules and thus targeting of cytotoxicity or cytostasis to various diverse cell types.

### B. Shiga Toxin Effector Regions Derived from A Subunits of Members of the Shiga Toxin Family

For purposes of the present invention, the phrase "Shiga toxin effector region" refers to a polypeptide region derived from a Shiga toxin A Subunit of a member of the Shiga toxin family that is capable of inactivating ribosomes and effectuating cytotoxicity and/or cytostatic effects. A member of the Shiga toxin family refers to any member of a family of naturally occurring protein toxins which are structurally and functionally related, notably toxins isolated from S. *dysenteriae* and *E. coli* (Johannes, Nat Rev Microbiol 8: 105-16 (2010)). For example, the Shiga toxin family encompasses true Shiga toxin (Stx) isolated from S. *dysenteriae* serotype 1, Shiga-like toxin 1 variants (SLT1 or Stx1 or SLT-1 or Slt-I) isolated from serotypes of enterohemorrhagic *E. coli,* and Shiga-like toxin 2 variants (SLT2 or Stx2 or SLT-2) isolated from serotypes of enterohemorrhagic *E. coli.* SLT1 differs by only one residue from Stx, and both have been referred to as Verocytotoxins or Verotoxins (VTs) (O'Brien, Curr Top Microbiol Immunol 180: 65-94 (1992)). Although SLT1 and SLT2 variants are only about 53-60% similar to each other at the amino acid sequence level, they share mechanisms of enzymatic activity and cytotoxicity common to the members of the Shiga toxin family (Johannes, Nat Rev Microbiol 8: 105-16 (2010)). Over 39 different Shiga toxins have been described, such as the defined subtypes Stx1a, Stx1c, Stx1d, and Stx2a-g (Scheutz F et al., J Clin Microbiol 50: 2951-63 (2012)). Members of the Shiga toxin family are not naturally restricted to any bacterial species because Shiga-toxin-encoding genes can spread among bacterial species via horizontal gene transfer (Strauch E et al., Infect Immun 69: 7588-95 (2001); Zhaxybayeva O, Doolittle W, Curr Biol. 21: R242-6 (2011)). As an example of interspecies transfer, a Shiga toxin was discovered in a strain of A. *haemolyticus* isolated from a patient (Grotiuz G et al., J Clin Microbiol 44: 3838-41 (2006)). Once a Shiga toxin encoding polynucleotide enters a new subspecies or species, the Shiga toxin amino acid sequence is presumed to be capable of developing slight sequence variations due to genetic drift and/or selective pressure while still maintaining a mechanism of cytotoxicity common to members of the Shiga toxin family *(see* Scheutz, J Clin Microbiol 50: 2951-63 (2012)).

Shiga toxin effector regions of the invention may comprise or consist essentially of a polypeptide derived from a Shiga toxin A Subunit dissociated from any form of its native Shiga toxin B Subunit. In addition, the cytotoxic proteins of the present invention may not comprise any polypeptide comprising or consisting essentially of a functional binding domain of a native Shiga toxin B subunit. Rather, the Shiga toxin A Subunit derived regions are functionally associated with heterologous immunoglobulin-type binding regions to effectuate cell targeting.

In certain embodiments, a Shiga toxin effector region of the invention as defined in the claims may comprise or consist of a full length Shiga toxin A Subunit SLT-1A (SEQ ID NO:1), StxA SEQ ID NO:2, or SLT-2A (SEQ ID NO:3), noting that naturally occurring Shiga toxin A Subunits may comprise precursor forms containing signal sequences of about 22 amino acids at their amino-terminals which are removed to produce mature Shiga toxin A Subunits. In other embodiments, the Shiga toxin effector region of the invention as defined in the claims comprises or consists essentially of a truncated Shiga toxin A Subunit which is shorter than a full-length Shiga toxin A Subunit.

Shiga-like toxin 1 A Subunit truncations are catalytically active, capable of enzymatically inactivating ribosomes *in vitro,* and cytotoxic when expressed within a cell (LaPointe, J Biol Chem 280: 23310-18 (2005)). The smallest Shiga toxin A Subunit fragment exhibiting full enzymatic activity is a polypeptide composed of residues 1-239 of Slt1A (LaPointe, J Biol Chem 280: 23310-18 (2005)). Although the smallest fragment of the Shiga toxin A Subunit reported to retain substantial catalytic activity was residues 75-247 of StxA (Al-Jaufy, Infect Immun 62: 956-60 (1994)), a StxA truncation expressed *de novo* within a eukaryotic cell requires only up to residue 240 to reach the cytosol and exert catalytic inactivation of ribosomes (LaPointe, J Biol Chem 280: 23310-18 (2005)).

Shiga toxin effector regions may commonly be less than the full length A subunit. It is preferred that the Shiga toxin effector region maintain the polypeptide region from amino acid position 77 to 239 (SLT-1A SEQ ID NO:1 or StxA SEQ ID NO:2) or the equivalent in other A Subunits of members of the Shiga toxin family. For example, in certain embodiments of the invention, the Shiga toxin effector regions derived from SLT-1A may comprise or consist of amino acids 75 to 251 of SEQ ID NO:1, 1 to 241 of SEQ ID NO:1, 1 to 251 of SEQ ID NO:1, or amino acids 1 to 261 of SEQ ID NO:1. Similarly, the Shiga toxin effector regions derived from StxA may comprise or consist of amino acids 75 to 251 of SEQ ID NO:2, 1 to 241 of SEQ ID NO:2, 1 to 251 of SEQ ID NO:2, or amino acids 1 to 261 of SEQ ID NO:2. Additionally, the Shiga toxin effector regions derived from SLT-2 may comprise or consist of amino acids 75 to 251 of SEQ ID NO:3, 1 to 241 of SEQ ID NO:3, 1 to 251 of SEQ ID NO:3, or amino acids 1 to 261 of SEQ ID NO:3.

The invention further provides variants of the cytotoxic proteins of the invention, wherein the Shiga toxin effector region differs from a naturally occurring Shiga toxin A Subunit by up to 1, 2, 3, 4, 5, 6, 7, 8, 9, 10, 15, 20, 25, 30, 35, 40 or more amino acid residues (but by no more than that which retains at least 85%, 90%, 95%, 99% or more amino acid sequence identity). Thus, a polypeptide region derived from an A Subunit of a member of the Shiga toxin family may comprise additions, deletions, truncations, or other alterations from the original sequence so long as at least 85%, 90%, 95%, 99% or more amino acid sequence identity is maintained to a naturally occurring Shiga toxin A Subunit, as defined in the claims.

Accordingly, in certain embodiments, the Shiga toxin effector region comprises or consists of amino acid sequences having at least 85%, 90%, 95%, 97%, 98%, 99%, 99.5% or 99.7% overall sequence identity to SLT-1A (SEQ ID NO:1), StxA (SEQ ID NO:2), or SLT-2A (SEQ ID NO:3).

Optionally, either a full length or a truncated version of the Shiga toxin A Subunit may comprise one or more mutations (*e.g*. substitutions, deletions, insertions or inversions). It is preferred in certain embodiments of the invention that the Shiga toxin effector region has sufficient sequence identity to a naturally occurring Shiga toxin A Subunit to retain cytotoxicity after entry into a cell, either by well-known methods of host cell transformation, transfection, infection or induction, or by internalization mediated by targeting immunoglobulin-type binding region linked with the Shiga toxin effector region. The most critical residues for enzymatic activity and/or cytotoxicity in the Shiga toxin A Subunits were mapped to the following residue-positions: aspargine-75, tyrosine-77, glutamate-167, arginine-170, and arginine-176 among others (Di, Toxicon 57: 535-39 (2011)). In any one of the embodiments of the invention, the Shiga toxin effector region may preferably but not necessarily maintain one or more conserved amino acids at positions, such as those found at positions 77, 167, 170, and 203 in StxA, SLT-1A, or the equivalent conserved position in other members of the Shiga toxin family which are typically required for cytotoxic activity. The capacity of a cytotoxic protein of the invention to cause cell death, *e.g.* its cytotoxicity, may be measured using any one or more of a number of assays well known in the art.

In certain embodiments of the disclosure, one or more amino acid residues may be mutated or deleted in order to reduce or eliminate cytotoxic activity of the Shiga toxin effector region. The cytotoxicity of the A Subunits of members of the Shiga toxin family may be abrogated or eliminated by mutation or truncation. The positions labeled tyrosine-77, glutamate-167, arginine-170, tyrosine-114, and tryptophan-203 have been shown to be important for the catalytic activity of Stx, Stx1, and Stx2 (Hovde C et al., Proc Natl Acad Sci USA 85: 2568-72 (1988); Deresiewicz R et al., Biochemistry 31: 3272-80 (1992); Deresiewicz R et al., Mol Gen Genet 241: 467-73 (1993); Ohmura M et al., Microb Pathog 15: 169-76 (1993); Cao C et al., Microbiol Immunol 38: 441-7 (1994); Suhan M, Hovde C, Infect Immun 66: 5252-9 (1998)). Mutating both glutamate-167 and arginine-170 eliminated the enzymatic activity of Slt-I A1 in a cell-free ribosome inactivation assay (LaPointe, J Biol Chem 280: 23310-18 (2005)). In another approach using *de novo* expression of Slt-I A1 in the endoplasmic reticulum, mutating both glutamate-167 and arginine-170 eliminated Slt-I A1 fragment cytotoxicity at that expression level (LaPointe, J Biol Chem 280: 23310-18 (2005)). A truncation analysis demonstrated that a fragment of StxA from residues 75 to 268 still retains significant enzymatic activity *in vitro* (Haddad, J Bacteriol 175: 4970-8 (1993)). A truncated fragment of Slt-I A1 containing residues 1-239 displayed significant enzymatic activity *in vitro* and cytotoxicity by *de novo* expression in the cytosol (LaPointe, J Biol Chem 280: 23310-18 (2005)). Expression of a Slt-I A1 fragment truncated to residues 1-239 in the endoplasmic reticulum was not cytotoxic because it could not retrotranslocate into the cytosol (LaPointe, J Biol Chem 280: 23310-18 (2005)).

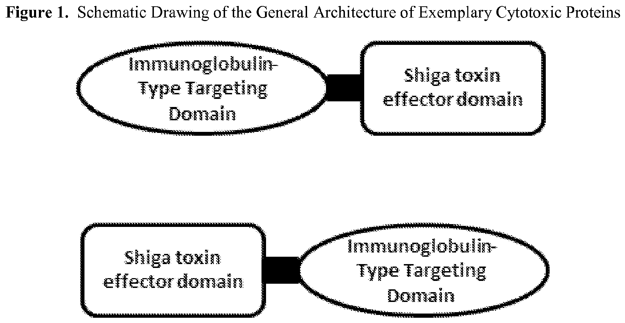

For the purposes of the present invention, the specific order or orientation is not fixed for the Shiga toxin effector region and the immunoglobulin-type binding region in relation to each other or the entire cytotoxic protein's N-terminal(s) and C-terminal(s) (*see e.g.* Figure 1). In the above cytotoxic proteins, the immunoglobulin-type regions and Shiga toxin effector regions may be directly linked to each other and/or suitably linked to each other via one or more intervening polypeptide sequences, such as with one or more linkers well known in the art.

### Extracellular Target Biomolecules

The immunoglobulin-type binding region of the cytotoxic protein of the invention comprises a polypeptide region capable of binding specifically to an extracellular target biomolecule, preferably which is physically-coupled to the surface of a cell type of interest, such as a cancer cell, tumor cell, plasma cell, infected cell, or host cell harboring an intracellular pathogen.

The term "target biomolecule" refers to a biological molecule, commonly a protein or a protein modified by post-translational modifications, such as glycosylation, which is capable of being bound by an immunoglobulin-type binding region to target a cytotoxic protein to a specific cell-type or location within an organism. Extracellular target biomolecules may include various epitopes, including unmodified polypeptides, polypeptides modified by the addition of biochemical functional groups, and glycolipids. It is desirable that an extracellular target biomolecule be endogenously internalized or be readily forced to internalize upon interaction with the cytotoxic protein.

For purposes of the present invention, the term "extracellular" with regard to modifying a target biomolecule refers to a biomolecule that has at least a portion of its structure exposed to the extracellular environment. Extracellular target biomolecules include cell membrane components, transmembrane spanning proteins, cell membrane-anchored biomolecules, cell-surface-bound biomolecules, and secreted biomolecules.

With regard to the present invention, the term "physically coupled" when used to describe a target biomolecule means both covalent and/or non-covalent intermolecular interactions that couple the target biomolecule, or a portion thereof, to the outside of a cell, such as a plurality of non-covalent interactions between the target biomolecule and the cell where the energy of each single interaction is on the order of about 1-5 kiloCalories (*e.g*. electrostatic bonds, hydrogen bonds, Van der Walls interactions, hydrophobic forces, etc.). All integral membrane proteins can be found physically coupled to a cell membrane, as well as peripheral membrane proteins. For example, an extracellular target biomolecule might comprise a transmembrane spanning region, a lipid anchor, a glycolipid anchor, and/or be non-covalently associated (*e.g*. via non-specific hydrophobic interactions and/or lipid binding interactions) with a factor comprising any one of the foregoing.

The immunoglobulin-type binding regions of the cytotoxic proteins of the invention may be designed or selected based on numerous criteria, such as the cell-type specific expression of their target biomolecules and/or the physical localization of their target biomolecules with regard to specific cell types. For example, certain cytotoxic proteins of the present invention comprise immunoglobulin-type binding domains capable of binding cell-surface targets which are expressed exclusive by only one cell-type to the cell surface. This permits the targeted cell-killing of specific cell types with a high preferentiality (at least a 3-fold cytotoxic effect) over "bystander" cell types that do not express the extracellular target biomolecule. Alternatively, the expression of the target biomolecule of the immunoglobulin-type binding region may be non-exclusive to one cell type if the extracellular target biomolecule is expressed in low enough amounts and/or physically coupled in low amounts with cell types that are not to be targeted. This also permits the targeted cell-killing of specific cell types with a high preferentiality (at least a 3-fold cytotoxic effect) over "bystander" cell types that do not express significant amounts of the extracellular target biomolecule or are not physically coupled to significant amounts of the extracellular target biomolecule. A targeted cell may be killed using the cytotoxic proteins of the invention under varied conditions of the cell, such as *ex vivo, in vitro* cultured, or *in vivo*-including cells *in situ* in their native locations within a multicellular organism.

Extracellular target biomolecules of the immunoglobulin-type binding region of the cytotoxic proteins of the invention may include biomarkers over-proportionately or exclusively present on cancer cells, immune cells, and cells infected with intracellular pathogens, such as viruses, bacteria, fungi, prions, or protozoans.

In certain embodiments, the immunoglobulin-type binding region is selected for specific and high-affinity binding to a surface antigen on the cell surface of a cancer cell, where the antigen is restricted in expression to cancer cells (*see* Glokler J et al., Molecules 15: 2478-90 (2010); Liu Y et al., Lab Chip 9: 1033-6 (2009)). In accordance with other embodiments, the immunoglobulin-type binding region is selected for specific and high-affinity binding to a surface antigen on the cell surface of a cancer cell, where the antigen is over-expressed or preferentially expressed by cancer cells as compared to non-cancer cells. Some representative target biomolecules include, but are not limited to the following enumerated targets associated with cancers and/or specific immune cell types. Many immunoglobulin-type binding regions that recognize epitopes associated with cancer cells exist in the prior art, such as binding regions that target B-lymphocyte antigen protein CD20 (CD20), CD22, CD25, CD30, CD40, CD79, CD248 (endosialin), CD33 (Siglec-3), CD52 (CAMPATH-1 antigen), carcinoembryonic antigen protein (CEA), epidermal growth factor receptor (EGFR/ErbB1), human epidermal growth factor receptor 2 (HER2/Neu/ErbB2/CD340), epithelial cell adhesion molecule (EpCAM), Ephrin type-B receptor 2 (EphB2), Epstein-Barr Virus antigen proteins, prostate-specific membrane antigen protein (PSMA), endoglin (END/CD105), fibroblast activation protein (FAP/seprase), and vascular endothelial growth factor receptors (VEGFRs) (*see* Bagley R et al., Int J Oncol 34: 619-27 (2009)). This list of target biomolecules is intended to be non-limiting. It will be appreciated by the skilled worker that any desired target biomolecule associated with a cancer cell type may be used to design or select an immunoglobulin-type binding region to be coupled with the Shiga toxin effector region to produce a cytotoxic protein of the invention.

Examples of other target biomolecules which are strongly associated with cancer cells are B-lymphocyte antigen protein CD19 (CD19), CD21, CS1 (SLAM family number 7 or SLAMF7), cell surface A33 antigen protein (gpA33), mucins (such as MUC1), tumor-associated glycoprotein 72 (TAG-72), carbonic anhydrase IX (CA9/CAIX), folate binding protein (FBP), ganglioside GD2, ganglioside GD3, ganglioside GM2, integrins alpha-V beta-3 (α_{V}β₃), integrins alpha-5 beta-1 (α₅β₁), receptor tyrosine-protein kinase erB-3, insulin-like growth factor 1 receptor (IGF1R), ephrin type-A receptor 3 (EphA3), tumor necrosis factor receptor 10A (TRAIL-R1/DR4), tumor necrosis factor receptor 10B (TRAIL-R2), receptor activator of nuclear factor kappa B (RANK), Tenascin C, Claudin proteins (CLDN3, CLDN4), mesothelin (MSLN) and CD64 (FcγRI) *(see,* Hough C et al., Cancer Res 60: 6281-7 (2000); Thepen T et al., Nat Biotechnol 18: 48-51 (2000); Pastan I et al., Nat Rev Cancer 6: 559-65 (2006); Pastan, Annu Rev Med 58: 221-37 (2007); Fitzgerald D et al., Cancer Res 71: 6300-9 (2011); Scott et al., Cancer Immun 12: 14-22 (2012)). This list of target biomolecules is intended to be non-limiting.

In addition, there are numerous other examples of target biomolecules, such as melanoma antigen recognized by T-cells protein (MART-1/melanA), melanocyte protein PMEL (gp100), tyrosinase, tyrosinease-related protein 1 (TRP-1), tyrosinease-related protein 2 (TRP-2), melanoma-associated antigen 1 protein (MAGE-1), melanoma-associated antigen 3 (MAGE-3), GAGE/PAGE proteins, BAGE proteins, Preferentially Expressed Antigen of Melanoma (PRAME) proteins, receptor for advanced glycation end products (RAGE), cancer-testis antigen NY-ESO-1, cancer-testis antigen LAGE proteins, lysophosphatidlglycerol acyltransferase 1 (LPGAT1/IAA0205), SART proteins, ADP-ribosyltransferases (ART1, ART4), Wilms' tumor antigen, prostate specific antigen protein (PSA), prostate stem cell antigen protein (PSCA), human aspartyl (asparaginyl) beta-hydroxylase (HAAH), ephrin type-A receptor 2 (EphA2), receptor tyrosine-protein kinase erbB-3, tyrosinase associated antigen (TAA), break point cluster region-c-abl oncogene (BCR-ABL) proteins, alpha-fetoprotein antigen 17-A1 protein, bladder tumor antigen (BTA), CD38, CD15, CD23, CD53, CD88, CD129, CD183, CD191, CD193, CD244, CD294, CD305, C3AR, FceRIa, galectin-9, mrp-14, siglec-8, siglec-10, CD49d, CD13, CD44, CD54, CD63, CD69, CD123, TLR4, IgE, CD107a, CD203c, CD14, CD15, CD64, CD68, CD80, CD86, CD105, CD115, F4/80, ILT-3, Galectin-3, CD11a-c, GITRL, MHC Class II, CD284-TLR4, CD107-Mac3, CD195-CCR5, HLA-DR, CD16/32, CD282-TLR2, and CD123 *(see,* Scott A et al., Cancer Immunity 12: 14 (2012); Cheever M et al., Clin Cancer Res 15: 5323-37 (2009), for target biomolecules and note the target molecules described therein are non-limiting examples). It will be appreciated by the skilled worker that any desired target biomolecule may be used to design or select an immunoglobulin-type binding region to be coupled with the Shiga toxin effector region to produce a cytotoxic protein of the invention.

### II. Examples of Specific Structural Variations of the Cytotoxic Protein

Among certain embodiments of the present invention, the cytotoxic proteins comprise the Shiga toxin effector region comprising or consisting of amino acids 75 to 251 of SLT-1A (SEQ ID NO:1), StxA (SEQ ID NO:2), or SLT-2A (SEQ ID NO:3). Further embodiments are cytotoxic proteins in which the Shiga toxin effector region comprises or consists of amino acids 1 to 241 of SLT-1A (SEQ ID NO:1), StxA (SEQ ID NO:2), or SLT-2A (SEQ ID NO:3). Further embodiments are cytotoxic proteins in which the Shiga toxin effector region comprises or consists of amino acids 1 to 251 of SLT-1A (SEQ ID NO:1), StxA (SEQ ID NO:2), or SLT-2A (SEQ ID NO:3). Further embodiments are cytotoxic proteins in which the Shiga toxin effector region comprises or consists of amino acids 1 to 261 of SLT-1A (SEQ ID NO:1), StxA (SEQ ID NO:2), or SLT-2A (SEQ ID NO:3).

For certain embodiments of the present invention, the immunoglobulin-type binding region comprises or consists of amino acids 269-508 of SEQ ID NO:4, which exhibits high affinity binding specifically to human CD38. Among certain embodiments of the present invention, the immunoglobulin-type binding region comprises or consists of amino acids 1-119 of SEQ ID NO:5, which exhibits high affinity binding specifically to human HER2.

As used herein, the term "heavy chain variable (V_{H}) domain" or "light chain variable (V_{L}) domain" respectively refer to any antibody V_{H} or V_{L} domain (*e.g.* a human V_{H} or V_{L} domain) as well as any derivative thereof retaining at least qualitative antigen binding ability of the corresponding native antibody (*e.g.* a humanized V_{H} or V_{L} domain derived from a native murine V_{H} or V_{L} domain). A V_{H} or V_{L} domain consists of a "framework" region interrupted by the three CDRs or ABRs. The framework regions serve to align the CDRs for specific binding to an epitope of an antigen. From amino-terminus to carboxyl-terminus, both V_{H} and V_{L} domains comprise the following framework (FR) and CDR regions: FR1, CDR1, FR2, CDR2, FR3, CDR3, and FR4.

It is within the scope of the disclosure to use fragments, variants, and/or derivatives of the polypeptides of the cytotoxic proteins of the invention which contain a functional extracellular target biomolecule binding site, and even more preferably capable of binding the target biomolecule with high affinity (*e.g.* as shown by K_{D}). For example, the invention provides polypeptide sequences that can bind to CD38 and polypeptide sequences that can bind to HER2. Any immunoglobulin-type binding region comprising a polypeptide may be substituted for this region that binds to CD38 or HER2, expressed on a cell surface, with a dissociation constant of 10⁻⁵ to 10⁻¹² moles/liter, preferably less than 200 nM.

Among certain embodiments of the present invention, the immunoglobulin-type binding region may be derived from a nanobody® or single domain immunoglobulin-derived region V_{HH}. Generally, nanobodies are constructed from fragments of naturally occurring single, monomeric variable domain antibodies (sdAbs) of the sort found in camelids and cartilaginous fishes (Chondrichthyes). Nanobodies are engineered from these naturally occurring antibodies by truncating the single, monomeric variable domain to create a smaller and more stable molecule. Due to their small size, nanobodies are able to bind to antigens that are not accessible to whole antibodies. Among certain embodiments of the present invention, the immunoglobulin-type binding region may be derived from a nanobody® or single domain immunoglobulin-derived region V_{HH} (*e.g.* amino acids 1-119 of SEQ ID NO:5), which exhibits high affinity binding specifically to human HER2 proteins.

### III. The General Function of the Cytotoxic Protein

The present invention provides various cytotoxic proteins for the selective killing of specific cell types, the proteins each comprising 1) an immunoglobulin-type binding region for cell targeting as defined in the claims; and 2) a Shiga toxin effector region for cell killing as defined in the claims. The linking of cell targeting immunoglobulin-type binding regions with Shiga-toxin-Subunit-A-derived regions enables the engineering of cell-type specific targeting of the potent Shiga toxin cytotoxicity or cytostasis. In certain embodiments, the cytotoxic proteins of the invention are capable of binding extracellular target biomolecules associated with the cell surface of particular cell types and entering those cells. Once internalized within a targeted cell type, certain embodiments of the cytotoxic proteins of the invention are capable of routing a cytotoxic Shiga toxin effector polypeptide fragment into the cytosol of the target cell. Once in the cytosol of a targeted cell type, certain embodiments of the cytotoxic proteins of the invention are capable of enzymatically inactivating ribosomes and eventually killing the cell. This system is modular in that any number of diverse immunoglobulin-type binding regions can be used to target this potent cytotoxicity to various, diverse cell types and because various Shiga-toxin-Subunit-A derived effector regions are potently cytotoxic once inside a eukaryotic cell.

### A. Cell Kill via Targeted Shiga Toxin Cytotoxicity

Because members of the Shiga toxin family are adapted to killing eukaryotic cells, cytotoxic proteins designed using Shiga toxin effector regions can show potent cell-kill activity. The A Subunits of members of the Shiga toxin family comprise enzymatic domains capable of killing a eukaryotic cell once in the cell's cytosol. Certain embodiments of the cytotoxic proteins of the invention take advantage of this cytotoxic mechanism.

As defined in the claims, upon contacting a cell physically coupled with an extracellular target biomolecule of the immunoglobulin-type binding region of a cytotoxic protein of the invention, the cytotoxic protein is capable of causing death of the cell. Cell kill may be accomplished using a cytotoxic protein of the invention under varied conditions of target cells, such as an *ex vivo* manipulated target cell, a target cell cultured *in vitro,* a target cell within a tissue sample cultured *in vitro,* or a target cell *in vivo.*

### B. Selective Cytotoxicity among Cell Types

By targeting the delivery of enzymatically active Shiga toxin regions using high-affinity immunoglobulin-type binding regions to specific cell types, this potent cell-kill activity can be restricted to preferentially killing selected cell types.

In certain embodiments, upon administration of the cytotoxic protein of the invention to a mixture of cell types, the cytotoxic protein is capable of selectively killing those cells which are physically coupled with an extracellular target biomolecule compared to cell types not physically coupled with an extracellular target biomolecule. Because members of the Shiga toxin family are adapted for killing eukaryotic cells, cytotoxic proteins designed using Shiga toxin effector regions can show potent cytotoxic activity. By targeting the delivery of enzymatically active Shiga toxin regions to specific cell types using high-affinity immunoglobulin-type binding regions, this potent cell kill activity can be restricted to preferentially killing only those cell types desired to be targeted by their physical association with a target biomolecule of the chosen immunoglobulin-type binding regions.

In certain embodiments, the cytotoxic protein of the invention is capable of selectively or preferentially causing the death of a specific cell type within a mixture of two or more different cell types. This enables the targeted cytotoxic activity to specific cell types with a high preferentiality, such as a 3-fold cytotoxic effect, over "bystander" cell types that do not express the target biomolecule. Alternatively, the expression of the target biomolecule of the immunoglobulin-type binding region may be non-exclusive to one cell type if the target biomolecule is expressed in low enough amounts and/or physically coupled in low amounts with cell types that are not to be targeted. This enables the targeted cell-killing of specific cell types with a high preferentiality, such as a 3-fold cytotoxic effect, over "bystander" cell types that do not express significant amounts of the target biomolecule or are not physically coupled to significant amounts of the target biomolecule.

In certain further embodiments, upon administration of the cytotoxic protein to two different populations of cell types, the cytotoxic protein is capable of causing cell death as defined by the half-maximal cytotoxic concentration (CD₅₀) on a population of target cells, whose members express an extracellular target biomolecule of the binding region of the cytotoxic protein, at a dose at least three-times lower than the CD₅₀ dose of the same cytotoxic protein to a population of cells whose members do not express an extracellular target biomolecule of the binding region of the cytotoxic protein.

In certain embodiments, the cytotoxic activity toward populations of cell types physically coupled with an extracellular target biomolecule is at least 3-fold higher than the cytotoxic activity toward populations of cell types not physically coupled with any extracellular target biomolecule of the immunoglobulin-type binding region. According to the present invention, selective cytotoxicity may be quantified in terms of the ratio (a/b) of (a) cytotoxicity towards a population of cells of a specific cell type physically coupled with a target biomolecule of the binding region to (b) cytotoxicity towards a population of cells of a cell type not physically coupled with a target biomolecule of the immunoglobulin-type binding region. In certain embodiments, the cytotoxicity ratio is indicative of selective cytotoxicity which is at least 3-fold, 5-fold, 10-fold, 15-fold, 20-fold, 25-fold, 30-fold, 40-fold, 50-fold, 75-fold, 100-fold, 250-fold, 500-fold, 750-fold, or 1000-fold higher for populations of cells or cell types physically coupled with a target biomolecule of the immunoglobulin-type binding region compared to populations of cells or cell types not physically coupled with a target biomolecule of the immunoglobulin-type binding region.

This preferential cell-killing function allows a targeted cell to be killed by certain cytotoxic proteins of the invention under varied conditions and in the presence of non-targeted bystander cells, such as *ex vivo* manipulated mixtures of cell types, *in vitro* cultured tissues with mixtures of cell types, or *in vivo* in the presence of multiple cell types (*e.g. in situ* or in its native location within a multicellular organism).

In addition, catalytically inactive forms of the cytotoxic proteins of the invention optionally may be used for diagnostic functions. The conjugating of additional diagnostic agents known in the art to cytotoxic proteins of the invention may allow for the imaging of intracellular organelles (*e.g*. Golgi, endoplasmic reticulum, and cytosolic compartments) of individual cancer cells, immune cells, or microbial infected cells in a patient or biopsy sample. For example, this may be useful in diagnosing neoplastic cell types, assaying the progression of anticancer therapies over time, and/or evaluating the presence of residual cancer cells after surgical excision of a tumor mass.

### Variations in the Polypeptide Sequence of the Cytotoxic Protein which Maintain Overall Structure and Function

In certain of the above embodiments, the cytotoxic protein of the invention is a variant in which there are one or more conservative amino acid substitutions introduced into the polypeptide region(s). As used herein, the term "conservative substitution" denotes that one or more amino acids are replaced by another, biologically similar amino acid residue. Examples include substitution of amino acid residues with similar characteristics, *e.g*. small amino acids, acidic amino acids, polar amino acids, basic amino acids, hydrophobic amino acids and aromatic amino acids (*see,* for example, Table B below). An example of a conservative substitution with a residue normally not found in endogenous, mammalian peptides and proteins is the conservative substitution of an arginine or lysine residue with, for example, ornithine, canavanine, aminoethylcysteine, or another basic amino acid. For further information concerning phenotypically silent substitutions in peptides and proteins (*see e.g.* Bowie J et al., Science 247: 1306-10 (1990)). In the scheme below are conservative substitutions of amino acids grouped by physicochemical properties. I: neutral, hydrophilic, II: acids and amides, III: basic, IV: hydrophobic, V: aromatic, bulky amino acids.

**TABLE B. Examples of Conservative Amino Acid Substitutions**

| **I** | **II** | **III** | **IV** | **V** |
|---|---|---|---|---|
| A | N | H | M | F |
| S | D | R | L | Y |
| T | E | K | I | W |
| P | Q | | V | |
| G | | | C | |

In certain embodiments, as defined in the claims, a cytotoxic protein of the invention may comprise functional fragments or variants of a polypeptide region of the invention that have, at most, 20, 15, 10, 9, 8, 7, 6, 5, 4, 3, 2, or 1 amino acid substitutions compared to a polypeptide sequence recited herein, as long as it retains measurable biological activity alone or as a component of a cytotoxic protein. As defined in the claims, variants of cytotoxic proteins are within the scope of the invention as a result of changing a polypeptide of the cytotoxic protein by altering one or more amino acids or deleting or inserting one or more amino acids, such as within the immunoglobulin-type binding region or the Shiga toxin effector region, in order to achieve desired properties, such as changed cytotoxicity, changed cytostatic effects, changed immunogenicity, and/or changed serum half-life. A polypeptide of a cytotoxic protein of the invention may further be with or without a signal sequence.

In certain embodiments, a cytotoxic protein of the invention shares at least 85%, 90%, 95%, 96%, 97%, 98%, 99% or more amino acid sequence identity to any one of the amino acid sequences of a cytotoxic protein recited herein, as long as it retains measurable biological activity, such as cytotoxicity, extracellular target biomolecule binding, enzymatic catalysis, or subcellular routing. The immunoglobulin-type binding region may differ from the amino acid sequences of a cytotoxic protein recited herein, as long as it retains binding functionality to its extracellular target biomolecule. Binding functionality will most likely be retained if the amino acid sequences of the CDRs or ABRs are identical. For example, a cytotoxic protein is within the claim scope that consists essentially of 85% amino acid identity to a cytotoxic protein recited herein which for the purposes of determining the degree of amino acid identity, the amino acid residues that form the CDRs or ABRs are disregarded. Binding functionality can be determined by the skilled worker using standard techniques.

In certain embodiments, the Shiga toxin effector region may be altered to change the enzymatic activity of the Shiga toxin effector region, while retaining a cytotoxic activity, as defined in the claims. Possible alterations include mutations to the Shiga toxin effect region selected from the group consisting of: amino acid residue deletion and substitution.

The cytotoxicity of the A Subunits of members of the Shiga toxin family may be abrogated or eliminated by mutation or truncation. The positions labeled tyrosine-77, glutamate-167, arginine-170, tyrosine-114, and tryptophan-203 have been shown to be important for the catalytic activity of Stx, Stx1, and Stx2 (Hovde C et al., Proc Natl Acad Sci USA 85: 2568-72 (1988); Deresiewicz R et al., Biochemistry 31: 3272-80 (1992); Deresiewicz R et al., Mol Gen Genet 241: 467-73 (1993); Ohmura M et al., Microb Pathog 15: 169-76 (1993); Cao C et al., Microbiol Immunol 38: 441-7 (1994); Suhan M, Hovde C, Infect Immun 66: 5252-9 (1998)). Mutating both glutamate-167 and arginine-170 eliminated the enzymatic activity of Slt-I A1 in a cell-free ribosome inactivation assay (LaPointe, J Biol Chem 280: 23310-18 (2005)). In another approach using *de novo* expression of Slt-I A1 in the endoplasmic reticulum, mutating both glutamate-167 and arginine-170 eliminated Slt-I A1 fragment cytotoxicity at that expression level (LaPointe, J Biol Chem 280: 23310-18 (2005)). A truncation analysis demonstrated that a fragment of StxA from residues 75 to 268 still retains significant enzymatic activity *in vitro* (Haddad, J Bacteriol 175: 4970-8 (1993)). A truncated fragment of Slt-I A1 containing residues 1-239 displayed significant enzymatic activity *in vitro* and cytotoxicity by *de novo* expression in the cytosol (LaPointe, J Biol Chem 280: 23310-18 (2005)). Expression of a Slt-I A1 fragment truncated to residues 1-239 in the endoplasmic reticulum was not cytotoxic because it could not retranslocate in the cytosol (LaPointe, J Biol Chem 280: 23310-18 (2005)).

The most critical residues for enzymatic activity and/or cytotoxicity in the Shiga toxin A Subunits were mapped to the following residue-positions: aspargine-75, tyrosine-77, glutamate-167, arginine-170, and arginine-176 among others (Di, Toxicon 57: 535-39 (2011)). In particular, a double-mutant construct of Stx2A containing glutamate-E167-to-lysine and arginine-176-to-lysine mutations was completely inactivated; whereas, many single mutations in Stx1 and Stx2 showed a 10-fold reduction in cytotoxicity. Further, truncation of Stx1A to 1-239 or 1-240 reduced its cytotoxicity, and similarly, truncation of Stx2A to a conserved hydrophobic residue reduced its cytotoxicity.

Shiga-like toxin 1 A Subunit truncations are catalytically active, capable of enzymatically inactivating ribosomes *in vitro,* and cytotoxic when expressed within a cell (LaPointe, J Biol Chem 280: 23310-18 (2005)). The smallest Shiga toxin A Subunit fragment exhibiting full enzymatic activity is a polypeptide composed of residues 1-239 of Slt1A (LaPointe, J Biol Chem 280: 23310-18 (2005)). Although the smallest fragment of the Shiga toxin A Subunit reported to retain substantial catalytic activity was residues 75-247 of StxA (Al-Jaufy, Infect Immun 62: 956-60 (1994)), a StxA truncation expressed *de novo* within a eukaryotic cell requires only up to residue 240 to reach the cytosol and exert catalytic inactivation of ribosomes (LaPointe, J Biol Chem 280: 23310-18 (2005)).

In certain embodiments of the disclosure derived from SLT-1A (SEQ ID NO:1) or StxA (SEQ ID NO:2), these changes include substitution of the asparagine at position 75, tyrosine at position 77, tyrosine at position 114, glutamate at position 167, arginine at position 170, arginine at position 176, and/or substitution of the tryptophan at position 203. Examples of such substitutions will be known to the skilled worker based on the prior art, such as asparagine at position 75 to alanine, tyrosine at position 77 to serine, substitution of the tyrosine at position 114 to alanine, substitution of the glutamate at position 167 to aspartate, substitution of the arginine at position 170 to alanine, substation of the arginine at position 176 to lysine, and/or substitution of the tryptophan at position 203 to alanine.

Cytotoxic proteins as defined in the claims of the invention may optionally be conjugated to one or more additional agents which may include therapeutic and/or diagnostic agents known in the art.

### Production, Manufacture, and Purification of a Cytotoxic Protein

The cytotoxic proteins of the invention may be produced using biochemical engineering techniques well known to those of skill in the art. For example, cytotoxic proteins of the invention may be manufactured by standard synthetic methods, by use of recombinant expression systems, or by any other suitable method. Thus, the cytotoxic proteins may be synthesized in a number of ways, including, e.g. methods comprising: (1) synthesizing a polypeptide or polypeptide component of a cytotoxic protein using standard solid-phase or liquid-phase methodology, either stepwise or by fragment assembly, and isolating and purifying the final peptide compound product; (2) expressing a polynucleotide that encodes a polypeptide or polypeptide component of a cytotoxic protein in a host cell and recovering the expression product from the host cell or host cell culture; or (3) cell-free *in vitro* expression of a polynucleotide encoding a polypeptide or polypeptide component of a cytotoxic protein, and recovering the expression product; or by any combination of the methods of (1), (2) or (3) to obtain fragments of the peptide component, subsequently joining (*e.g*. ligating) the fragments to obtain the peptide component, and recovering the peptide component.

It may be preferable to synthesize a polypeptide or polypeptide component of a cytotoxic protein of the invention by means of solid-phase or liquid-phase peptide synthesis. Cytotoxic proteins of the invention may suitably be manufactured by standard synthetic methods. Thus, peptides may be synthesized by, e.g. methods comprising synthesizing the peptide by standard solid-phase or liquid-phase methodology, either stepwise or by fragment assembly, and isolating and purifying the final peptide product. In this context, reference may be made to WO 1998/11125 or, *inter alia,* Fields, G et al., Principles and Practice of Solid-Phase Peptide Synthesis (Synthetic Peptides, Gregory A. Grant (ed.), Oxford University Press, U.K., 2nd ed., 2002) and the synthesis examples therein.

Cytotoxic proteins of the invention may be prepared (produced and purified) using recombinant techniques well known in the art. In general, methods for preparing polypeptides by culturing host cells transformed or transfected with a vector comprising the encoding polynucleotide and recovering the polypeptide from cell culture are described in, e.g. Sambrook et al., Molecular Cloning: A Laboratory Manual (Cold Spring Harbor Laboratory Press, NY, U.S., 1989); Dieffenbach et al., PCR Primer: A Laboratory Manual (Cold Spring Harbor Laboratory Press, N.Y., U.S., 1995). Any suitable host cell may be used to produce a cytotoxic protein of the invention. Host cells may be cells stably or transiently transfected, transformed, transduced or infected with one or more expression vectors which drive expression of a polypeptide of the invention. In addition, a cytotoxic protein of the invention may be produced by modifying the polynucleotide encoding the cytotoxic protein that result in altering one or more amino acids or deleting or inserting one or more amino acids in order to achieve desired properties, such as changed cytotoxicity, changed cytostatic effects, changed immunogenicity, and/or changed serum half-life.

Accordingly, the present disclosure also provides methods for producing a cytotoxic protein of the invention according to above recited methods and using a polynucleotide encoding part or all of a polypeptide of the invention, an expression vector comprising at least one polynucleotide of the invention capable of encoding part or all of a polypeptide of the invention when introduced into a host cell, and/or a host cell comprising a polynucleotide or expression vector of the invention.

When a polypeptide or protein is expressed using recombinant techniques in a host cell or cell-free system, it is advantageous to separate (or purify) the desired polypeptide or protein away from other components, such as host cell factors, in order to obtain preparations that are of high purity or are substantially homogeneous. Purification can be accomplished by methods well known in the art, such as centrifugation techniques, extraction techniques, chromatographic and fractionation techniques (*e.g*. size separation by gel filtration, charge separation by ion-exchange column, hydrophobic interaction chromatography, reverse phase chromatography, chromatography on silica or cation-exchange resins such as DEAE, chromatofocusing, and Protein A Sepharose chromatography to remove contaminants), and precipitation techniques (*e.g*. ethanol precipitation or ammonium sulfate precipitation. Any number of biochemical purification techniques may be used to increase the purity of a cytotoxic protein of the invention. In certain embodiments, the cytotoxic proteins of the invention may optionally be purified in homo-multimeric forms (*i.e.* a protein complex of two or more identical cytotoxic proteins).

In the Examples below are descriptions of non-limiting examples of methods for producing a cytotoxic protein of the invention, as well as specific but non-limiting aspects of cytotoxic protein production for the disclosed, exemplary, cytotoxic proteins.

### Pharmaceutical Compositions Comprising a Cytotoxic Protein

The present invention provides cytotoxic proteins for use, alone or in combination with one or more additional therapeutic agents, in a pharmaceutical composition, for treatment or prophylaxis of conditions, diseases, disorders, or symptoms described in further detail below (*e.g*. cancers, malignant tumors, non-malignant tumors, immune disorders, and microbial infections). The present invention further provides pharmaceutical compositions comprising a cytotoxic protein of the invention, or a pharmaceutically acceptable salt or solvate thereof, according to the invention, together with at least one pharmaceutically acceptable carrier or excipient. In certain embodiments, the pharmaceutical composition of the invention may comprise homo-multimeric and/or hetero-multimeric forms of the cytotoxic proteins of the invention. The pharmaceutical compositions will be useful in methods of treating, ameliorating, or preventing a disease, condition, disorder, or symptom described in further detail below. Each such disease, condition, disorder, or symptom is envisioned to be a separate embodiment with respect to uses of a pharmaceutical composition according to the invention. The invention further provides pharmaceutical compositions for use in at least one method of treatment as defined in the claims, as described in more detail below.

As used herein, the terms "patient" and "subject" are used interchangeably to refer to any organism, commonly vertebrates such as humans and animals, which presents symptoms, signs, and/or indications of at least one disease, disorder, or condition. These terms include mammals such as the non-limiting examples of primates, livestock animals (*e.g*. cattle, horses, pigs, sheep, goats, *etc*.), companion animals (*e.g.* cats, dogs, *etc*.) and laboratory animals (*e.g.* mice, rabbits, rats, *etc*.).

As used herein, "treat," "treating," or "treatment" and grammatical variants thereof refer to an approach for obtaining beneficial or desired clinical results. The terms may refer to slowing the onset or rate of development of a condition, disorder or disease, reducing or alleviating symptoms associated with it, generating a complete or partial regression of the condition, or some combination of any of the above. For the purposes of this invention, beneficial or desired clinical results include, but are not limited to, reduction or alleviation of symptoms, diminishment of extent of disease, stabilization (*e.g.* not worsening) of state of disease, delay or slowing of disease progression, amelioration or palliation of the disease state, and remission (whether partial or total), whether detectable or undetectable. "Treat," "treating," or "treatment" can also mean prolonging survival relative to expected survival time if not receiving treatment. A subject (*e.g.* a human) in need of treatment may thus be a subject already afflicted with the disease or disorder in question. The terms "treat," "treating," or "treatment" includes inhibition or reduction of an increase in severity of a pathological state or symptoms relative to the absence of treatment, and is not necessarily meant to imply complete cessation of the relevant disease, disorder, or condition.

As used herein, the terms "prevent," "preventing," "prevention" and grammatical variants thereof refer to an approach for preventing the development of, or altering the pathology of, a condition, disease, or disorder. Accordingly, "prevention" may refer to prophylactic or preventive measures. For the purposes of this invention, beneficial or desired clinical results include, but are not limited to, prevention or slowing of symptoms, progression or development of a disease, whether detectable or undetectable. A subject (*e.g.* a human) in need of prevention may thus be a subject not yet afflicted with the disease or disorder in question. The term "prevention" includes slowing the onset of disease relative to the absence of treatment, and is not necessarily meant to imply permanent prevention of the relevant disease, disorder or condition. Thus "preventing" or "prevention" of a condition may in certain contexts refer to reducing the risk of developing the condition, or preventing or delaying the development of symptoms associated with the condition.

As used herein, an "effective amount" or "therapeutically effective amount" is an amount or dose of a composition (*e.g*. a therapeutic composition or agent) that produces at least one desired therapeutic effect in a subject, such as preventing or treating a target condition or beneficially alleviating a symptom associated with the condition. The most desirable therapeutically effective amount is an amount that will produce a desired efficacy of a particular treatment selected by one of skill in the art for a given subject in need thereof. This amount will vary depending upon a variety of factors understood by the skilled worker, including but not limited to the characteristics of the therapeutic compound (including activity, pharmacokinetics, pharmacodynamics, and bioavailability), the physiological condition of the subject (including age, sex, disease type, disease stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, namely by monitoring a subject's response to administration of a compound and adjusting the dosage accordingly (*see e.g.* Remington: The Science and Practice of Pharmacy (Gennaro, ed., Mack Publishing Co., Easton, PA, U.S., 19th ed., 1995)).

### Production or Manufacture of a Pharmaceutical Composition Comprising a Cytotoxic Protein

Pharmaceutically acceptable salts or solvates of any of the cytotoxic proteins of the invention are likewise within the scope of the present invention.

The term "solvate" in the context of the present invention refers to a complex of defined stoichiometry formed between a solute (*in casu,* a polypeptide compound or pharmaceutically acceptable salt thereof according to the invention) and a solvent. The solvent in this connection may, for example, be water, ethanol or another pharmaceutically acceptable, typically small-molecular organic species, such as, but not limited to, acetic acid or lactic acid. When the solvent in question is water, such a solvate is normally referred to as a hydrate.

Cytotoxic proteins of the present invention, or salts thereof, may be formulated as pharmaceutical compositions prepared for storage or administration, which typically comprise a therapeutically effective amount of a compound of the invention, or a salt thereof, in a pharmaceutically acceptable carrier. The term "pharmaceutically acceptable carrier" includes any of the standard pharmaceutical carriers. Pharmaceutically acceptable carriers for therapeutic use are well known in the pharmaceutical art, and are described, for example, in Remington's Pharmaceutical Sciences (Mack Publishing Co. (A. Gennaro, ed., 1985). As used herein, "pharmaceutically acceptable carrier" includes any and all physiologically acceptable, *i.e.* compatible, solvents, dispersion media, coatings, antimicrobial agents, isotonic, and absorption delaying agents. Pharmaceutically acceptable carriers or diluents include those used in formulations suitable for oral, rectal, nasal or parenteral (including subcutaneous, intramuscular, intravenous, intradermal, and transdermal) administration. Exemplary pharmaceutically acceptable carriers include sterile aqueous solutions or dispersions and sterile powders for the extemporaneous preparation of sterile injectable solutions or dispersion. Examples of suitable aqueous and nonaqueous carriers that may be employed in the pharmaceutical compositions of the invention include water, ethanol, polyols (such as glycerol, propylene glycol, and polyethylene glycol), and suitable mixtures thereof, vegetable oils, such as olive oil, and injectable organic esters, such as ethyloleate. Proper fluidity can be maintained, for example, by the use of coating materials, such as lecithin, by the maintenance of the required particle size in the case of dispersions, and by the use of surfactants. In certain embodiments, the carrier is suitable for intravenous, intramuscular, subcutaneous, parenteral, spinal or epidermal administration (*e.g*. by injection or infusion). Depending on selected route of administration, the cytotoxic protein or other pharmaceutical component may be coated in a material intended to protect the compound from the action of low pH and other natural inactivating conditions to which the active cytotoxic protein may encounter when administered to a patient by a particular route of administration.

The formulations of the pharmaceutical compositions of the invention may conveniently be presented in unit dosage form and may be prepared by any of the methods well known in the art of pharmacy. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms. It may be provided in single dose injectable form, for example in the form of a pen. Compositions may be formulated for any suitable route and means of administration. Subcutaneous or transdermal modes of administration may be particularly suitable for therapeutic cytotoxic proteins described herein.

The pharmaceutical compositions of the invention may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol, or sorbic acid. Inclusion of isotonic agents, such as sugars, or sodium chloride, into the compositions, may also be desirable. In addition, prolonged absorption of the injectable pharmaceutical form may be brought about by the inclusion of agents which delay absorption such as, aluminum monostearate and gelatin.

A pharmaceutical composition of the invention also optionally includes a pharmaceutically acceptable antioxidant. Exemplary pharmaceutically acceptable antioxidants are water soluble antioxidants such as ascorbic acid, cysteine hydrochloride, sodium bisulfate, sodium metabisulfite, sodium sulfite; oil-soluble antioxidants, such as ascorbyl palmitate, butylated hydroxyanisole (BHA), butylated hydroxytoluene (BHT), lecithin, propylgallate, and alpha-tocopherol; and metal chelating agents, such as citric acid, ethylenediamine tetraacetic acid (EDTA), sorbitol, tartaric acid, and phosphoric acid.

In another aspect, the present invention provides pharmaceutical compositions comprising one or a combination of different cytotoxic proteins of the invention, and at least one pharmaceutically acceptable carrier.

Therapeutic compositions are typically sterile and stable under the conditions of manufacture and storage. The composition may be formulated as a solution, microemulsion, liposome, or other ordered structure suitable to high drug concentration. The carrier may be a solvent or dispersion medium containing, for example, water, alcohol such as ethanol, polyol (*e.g.* glycerol, propylene glycol, and liquid polyethylene glycol), or any suitable mixtures. The proper fluidity may be maintained, for example, by the use of a coating such as lecithin, by the maintenance of the required particle size in the case of dispersion and by use of surfactants according to formulation chemistry well known in the art. In certain embodiments, isotonic agents, *e.g*. sugars, polyalcohols such as mannitol, sorbitol, or sodium chloride may be desirable in the composition. Prolonged absorption of injectable compositions may be brought about by including in the composition an agent that delays absorption for example, monostearate salts and gelatin.

Solutions or suspensions used for intradermal or subcutaneous application typically include one or more of: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates; and tonicity adjusting agents such as, *e.g.,* sodium chloride or dextrose. The pH can be adjusted with acids or bases, such as hydrochloric acid or sodium hydroxide, or buffers with citrate, phosphate, and acetate. Such preparations may be enclosed in ampoules, disposable syringes or multiple dose vials made of glass or plastic.

Sterile injectable solutions may be prepared by incorporating a cytotoxic protein of the invention in the required amount in an appropriate solvent with one or a combination of ingredients described above, as required, followed by sterilization microfiltration. Dispersions may be prepared by incorporating the active compound into a sterile vehicle that contains a dispersion medium and other ingredients, such as those described above. In the case of sterile powders for the preparation of sterile injectable solutions, the methods of preparation are vacuum drying and freeze-drying (lyophilization) that yield a powder of the active ingredient in addition to any additional desired ingredient from a sterile-filtered solution thereof.

When a therapeutically effective amount of a cytotoxic protein of the invention is designed to be administered by, *e.g*. intravenous, cutaneous or subcutaneous injection, the binding agent will be in the form of a pyrogen-free, parenterally acceptable aqueous solution. Methods for preparing parenterally acceptable protein solutions, taking into consideration appropriate pH, isotonicity, and stability, are within the skill in the art. A preferred pharmaceutical composition for intravenous, cutaneous, or subcutaneous injection will contain, in addition to binding agents, an isotonic vehicle such as sodium chloride injection, Ringer's injection, dextrose injection, dextrose and sodium chloride injection, lactated Ringer's injection, or other vehicle as known in the art. A pharmaceutical composition of the present invention may also contain stabilizers, preservatives, buffers, antioxidants, or other additives well known to those of skill in the art.

As described elsewhere herein, a compound may be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art (*see e.g.* Sustained and Controlled Release Drug Delivery Systems (J. Robinson, ed., Marcel Dekker, Inc., NY, U.S., 1978)).

In certain embodiments, the pharmaceutical composition of the invention may be formulated to ensure a desired distribution *in vivo.* For example, the blood-brain barrier excludes many large and/or hydrophilic compounds. To target a therapeutic compound or composition of the invention to a particular *in vivo* location, they can be formulated, for example, in liposomes which may comprise one or more moieties that are selectively transported into specific cells or organs, thus enhancing targeted drug delivery. Exemplary targeting moieties include folate or biotin; mannosides; antibodies; surfactant protein A receptor; and p120 catenin.

### Polynucleotides, Expression Vectors, and Host Cells

Beyond the cytotoxic proteins of the present invention, the polynucleotides which encode such cytotoxic proteins are within the scope of the present invention. The term "polynucleotide" is equivalent to the term "nucleic acids" both of which include polymers of deoxyribonucleic acids (DNAs), polymers of ribonucleic acids (RNAs), analogs of these DNAs or RNAs generated using nucleotide analogs, and derivatives, fragments and homologs thereof. The polynucleotide of the invention may be single-, double-, or triple-stranded. Disclosed polynucleotides are specifically disclosed to include all polynucleotides capable of encoding an exemplary cytotoxic protein, for example, taking into account the wobble known to be tolerated in the third position of RNA codons, yet encoding for the same amino acid as a different RNA codon.

In one aspect, the invention provides polynucleotides which encode a cytotoxic protein of the invention or a complement thereof. The polynucleotides may include, *e.g*., nucleic acid sequence encoding a polypeptide at least 50%, 55%, 60%, 65%, 70%, 75%, 80%, 85%, 90%, 95%, 99% or more, identical to a polypeptide comprising one of the amino acid sequences of the cytotoxic protein. The disclosure also includes polynucleotides comprising nucleotide sequences that hybridize under stringent conditions to a polynucleotide which encodes a cytotoxic protein of the invention, or a fragment or derivative thereof, or the antisense or complement of any such sequence.

Derivatives or analogs of the polynucleotides (or cytotoxic proteins) of the invention include, *inter alia,* polynucleotide (or polypeptide) molecules having regions that are substantially homologous to the polynucleotides or cytotoxic proteins of the invention, *e.g*. by at least about 45%, 50%, 70%, 80%, 95%, 98%, or even 99% identity (with a preferred identity of 80-99%) over a polynucleotide or polypeptide sequence of the same size or when compared to an aligned sequence in which the alignment is done by a computer homology program known in the art. An exemplary program is the GAP program (Wisconsin Sequence Analysis Package, Version 8 for UNIX, Genetics Computer Group, University Research Park, Madison, WI, U.S.) using the default settings, which uses the algorithm of Smith T, Waterman M, Adv. Appl. Math. 2: 482-9 (1981). Also included are polynucleotides capable of hybridizing to the complement of a sequence encoding the proteins of the invention under stringent conditions (*see e.g.* Ausubel F, et al., Current Protocols in Molecular Biology (John Wiley & Sons, New York, NY, U.S., 1993)), and below. Stringent conditions are known to those skilled in the art and may be found in Current Protocols in Molecular Biology (John Wiley & Sons, NY, U.S., Ch. Sec. 6.3.1-6.3.6 (1989).

The present invention further provides expression vectors that comprise the polynucleotides within the scope of the invention. The polynucleotides capable of encoding the cytotoxic proteins of the invention may be inserted into known vectors, including bacterial plasmids, viral vectors and phage vectors, using material and methods well known in the art to produce expression vectors. Such expression vectors will include the polynucleotides necessary to support production of contemplated cytotoxic proteins within any host cell of choice or cell-free expression systems (*e.g*. pTxb1 and pIVEX2.3 described in the Examples below). The specific polynucleotides comprising expression vectors for use with specific types of host cells or cell-free expression systems are well known to one of ordinary skill in the art, can be determined using routine experimentation, or may be purchased.

The term "expression vector," as used herein, refers to a polynucleotide, linear or circular, comprising one or more expression units. The term "expression unit" denotes a polynucleotide segment encoding a polypeptide of interest and capable of providing expression of the nucleic acid segment in a host cell. An expression unit typically comprises a transcription promoter, an open reading frame encoding the polypeptide of interest, and a transcription terminator, all in operable configuration. An expression vector contains one or more expression units. Thus, in the context of the present invention, an expression vector encoding a cytotoxic protein comprising a single polypeptide chain (*e.g.* a scFv genetically recombined with a Shiga toxin effector region) includes at least an expression unit for the single polypeptide chain, whereas a cytotoxic protein comprising, *e.g.* two or more polypeptide chains (*e.g.* one chain comprising a V_{L} domain and a second domain comprising a V_{H} domain linked to a toxin effector region) includes at least two expression units, one for each of the two polypeptide chains of the protein. For expression of multi-chain cytotoxic proteins, an expression unit for each polypeptide chain may also be separately contained on different expression vectors (*e.g*. expression may be achieved with a single host cell into which expression vectors for each polypeptide chain has been introduced).

Expression vectors capable of directing transient or stable expression of polypeptides and proteins are well known in the art. The expression vectors generally include, but are not limited to, one or more of the following: a heterologous signal sequence or peptide, an origin of replication, one or more marker genes, an enhancer element, a promoter, and a transcription termination sequence, each of which is well known in the art. Optional regulatory control sequences, integration sequences, and useful markers that can be employed are known in the art.

The term "host cell" refers to a cell which can support the replication or expression of the expression vector. Host cells may be prokaryotic cells, such as *E. coli* or eukaryotic cells (*e.g.* yeast, insect, amphibian, bird, or mammalian cells). Creation and isolation of host cell lines comprising a polynucleotide of the invention or capable of producing a cytotoxic protein of the invention can be accomplished using standard techniques known in the art.

Cytotoxic proteins within the scope of the present invention may be variants or derivatives of the cytotoxic proteins described herein that are produced by modifying the polynucleotide encoding a cytotoxic protein by altering one or more amino acids or deleting or inserting one or more amino acids that may render it more suitable to achieve desired properties, such as more optimal expression by a host cell.

### Methods for Using a Cytotoxic Protein or a Pharmaceutical Composition Thereof

Generally, it is an object of the invention to provide pharmacologically active agents, as well as compositions comprising the same, that can be used in the prevention and/or treatment of diseases, disorders, and conditions, such as certain cancers, tumors, immune disorders, or further pathological conditions mentioned herein. Accordingly, the present invention provides an *in vitro* method of using the cytotoxic proteins of the invention for the killing of cells and also provides for the treatment of diseases, disorders, and conditions as described herein.

In particular, it is an object of the invention to provide such pharmacologically active agents, compositions, and/or methods that have certain advantages compared to the agents, compositions, and/or methods that are currently known in the art. Accordingly, the present invention provides methods as defined in the claims of using cytotoxic proteins with specified polypeptide sequences and pharmaceutical compositions thereof. For example, any of the polypeptide sequences in SEQ ID NOs: 1, 2, 3, 4, or 5 can be specifically utilized as a component of the cytotoxic protein used in the following methods.

The present invention provides *in vitro* methods of killing a cell comprising the step of contacting the cell, with a cytotoxic protein of the present invention, as defined in the claims. The cytotoxic proteins and pharmaceutical compositions of the invention can be used to kill a specific cell type upon contacting a cell or cells with one of the claimed compositions of matter. In certain embodiments, a cytotoxic protein or pharmaceutical composition of the present invention can be used to kill specific cell types in a mixture of different cell types, such as mixtures comprising cancer cells, infected cells, and/or hematological cells. In certain embodiments, a cytotoxic protein or pharmaceutical composition of the present invention can be used to kill cancer cells in a mixture of different cell types. In certain embodiments, a cytotoxic protein or pharmaceutical composition of the present invention can be used to kill specific cell types in a mixture of different cell types, such as pre-transplantation tissues. In certain embodiments, a cytotoxic protein or pharmaceutical composition of the present invention can be used to kill specific cell types in a mixture of cell types, such as pre-administration tissue material for therapeutic purposes. In certain embodiments, a cytotoxic protein or pharmaceutical composition of the present invention can be used to selectively kill cells infected by viruses or microorganisms, or otherwise selectively killing cells expressing a particular extracellular target biomolecule, such as a cell surface biomolecule. The cytotoxic proteins and pharmaceutical compositions of the invention have varied applications, including, *e.g*., uses in depleting unwanted cell types from tissues either *in vitro* or *in vivo,* uses in modulating immune responses to treat graft versus host, uses as antiviral agents, uses as antiparasitic agents, and uses in purging transplantation tissues of unwanted cell types.

In certain embodiments, a cytotoxic protein or pharmaceutical composition of the present invention, alone or in combination with other compounds or pharmaceutical compositions can show potent cell-kill activity when administered to a population of cells, *in vitro* or *in vivo* in a subject such as in a patient in need of treatment. By targeting the delivery of enzymatically active Shiga toxin regions using high-affinity immunoglobulin-type binding regions to cancer cell types, this potent cell-kill activity can be restricted to specifically and selectively kill certain cell types within an organism, such as certain cancer cells, neoplastic cells, malignant cells, non-malignant tumor cells, or infected cells.

The present disclosure provides a method of killing a cell in a patient, the method comprising the step of administering to the patient at least one cytotoxic protein of the present invention or a pharmaceutical composition thereof.

Certain embodiments of the cytotoxic protein or pharmaceutical compositions thereof can be used to kill a cancer cell in a patient by targeting an extracellular biomolecule found physically coupled with a cancer or tumor cell. The terms "cancer cell" or "cancerous cell" refers to various neoplastic cells which grow and divide in an abnormally accelerated fashion and will be clear to the skilled person. The term cancer cell includes both malignant and non-malignant cells. Generally, cancers and/or tumors can be defined as diseases, disorders, or conditions that are amenable to treatment and/or prevention. The cancers and tumors (either malignant or non-malignant) which are comprised by cancer cells and/or tumor cells will be clear to the skilled person.

Certain embodiments of the cytotoxic protein or pharmaceutical compositions thereof can be used to kill an immune cell (whether healthy or malignant) in a patient by targeting an extracellular biomolecule found physically coupled with an immune cell.

Certain embodiments of the cytotoxic protein or pharmaceutical compositions thereof can be used to kill an infected cell in a patient by targeting an extracellular biomolecule found physically coupled with an infected cell.

It is within the scope of the present invention to utilize the cytotoxic protein of the invention or pharmaceutical composition thereof for the purposes of *ex vivo* depletion of T cells from isolated cell populations removed from a patient. In one non-limiting example, the cytotoxic protein can be used in a method for prophylaxis of organ transplant rejection wherein the donor organ is perfused prior to transplant with the cytotoxic protein of the invention or a pharmaceutical composition thereof in order to purge the organ of donor T-cells.

It is also within the scope of the present invention to utilize the cytotoxic protein of the invention or pharmaceutical composition thereof for the purposes of purging patient cell populations (*e.g*. bone marrow) of malignant, neoplastic, or otherwise unwanted T-cells and then reinfusing the T-cell depleted material into the patient.

It is also within the scope of the present invention to utilize the cytotoxic protein of the invention or pharmaceutical composition thereof for the purposes of depleting T-cells from a donor cell population as a prophylaxis against graft versus host disease, and induction of tolerance, in a patient to undergo a bone marrow and or stem cell transplant.

Certain embodiments of the cytotoxic protein or pharmaceutical compositions thereof can be used to kill an infected cell in a patient by targeting an extracellular biomolecule found physically coupled with an infected cell.

Additionally, the present disclosure provides a method of treating a disease, disorder, or condition in a patient comprising the step of administering to a patient in need thereof a therapeutically effective amount of at least one of the cytotoxic proteins of the present invention or a pharmaceutical composition thereof. Contemplated diseases, disorders, and conditions that can be treated using this method include cancers, malignant tumors, non-malignant tumors, immune disorders, and microbial infections. Administration of a "therapeutically effective dosage" of a compound of the invention can result in a decrease in severity of disease symptoms, an increase in frequency and duration of disease symptom-free periods, or a prevention of impairment or disability due to the disease affliction.

The therapeutically effective amount of a compound of the present invention will depend on the route of administration, the type of mammal being treated, and the physical characteristics of the specific patient under consideration. These factors and their relationship to determining this amount are well known to skilled practitioners in the medical arts. This amount and the method of administration can be tailored to achieve optimal efficacy, and may depend on such factors as weight, diet, concurrent medication and other factors, well known to those skilled in the medical arts. The dosage sizes and dosing regimen most appropriate for human use may be guided by the results obtained by the present invention, and may be confirmed in properly designed clinical trials. An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are known to the skilled person.

An acceptable route of administration may refer to any administration pathway known in the art, including but not limited to aerosol, enteral, nasal, ophthalmic, oral, parenteral, rectal, vaginal, or transdermal (*e.g*. topical administration of a cream, gel or ointment, or by means of a transdermal patch). "Parenteral administration" is typically associated with injection at or in communication with the intended site of action, including intratumoral injection, infraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal administration.

For administration of a pharmaceutical composition of the invention, the dosage range will generally be from about 0.0001 to 100 mg/kg, and more usually 0.01 to 5 mg/kg, of the host body weight. Exemplary dosages may be 0.25 mg/kg body weight, 1 mg/kg body weight, 3 mg/kg body weight, 5 mg/kg body weight or 10 mg/kg body weight or within the range of 1-10 mg/kg. An exemplary treatment regime is a once or twice daily administration, or a once or twice weekly administration, once every two weeks, once every three weeks, once every four weeks, once a month, once every two or three months or once every three to 6 months. Dosages may be selected and readjusted by the skilled health care professional as required to maximize therapeutic benefit for a particular patient.

Pharmaceutical compositions of the invention will typically be administered to the same patient on multiple occasions. Intervals between single dosages can be, for example, 2-5 days, weekly, monthly, every two or three months, every six months, or yearly. Intervals between administrations can also be irregular, based on regulating blood levels or other markers in the subject or patient. Dosage regimens for a compound of the invention include intravenous administration of 1 mg/kg body weight or 3 mg/kg body weight with the compound administered every two to four weeks for six dosages, then every three months at 3 mg/kg body weight or 1 mg/kg body weight.

A pharmaceutical composition of the present invention may be administered via one or more routes of administration, using one or more of a variety of methods known in the art. As will be appreciated by the skilled worker, the route and/or mode of administration will vary depending upon the desired results. Routes of administration for cytotoxic proteins or pharmaceutical compositions of the invention include, *e.g*. intravenous, intramuscular, intradermal, intraperitoneal, subcutaneous, spinal, or other parenteral routes of administration, for example by injection or infusion at or in communication with the intended site of action (*e.g*. intratumoral injection). In other embodiments, a cytotoxic protein or pharmaceutical composition of the invention may be administered by a non-parenteral route, such as a topical, epidermal or mucosal route of administration, for example, intranasally, orally, vaginally, rectally, sublingually, or topically.

Therapeutic cytotoxic proteins or pharmaceutical compositions of the invention may be administered with one or more of a variety of medical devices known in the art. For example, in one embodiment, a pharmaceutical composition of the invention may be administered with a needleless hypodermic injection device. Examples of well-known implants and modules useful in the present invention are in the art, including *e.g*., implantable micro-infusion pumps for controlled rate delivery; devices for administering through the skin; infusion pumps for delivery at a precise infusion rate; variable flow implantable infusion devices for continuous drug delivery; and osmotic drug delivery systems. These and other such implants, delivery systems, and modules are known to those skilled in the art.

A cytotoxic protein or pharmaceutical composition of the present invention may be administered alone or in combination with one or more other therapeutic or diagnostic agents. A combination therapy may include a cytotoxic protein of the invention or pharmaceutical composition thereof combined with at least one other therapeutic agent selected based on the particular patient, disease or condition to be treated. Examples of other such agents include, *inter alia,* a cytotoxic, anti-cancer or chemotherapeutic agent, an anti-inflammatory or antiproliferative agent, an antimicrobial or antiviral agent, growth factors, cytokines, an analgesic, a therapeutically active small molecule or polypeptide, a single chain antibody, a classical antibody or fragment thereof, or a nucleic acid molecule which modulates one or more signaling pathways, and similar modulating therapeutics which might complement or otherwise be beneficial in a therapeutic or prophylactic treatment regimen.

Treatment of a patient with a cytotoxic protein or pharmaceutical composition of the invention preferably leads to cell death of targeted cells and/or the inhibition of growth of targeted cells. As such, cytotoxic proteins of the invention, and pharmaceutical compositions comprising them, will be useful in methods for treating a variety of pathological disorders in which killing or depleting target cells might be beneficial, such as, *inter alia,* cancer, immune disorders, and infected cells. The present disclosure provides methods for suppressing cell proliferation, and treating cell disorders, including neoplasia, overactive B-cells, and overactive T-cells.

In certain embodiments, cytotoxic proteins and pharmaceutical compositions of the invention can be used to treat or prevent cancers, tumors (malignant and non-malignant), immune disorders, and microbial infections. In a further aspect, the above *ex vivo* method can be combined with the above *in vivo* method to treat or prevent rejection in bone marrow transplant recipients, and for achieving immunological tolerance.

In certain embodiments, the present disclosure provides methods for treating malignancies or neoplasms and other blood cell associated cancers in a mammalian subject, such as a human, the method comprising the step of administering to a subject in need thereof a therapeutically effective amount of a cytotoxic protein or pharmaceutical composition of the invention.

The cytotoxic proteins and pharmaceutical compositions of the invention have varied applications, including, *e.g*., uses in removing unwanted T-cells, uses in modulating immune responses to treat graft versus host, uses as antiviral agents, uses as antimicrobial agents, and uses in purging transplantation tissues of unwanted cell types. The cytotoxic proteins and pharmaceutical compositions of the present invention are commonly anti-neoplastic agents - meaning they are capable of treating and/or preventing the development, maturation, or spread of neoplastic or malignant cells by inhibiting the growth and/or causing the death of cancer or tumor cells.

In certain embodiments, a cytotoxic protein or pharmaceutical composition of the present invention can be for use in treating a B cell-, plasma cell- or antibody- mediated disease or disorder, such as for example leukemia, lymphoma, myeloma, amyloidosis, ankylosing spondylitis, asthma, Crohn's disease, diabetes, graft rejection, graft versus host disease, Hashimoto's thyroiditis, hemolytic uremic syndrome, HIV-related disease, lupus erythematosis, multiple sclerosis, polyarteritis, psoriasis, psoriatic arthritis, rheumatoid arthritis, scleroderma, septic shock, Sjögren's syndrome, ulcerative colitis, and vasculitis.

In another aspect, certain embodiments of the cytotoxic proteins and pharmaceutical compositions of the present invention are antimicrobial agents - meaning they are capable of treating and/or preventing the acquisition, development, or consequences of microbiological pathogenic infections, such as caused by viruses, bacteria, fungi, prions, or protozoans.

It is within the scope of the present invention to provide a prophylaxis or treatment for diseases or conditions mediated by T-cells or B-cells by using the cytotoxic protein of the invention, or a pharmaceutical composition thereof, in a patient for the purpose of systematically killing T-cells or B-cells in the patient. This usage is compatible with preparing or conditioning a patient for bone marrow transplantation, stem cell transplantation, tissue transplantation, or organ transplantation, regardless of the source of the transplanted material, *e.g*. human or non-human sources.

It is within the scope of the present invention to provide a bone marrow recipient for prophylaxis or treatment of host versus graft disease via the targeted cell-killing of host T-cells using a cytotoxic protein or pharmaceutical composition of the present invention.

The cytotoxic proteins and pharmaceutical compositions of the present invention can be for use in a method of treating cancer comprising administering to a patient, in need thereof, a therapeutically effective amount of the cytotoxic protein or a pharmaceutical composition of the present invention. In certain embodiments of the present invention, the cancer being treated is selected from the group consisting bone cancer (such as multiple myeloma or Ewing's sarcoma), breast cancer, central/peripheral nervous system cancer (such as brain cancer, neurofibromatosis, or glioblastoma), gastrointestinal cancer (such as stomach cancer or colorectal cancer), germ cell cancer (such as ovarian cancers and testicular cancers, glandular cancer (such as pancreatic cancer, parathyroid cancer, pheochromocytoma, salivary gland cancer, or thyroid cancer), head-neck cancer (such as nasopharyngeal cancer, oral cancer, or pharyngeal cancer), hematological cancers (such as leukemia, lymphoma, or myeloma), kidney-urinary tract cancer (such as renal cancer and bladder cancer), liver cancer, lung/pleura cancer (such as mesothelioma, small cell lung carcinoma, or non-small cell lung carcinoma), prostate cancer, sarcoma (such as angiosarcoma, fibrosarcoma, Kaposi's sarcoma, or synovial sarcoma), skin cancer (such as basal cell carcinoma, squamous cell carcinoma, or melanoma), and uterine cancer.

The cytotoxic proteins and pharmaceutical compositions of the present invention can be for use in a method of treating an immune disorder comprising administering to a patient, in need thereof, a therapeutically effective amount of the cytotoxic protein or a pharmaceutical composition of the present invention. In certain embodiments of the present invention, the immune disorder is related to an inflammation associated with a disease selected from the group consisting amyloidosis, ankylosing spondylitis, asthma, Crohn's disease, diabetes, graft rejection, graft versus host disease, Hashimoto's thyroiditis, hemolytic uremic syndrome, HIV-related disease, lupus erythematosis, multiple sclerosis, polyarteritis, psoriasis, psoriatic arthritis, rheumatoid arthritis, scleroderma, septic shock, Sjögren's syndrome, ulcerative colitis, and vasculitis.

Among certain embodiments of the present invention is a cytotoxic protein as a component of a medicament for use in the treatment or prevention of a cancer, tumor, immune disorder, or microbial infection. For example, immune disorders presenting on the skin of a patient may be treated with such a medicament in efforts to reduce inflammation. In another example, skin tumors may be treated with such a medicament in efforts to reduce tumor size or eliminate the tumor completely.

Among certain embodiment of the present disclosure is a method of using a cytotoxic protein or pharmaceutical composition of the invention to label or detect the interiors of cancer cells and/or immune cell types. Based on the ability of the cytotoxic proteins and pharmaceutical compositions of the invention to enter specific cell types and route within cells via retrograde intracellular transport, the interior compartments of specific cell types are labeled for detection. This can be performed on cells *in situ* within a patient or on cells and tissues removed from an organism, *e.g*. biopsy material.

The present invention is further illustrated by the following non-limiting examples of selectively cytotoxic proteins comprising Shiga toxin effector regions derived from A Subunits of members of the Shiga toxin family and immunoglobulin-type binding regions capable of binding extracellular target biomolecules physically coupled to specific cell types.

### EXAMPLES

The following examples demonstrate certain embodiments of the present invention. However, it is to be understood that these examples are for illustration purposes only and do not intend, nor should any be construed, to be wholly definitive as to conditions and scope of this invention, which is defined in the claims. The examples were carried out using standard techniques, which are well known and routine to those of skill in the art, except where otherwise described in detail.

The following examples demonstrate the ability of exemplary cytotoxic proteins to selectively kill cells physically coupled with an extracellular target biomolecule of the immunoglobulin-type binding region. The exemplary cytotoxic proteins bound to target biomolecules expressed by targeted cell types and entered the targeted cells. The internalized cytotoxic proteins routed their Shiga toxin effector region to the cytosol to inactivate ribosomes and subsequently caused the apoptotic death of the targeted cells. Thus, the exemplary cytotoxic proteins were capable of targeting specific cell types by virtue of their immunoglobulin-type bind regions bringing the cytotoxic proteins in close proximity with cell types that are physically coupled with a target biomolecule of the immunoglobulin-type binding region.

One exemplary embodiment comprises a Shiga toxin A Subunit fragment combined with a single-chain, variable fragment, binding region capable of binding CD38 with high affinity. This exemplary cytotoxic protein is capable of selectively killing cells that express CD38 on their surface. A second exemplary cytotoxic protein comprises a Shiga toxin A Subunit fragment combined with a single-domain antibody fragment, binding region capable of binding HER2 with high affinity. This second exemplary cytotoxic protein is capable of selectively killing cells that express HER2 on their surface. A third exemplary embodiment comprises a Shiga toxin A Subunit fragment combined with a single-domain antibody fragment, binding region capable of binding CD20 with high affinity. This third exemplary cytotoxic protein is capable of selectively killing cells that express CD20 on their surface. Other exemplary cytotoxic proteins include those with binding regions targeting Epstein-Barr antigens, Leishmania antigens, neurotensin receptors, epidermal growth factor receptors, and CCR5.

### Example 1. A CD38-targeted, cytotoxic protein derived from the A Subunit of Shiga-like toxin-1 ("αCD38-scFv fused with SLT-1A")

### Construction, Production, and Purification of the Cytotoxic Protein "αCD38-scFv fused with SLT-1A"

First, a Shiga toxin effector region and an immunoglobulin-type binding region were designed or selected. In this example, the Shiga toxin effector region was derived from the A subunit of Shiga-like Toxin 1 (SLT-1A). A polynucleotide was obtained that encoded amino acids 1-251 of SLT-1A (Cheung M et al., Mol Cancer 9: 28 (2010)). An immunoglobulin-type binding region αCD38scFv was derived from the monoclonal antibody αCD38 HB7 (Peng et al., Blood 101: 2557-62 (2003); *see also* GenBank Accession BD376144, National Center for Biotechnology Information, U.S.) such that a single-chain variable fragment (scFv) is created with the two immunoglobulin variable regions (V_{L} and V_{H}) separated by a linker known in the art.

Second, the immunoglobulin-type binding region and Shiga toxin effector region were linked together to form a fused protein. In this example, a polynucleotide encoding the Shiga toxin effector region comprising amino acids 1-251 of SEQ ID NO:1 was cloned in frame with a polynucleotide encoding a linker derived from a murine IgG3 molecule and in frame with a polynucleotide encoding the immunoglobulin-type binding region αCD38scFv comprising amino acids 269-508 of SEQ ID NO:4. In certain experiments, the full-length coding sequence of the cytotoxic protein of this example began with a polynucleotide encoding a Strep-tag® to facilitate detection and purification. The polynucleotide sequence encoding the cytotoxic protein "αCD38-scFv fused with SLT-1A" of this example was codon optimized for efficient expression in *E. coli* using services from DNA 2.0, Inc. (Menlo Park, CA, U.S.).

Third, a fusion protein was produced by expressing the polynucleotide encoding the cytotoxic protein αCD38-scFv fused with SLT-1A. Expression of the "αCD38-scFv fused with SLT-1A" cytotoxic protein (SEQ ID NO:4) was accomplished using both bacterial and cell-free, protein translation systems.

In this example of "αCD38-scFv fused with SLT-1A" production by an *E. coli* expression system, the polynucleotide "insert" sequence encoding "αCD38-scFv fused with SLT-1A" was cloned into the pTxb1 vector (New England Biolabs, Ipswich, MA, U.S.) using standard procedures to produce a polynucleotide sequence encoding the cytotoxic protein "αCD38-scFv fused with SLT-1A" ligated in frame to polynucleotide sequences encoding the amino-terminal intein of the vector. The plasmid insert polynucleotide sequence was verified by Sanger sequencing (Functional Biosciences, Madison, WI, U.S.) and transformed into the T7 Shuffle® cells (New England Biolabs, Ipswich, MA, U.S.). The "αCD38-scFv fused with SLT-1A" protein was produced and purified according to the IMPACT™ (Intein Mediated Purification with an Affinity Chitin-binding Tag) system manual (New England Biolabs, Ipswich, MA, U.S.). Purification was accomplished using standard techniques known in the art, such as using immobilized targets of the Strep-tag® or the immunoglobulin-type binding region.

In this example of "αCD38-scFv fused with SLT-1A" production by a cell-free, protein translation system, the polynucleotide "insert" sequence encoding "αCD38-scFv fused with SLT-1A" was cloned into the pIVEX2.3 vector with a stop codon directly after the coding region using the In-Fusion® HD Cloning Kit (Clonetech, Mountain View, CA, U.S.) according to the manufacturer's instructions. The plasmid insert polynucleotide sequence was verified by Sanger sequencing (Functional Biosciences, Madison, WI, U.S.). "αCD38-scFv fused with SLT-1A" protein was produced using the rapid translation system 5 Prime™ RTS 100 *E. coli* Disulfide Kit (5 Prime, Gaithersburg, MD, U.S.) according to the manufacturer's instructions. Purification was accomplished using standard techniques known in the art, such as using immobilized targets of the Strep-tag® or the immunoglobulin-type binding region.

### Determining the Maximum Specific Binding (Bₘₐₓ) and Equilibrium Binding Constant (K_{D}) of "αCD38-scFv fused with SLT-1A" Binding Targeted Cell Types

The binding characteristics of the "αCD38-scFv fused with SLT-1A" protein produced as described above were determined by a fluorescence-based, flow-cytometry assay. Samples containing CD38 positive (+) cells and CD38 negative (-) cells were suspended in phosphate buffered saline (IX PBS) (Hyclone Brand, Fisher Scientific, Waltham, MA, U.S.) containing 1 percent bovine serum albumin (BSA) (Calbiochem, San Diego, CA, U.S.), hereinafter referred to as "1X PBS+1%BSA" and incubated for 1 hour at 4 degrees Celsius (°C) with 100 µL of various dilutions of the "αCD38-scFv fused with SLT-1A" protein to be assayed. The highest concentrations of "αCD38-scFv fused with SLT-1A" protein was selected to lead to saturation of the binding reaction. After the one hour incubation, cell samples were washed twice with 1X PBS+1%BSA. The cell samples were incubated for 1 hour at 4°C with 100 µL of 1X PBS+1%BSA containing 0.3 µg of αStrep-tag® mAb-FITC (# A01736-100, Genscript, Piscataway, NJ, U.S.).

The cell samples were next washed twice with IX PBS+1%BSA, resuspended in 200 µL of 1X PBS and subjected to fluorescence-based, flow cytometry. The mean fluorescence intensity (MFI) data for all the samples was obtained by gating the data using a FITC-only sample as a negative control. Graphs were plotted of MFI versus "concentration of cells" using Prism software (GraphPad Software, San Diego, CA, U.S.). Using the Prism software function of one-site binding [Y = Bₘₐₓ^{∗}X / (K_{D} + X)] under the heading binding-saturation, the Bₘₐₓ and K_{D} were calculated using baseline corrected data. Bₘₐₓ is the maximum specific binding reported in MFI. K_{D} is the equilibrium binding constant, reported in nanomolar (nM).

The Bₘₐₓ for "αCD38-scFv fused with SLT-1A" binding to CD38+ cells was measured to be about 100,000 MFI with a K_{D} of about 13 nM (Table 1), whereas there was no binding to CD38- cells observed in this assay.

**Table 1. Binding Characteristics: Representative values for Bₘₐₓ and K_{D} for exemplary cytotoxic proteins**

| | | **Target Positive Cells** | | **Target Negative Cells** | |
|---|---|---|---|---|---|
| Cytotoxic Protein | target biomolecule | Bₘₐₓ (MFI) | K_{D} (nM) | Bₘₐₓ (MFI) | K_{D} (nM) |
| **αCD38-scFv fused with SLT-1A** | CD38 | 104,000 | 13.40 | no binding | no binding |
| **αHER2-V_{HH} fused with SLT-1A** | HER2 | 113,000 | 2.35 | 11,700 | 811 |
| **αCD20-scFv fused with SLT-1A** | CD20 | 139,000 | 82.50 | 15,800 | 1,050 |

### Determining the Half-Maximal Inhibitory Concentration (IC₅₀) of "αCD38-scFv fused with SLT-1A" to Eukaryotic Ribosomes in vitro

The ribosome inactivation capabilities of "αCD38-scFv fused with SLT-1A" was determined in a cell-free, *in vitro* protein translation assay using the TNT® Quick Coupled Transcription/Translation Kit (L1170 Promega, Madison, WI, U.S.). The kit includes Luciferase T7 Control DNA and TNT® Quick Master Mix. The ribosome activity reaction was prepared according to the manufacturer's instructions to create "TNT" reaction mixtures.

A series of 10-fold dilutions of "αCD38-scFv fused with SLT-1A" to be tested was prepared in appropriate buffer and a series of identical TNT reaction mixture components was created for each dilution of "αCD38-scFv fused with SLT-1A." Each sample in the dilution series of "αCD38-scFv fused with SLT-1A" protein was combined with each of the TNT reaction mixtures along with the Luciferase T7 Control DNA. The test samples were incubated for 1.5 hours at 30°C. After the incubation, Luciferase Assay Reagent (E1483 Promega, Madison, WI, U.S.) was added to all test samples and the amount of luciferase protein translation was measured by luminescence according to the manufacturer instructions. The level of translational inhibition was determined by non-linear regression analysis of log-transformed concentrations of total protein versus relative luminescence units. Using statistical software (GraphPad Prism, San Diego, CA, U.S.), the half maximal inhibitory concentration (IC₅₀) value was calculated for each sample. Then, the data were normalized by calculating the "percent of SLT-1A-only control protein" using the Prism software function of log(inhibitor) vs. response (three parameters) [Y = Bottom + ((Top-Bottom) / (1+10^(X-LogIC50)))] under the heading dose-response-inhibition. The IC₅₀ for experimental proteins and SLT-1A-only control protein were calculated. The percent of SLT-1A-only control protein was calculated by [(IC50 of SLT-1A control protein / IC50 of experimental protein) x 100].

The inhibitory effect of "αCD38-scFv fused with SLT-1A" on cell-free protein synthesis was strong. Dose dependence experiments determined that the IC₅₀ of "αCD38-scFv fused with SLT-1A" on protein synthesis in this cell-free assay was about 14 picomolar (pM) or within 10% of the SLT-1A-only positive control (Table 2).

**Table 2. Ribosome Inactivation: Representative half-maximal inhibitory concentrations (IC₅₀) for exemplary cytotoxic proteins**

| Cytotoxic Protein | IC₅₀ (pM) | IC₅₀ of SLT-1A-only positive control (pM) | Percent of IC₅₀ of SLT-1A control protein |
|---|---|---|---|
| **αCD38-scFv fused with SLT-1A** | 13.7 | 15.0 | 110% |
| **αHER2-V_{HH} fused with SLT-1A** | 12.5 | 15.0 | 120% |
| **αCD20-scFv fused with SLT-1A** | 38.3 | 31.2 | 81% |

### Determining the Selective Cytotoxicity and Half-Maximal Cytotoxic Concentration (CD₅₀) of "αCD38-scFv fused with SLT-1A" Using a Cell-Kill Assay

The cytotoxicity characteristics of "αCD38-scFv fused with SLT-1A" was determined by the following cell-kill assay. This assay determines the capacity of a cytotoxic protein to kill cells expressing the target biomolecule of its immunoglobulin-type binding region as compared to cells that do not express the target biomolecule. Cells were plated (2 x 10³ per well) in 20 µL media in 384 well plates. The "αCD38-scFv fused with SLT-1A" protein was diluted either 5-fold or 10-fold in a 1X PBS and 5 µL of the dilutions were added to the cells. Control wells containing only media were used for baseline correction. The cell samples were incubated with "αCD38-scFv fused with SLT-1A," or just buffer, for 3 days at 37°C and in an atmosphere of 5% carbon dioxide (CO₂). The total cell survival or percent viability was determined using a luminescent readout using the CellTiter-Glo® Luminescent Cell Viability Assay (G7573 Promega Madison, WI, U.S.) according to the manufacturer's instructions. The Percent Viability of experimental wells was calculated using the following equation: (Test RLU - Average Media RLU) / (Average Cells RLU - Average Media RLU) ^{∗} 100. Log polypeptide concentration versus Percent Viability was plotted in Prism (GraphPad Prism, San Diego, CA, U.S.) and log (inhibitor) vs. normalized response (variable slope) analysis was used to determine the half-maximal cytotoxic concentration (CD₅₀) value for "αCD38-scFv fused with SLT-1A."

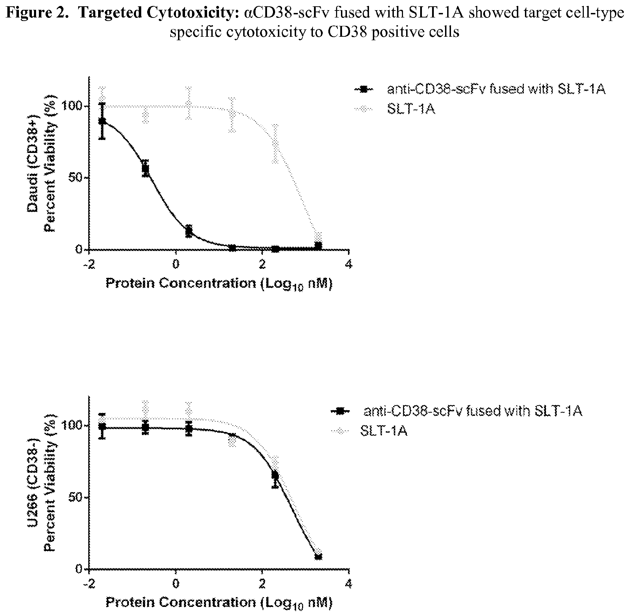

. Dose dependence experiments determined that the CD₅₀ of the "αCD38-scFv fused with SLT-1A" protein was about 0.2-0.7 nM for CD38 + cells depending on the cell line as compared to 470 nM for a CD38- cell line, which was similar to the CD₅₀ for the SLT-1A-only negative control (Table 3; Figure 2). The CD₅₀ of the "αCD38-scFv fused with SLT-1A" was about 700-3000 fold greater (less cytotoxic) for cells not physically coupled with the extracellular target biomolecule CD38 as compared to cells which were physically coupled with the extracellular target biomolecule CD38, *e.g.* cell lines which express CD38 on their cell surface.

**Table 3. Selective Cytotoxicity: Representative half-maximal cytotoxic concentrations (CD₅₀) for αCD38-scFv fused with SLT-1A**

| | | CD₅₀ (nM) | |
|---|---|---|---|
| **Cell Line** | **CD38 status** | **αCD38-scFv fused with SLT-1A** | **SLT-1A only negative control** |
| Daudi | positive | 0.28 | 750 |
| Raji | positive | 0.68 | 1,100 |
| ST486 | positive | 0.21 | 940 |
| BC-1 | positive | 0.18 | 510 |
| U226 | negative | 470.00 | 490 |

### Example 2. A HER2-targeted, cytotoxic protein derived from the A Subunit of Shiga-like toxin 1 (αHER2-V_{HH} fused with SLT-1A)

### Construction, Production, and Purification of "αHER2-V_{HH} fused with SLT-1A"

In this example, the Shiga toxin effector region was derived from the A subunit of Shiga-like Toxin 1 (SLT-1A). A polynucleotide was obtained that encoded amino acids 1-251 of SLT-1A (Cheung M et al., Mol Cancer 9: 28 (2010)). The immunoglobulin-type binding region is αHER2 V_{HH} derived from a single-domain variable region of the camelid antibody (V_{HH}) protein 5F7, as described in U.S. Patent Application Publication 2011/0059090.

Second, the immunoglobulin-type binding region and Shiga toxin effector region were linked together to form a fused protein. In this example, a polynucleotide encoding the αHER2-V_{HH} variable region derived from protein 5F7 comprising amino acids 1-119 of SEQ ID NO: 5 was cloned in frame with a polynucleotide encoding a linker known in the art and in frame with a polynucleotide encoding the Shiga toxin effector region comprising amino acids 1-251 of SEQ ID NO:1. In certain experiments, the full-length coding sequence of the cytotoxic protein of this example began with a polynucleotide encoding a Strep-tag® to facilitate detection and purification. The polynucleotide sequence encoding the cytotoxic protein "αHER2-V_{HH} fused with SLT-1A" of this example was codon optimized for efficient expression in *E. coli* using services from DNA 2.0, Inc. (Menlo Park, CA, U.S.).

The "αHER2-V_{HH} fused with SLT-1A" fusion protein (SEQ ID NO: 5) was produced by expression from the polynucleotide encoding it. Expression of the "αHER2-V_{HH} fused with SLT-1A" cytotoxic protein was accomplished using both bacterial and cell-free, protein translation systems.

In this example of "αHER2-V_{HH} fused with SLT-1A" production by an *E. coli* expression system, the polynucleotide "insert" sequence encoding "αHER2-V_{HH} fused with SLT-1A" was cloned into the pTxb1 vector (New England Biolabs, Ipswich, MA, U.S.) using standard procedures to produce a polynucleotide sequence encoding the cytotoxic protein "αHER2-V_{HH} fused with SLT-1A" ligated in frame to polynucleotide sequences encoding the amino-terminal intein of the vector. The plasmid insert polynucleotide sequence was verified by Sanger sequencing (Functional Biosciences, Madison, WI, U.S.) and transformed into the T7 Shuffle® cells (New England Biolabs, Ipswich, MA, U.S.). The "αHER2-V_{HH} fused with SLT-1A" protein was produced and purified according to the IMPACT™ (Intein Mediated Purification with an Affinity Chitin-binding Tag) system manual (New England Biolabs, Ipswich, MA, U.S.). Purification was accomplished using standard techniques known in the art, such as using immobilized targets of the Strep-tag® or the immunoglobulin-type binding region.

In this example of "αHER2-V_{HH} fused with SLT-1A" production by a cell-free, protein translation system, the polynucleotide "insert" sequence encoding "αHER2-V_{HH} fused with SLT-1A" was cloned into the pIVEX2.3 vector with a stop codon directly after the coding region using the In-Fusion® HD Cloning Kit (Clonetech, Mountain View, CA, U.S.) according to the manufacturer's instructions. The plasmid insert polynucleotide sequence was verified by Sanger sequencing (Functional Biosciences, Madison, WI, U.S.). "αHER2-V_{HH} fused with SLT-1A" protein was produced using the rapid translation system 5 Prime™ RTS 100 *E. coli* Disulfide Kit (5 Prime, Gaithersburg, MD, U.S.) according to the manufacturer's instructions. Purification was accomplished using standard techniques known in the art, such as using immobilized targets of the Strep-tag® or the immunoglobulin-type binding region.

### Determining the Maximum Specific Binding (Bₘₐₓ) and Equilibrium Binding Constant (K_{D}) of "αHER2-V_{HH} fused with SLT-1A" to a Targeted Cell Type

The binding characteristics of the "αHER2-V_{HH} fused with SLT-1A" protein produced as described above were determined by a fluorescence-based, flow-cytometry assay. Samples containing HER2 positive (+) cells and HER2-negative (-) cells were suspended in 1X PBS+1%BSA and incubated for 1 hour at 4°C with 100 µL of various dilutions of the "αHER2-V_{HH} fused with SLT-1A" protein to be assayed. The highest concentrations of "αHER2-V_{HH} fused with SLT-1A" protein was selected to lead to saturation of the binding reaction. After the one hour incubation, cell samples were washed twice with 1X PBS+1%BSA. The cell samples were incubated for 1 hour at 4°C with 100 µL of 1X PBS+1%BSA containing 0.3 µg of αStrep-tag® mAb-FITC (# A01736-100, Genscript, Piscataway, NJ, U.S.).

The cell samples were next washed twice with 1X PBS+1%BSA, resuspended in 200 µL of 1X PBS and subjected to fluorescence-based, flow cytometry. The mean fluorescence intensity (MFI) data for all the samples was obtained by gating the data using a FITC-only sample as a negative control. Graphs were plotted of MFI versus "concentration of cells" using Prism software (GraphPad Software, San Diego, CA, U.S.). Using the Prism software function of one-site binding [Y = Bₘₐₓ^{∗}X / (K_{D} + X)] under the heading binding-saturation, the Bₘₐₓ and K_{D} were calculated using baseline corrected data. Bₘₐₓ is the maximum specific binding reported in MFI. K_{D} is the equilibrium binding constant, reported in nM.

The Bₘₐₓ for "αHER2-V_{HH} fused with SLT-1A" binding to HER2+ cells was measured to be about 110,00 MFI with a K_{D} of about 2.4 nM (Table 1); whereas the Bₘₐₓ for "αHER2-V_{HH} fused with SLT-1A" binding to HER2- cells was measured to be about 12,000 MFI with a K_{D} of about 810 nM (Table 1).

### Determining the Half-Maximal Inhibitory Concentration (IC₅₀) of "αHER2-V_{HH} fused with SLT-1A" to Eukaryotic Ribosomes in vitro

The ribosome inactivation capabilities of "αHER2-V_{HH} fused with SLT-1A" was determined in a cell-free, *in vitro* protein translation assay using the TNT® Quick Coupled Transcription/Translation Kit (L1170 Promega, Madison, WI, U.S.). The kit includes Luciferase T7 Control DNA and TNT® Quick Master Mix. The ribosome activity reaction was prepared according to the manufacturer's instructions to create "TNT" reaction mixtures.

A series of 10-fold dilutions of "αHER2-V_{HH} fused with SLT-1A" to be tested were prepared in appropriate buffer and a series of identical TNT reaction mixture components were created for each dilution of "αHER2-V_{HH} fused with SLT-1A." Each sample in the dilution series of "αHER2-V_{HH} fused with SLT-1A" protein was combined with each of the TNT reaction mixtures along with the Luciferase T7 Control DNA. The test samples were incubated for 1.5 hours at 30°C. After the incubation, Luciferase Assay Reagent (E1483 Promega, Madison, WI, U.S.) was added to all test samples and the amount of luciferase protein translation was measured by luminescence according to the manufacturer instructions. The level of translational inhibition was determined by non-linear regression analysis of log-transformed concentrations of total protein versus relative luminescence units. Using statistical software (GraphPad Prism, San Diego, CA, U.S.), the half maximal inhibitory concentration (IC₅₀) value was calculated for each sample. Then, the data were normalized by calculating the "percent of SLT-1A-only control protein" using the Prism software function of log(inhibitor) vs. response (three parameters) [Y = Bottom + ((Top-Bottom) / (1+10^(X-LogIC50)))] under the heading dose-response-inhibition. The IC₅₀ for experimental proteins and SLT-1A-only positive control protein were calculated. The percent of SLT-1A-only positive control protein was calculated by [(IC50 of SLT-1A control protein / IC50 of experimental protein) x 100].

The inhibitory effect of "αHER2-V_{HH} fused with SLT-1A" on cell-free protein synthesis was strong. Dose dependence experiments determined that the IC₅₀ of "αHER2-V_{HH} fused with SLT-1A" on protein synthesis in this cell-free assay was about 13 pM or similar to the SLT-1A-only positive control (Table 2).

### Determining the Selective Cytotoxicity and Half-Maximal Cytotoxic Concentration (CD₅₀) of "αHER2-V_{HH} fused with SLT-1A" Using a Cell-Kill Assay

The cytotoxicity characteristics of "αHER2-V_{HH} fused with SLT-1A" was determined by the following cell-kill assay. This assay determines the capacity of a cytotoxic protein to kill cells expressing the target biomolecule of its immunoglobulin-type binding region as compared to cells that do not express the target biomolecule. Cells were plated (2 x 10³ per well) in 20 µL media in 384 well plates. The "αHER2-V_{HH} fused with SLT-1A" protein was diluted either 5-fold or 10-fold in a 1X PBS and 5 µL of the dilutions were added to the cells. Control wells containing only media were used for baseline correction. The cell samples were incubated with "αHER2-V_{HH} fused with SLT-1A," or just buffer, for 3 days at 37°C and in an atmosphere of 5% CO₂. The total cell survival or percent viability was determined using a luminescent readout using the CellTiter-Glo® Luminescent Cell Viability Assay (G7573 Promega Madison, WI, U.S.) according to the manufacturer's instructions. The Percent Viability of experimental wells was calculated using the following equation: (Test RLU - Average Media RLU) / (Average Cells RLU - Average Media RLU) ^{∗} 100. Log polypeptide concentration versus Percent Viability was plotted in Prism (GraphPad Prism, San Diego, CA, U.S.) and log (inhibitor) vs. normalized response (variable slope) analysis was used to determine the half-maximal cytotoxic concentration (CD₅₀) value for "αHER2-V_{HH} fused with SLT-1A."

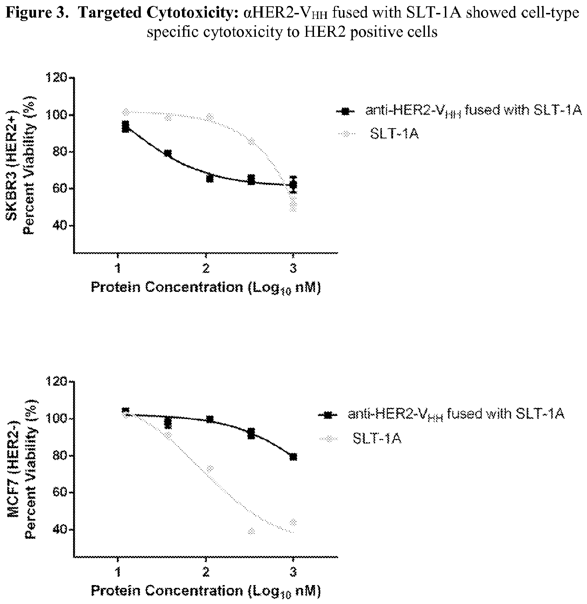

. Dose dependence experiments determined that the CD₅₀ of the "αHER2-V_{HH} fused with SLT-1A" protein was about 15 nM for a HER2 + cell line as compared to about 1700 nM for a HER2- cell line (Table 4; Figure 3). The CD₅₀ of the "αHER2-V_{HH} fused with SLT-1A" protein was about 110 fold greater (less cytotoxic) for cells not physically coupled with the extracellular target biomolecule HER2 as compared to cells which were physically coupled with the extracellular target biomolecule HER2, *e.g.* a cell line which expresses HER2 on its cell surface.

**Table 4. Selective Cytotoxicity: Representative half-maximal cytotoxic concentrations (CD₅₀) for αHER2-V_{HH} fused with SLT-1A**

| | | CD₅₀ (nM) | |
|---|---|---|---|
| **Cell Line** | **HER2 status** | **αHER2-V_{HH} fused with SLT-1A** | **SLT-1A only negative control** |
| SKBR3 | positive | 14.9 | 2610.0 |
| MCF7 | negative | 1650.0 | 92.0 |

### Example 3. A CD20-targeted, cytotoxic protein derived from the A Subunit of Shiga-like toxin 1 (αCD20-scFv fused with SLT-1A)

In this example, the Shiga toxin effector region is derived from the A subunit of Shiga-like Toxin 1 (SLT-1A). An immunoglobulin-type binding region αCD20-scFv was designed as a recombinant scFv derived from the 1H4 CD20 monoclonal antibody (Haisma et al. (1999), Blood 92: 184-90), which comprises an immunoglobulin-type binding region capable of binding human CD20. These heterologous regions were combined as described above in Examples 1-2. Briefly, a polynucleotide encoding the recombinant scFv derived from 1H4 CD20 monoclonal antibody was cloned in frame with a polynucleotide derived encoding a "murine hinge" from a murine IgG3 molecule and in frame with a polynucleotide encoding SLT-1A (residues 1-251 of SEQ ID NO:1). A different CD20-targeted cytotoxic protein, "αCD20scFv fused with SLT-1A" version 2 was constructed and produced in a similar manner that differed only in the linker region between the αCD20-scFv and the SLT1-A derived Shiga toxin effector region. Expression of both versions of "αCD20-scFv fused with SLT-1A" cytotoxic protein was accomplished using either bacterial and/or cell-free, protein translation systems as described in the previous examples.

The cell binding characteristics of the cytotoxic protein "αCD20scFv fused with SLT-1A" was determined by a fluorescence-based flow cytometry assay as described in Examples 1-2. Over multiple experiments, the K_{D} of "αCD20-scFv fused with SLT-1A" for Raji (CD20+) cells was determined to be about 80-100 nM. In one experiment, the Bₘₐₓ for the "αCD20-scFv fused with SLT-1A" was measured to be about 140,000 MFI with a K_{D} of about 83 nM, whereas there was no meaningful binding to CD20- cells observed in this assay (Table 1).

The inhibitory effect of "αCD20-scFv fused with SLT-1A" on cell-free protein synthesis was strong. Multiple experiments determined that the IC₅₀ of "αCD20-scFv fused with SLT-1A" was around 50 pM. In one experiment, the IC₅₀ of "αCD20-scFv fused with SLT-1A" on protein synthesis was about 38 pM or within 19% of the SLT-1A-only positive control (Table 2).

The cytotoxicity profiles of "αCD20-scFv fused with SLT-1A" was determined by a cell kill assay as described in examples 1-2, but targeting CD20+ cells. Over multiple experiments, "αCD20-scFv fused with SLT-1A" demonstrated CD20-specific cell kill with 10 to 1000-fold specificity compared to cell kill of CD20 negative cell lines. The CD₅₀ of the "αCD20-scFv fused with SLT-1A" protein was measured to be about 3-70 nM for CD20+ cells, depending on the cell line, as compared to over 600-2,000 for CD20- cell lines (Table 5). The CD₅₀ of the "αCD20-scFv fused with SLT-1A" protein was over 100 to 400 fold greater (less cytotoxic) for cells not physically coupled with a CD20 extracellular target as compared to cells physically coupled with the extracellular target biomolecule CD20.

**Table 5. Selective Cytotoxicity: Representative half-maximal cytotoxic concentrations (CD₅₀) for αCD20-scFv fused with SLT-1A**

| | | CD₅₀ (nM) | |
|---|---|---|---|
| **Cell Line** | **HER2 status** | **αCD20-scFv fused with SLT-1A** | **SLT-1A only negative control** |
| Daudi | positive | 5.6 | 650 |
| Raji | positive | 2.8 | 1,100 |
| ST486 | positive | 3.7 | 940 |
| Ramos | positive | 27.0 | 470 |
| BC-1 | negative | 2,000 | 160 |
| Jurkat | negative | 1,400 | 120 |
| U226 | negative | 2,500 | 960 |

### Determining the Targeted Cytotoxicity of a Cytotoxic Protein Using in vivo Xenograft Studies

Two xenograft model systems based on an immuno-compromised mouse strains were used to study the ability of the cytotoxic proteins "αCD20-scFv fused with SLT-1A" versions 1 and 2 to kill CD20+ tumor cells *in vivo* and in a tumor environment over time and for various dosages. These xenograft model systems rely on well-characterized mouse strains that lack graft-versus-host responses, among other immune system deficiencies. First, an intravenous tumor model was studied using SCID (severe combined immune deficiency) mice to create disseminated tumors throughout the mice in order to test the *in vivo* effects of "αCD20-scFv fused with SLT-1A" on human tumor cells. Second, a subcutaneous tumor model was studied using BALBc/nude mice to create subcutaneous tumors on the mice, again in order to test the *in vivo* effects "αCD20-scFv fused with SLT-1A" on human tumor cells.

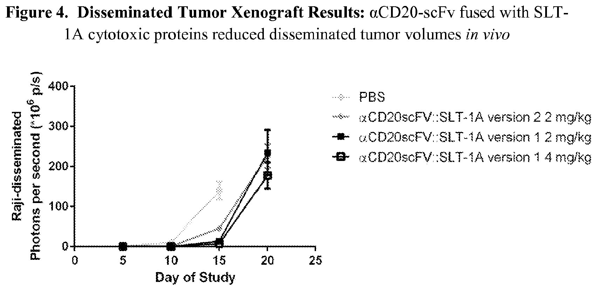

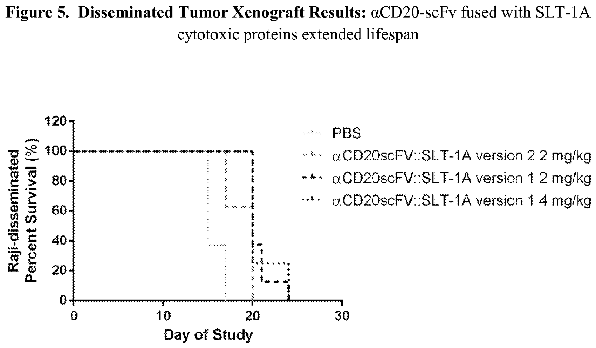

For the first xenograft system, thirty-two C.B.-17 SCID mice (in four groups of eight animals) were challenged with 1 x 10⁷ Raji-luc human lymphoma derived cells (Molecular Imaging, Ann Arbor, MI, U.S.) in 200 µL PBS. On days 5-9 and 12-16 following tumor challenge the groups received through intravenous administration of the following by group: Group 1: PBS; Group 2: "αCD20-scFv fused with SLT-1A" version 2 at a dose of 2mg/kg; Group 3: "αCD20-scFv fused with SLT-1A" version 1 at a dose of 2mg/kg; and Group 4: αCD20scFv::SLT-1A version 1 at a dose of 4mg/kg (Days 5-9 only). Bioluminescence, in 1 X 10⁶ photons/second units (p/s), was measured on days 5, 10, 15, and 20 using a Caliper IVIS 50 optical imaging system (Perkin Elmer, Waltham, MA, U.S.). Figure 4 shows how both versions of "αCD20-scFv fused with SLT-1A," and "αCD20-scFv fused with SLT-1A" version 1 at both dosage levels, resulted in statistically significant less total bioluminescence compared to the PBS control. The decrease in total bioluminescence was reflective of statistically significant reductions in disseminated tumor burdens after treatment with a cytotoxic protein of the invention. Figure 5 indicates a statistically significant increase in survival with administration of either version of αCD20scFv::SLT-1A. The mean survival age was increased by five days with all treatments compared to the PBS control.

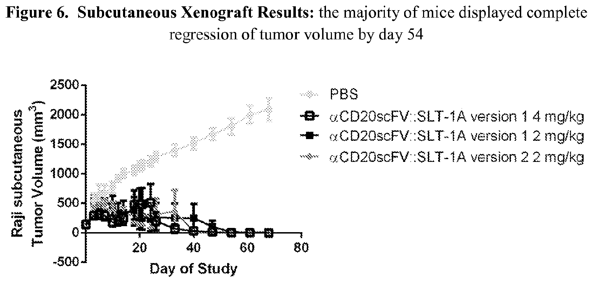

For the second xenograft model, twenty-eight BALBc/nude (in four groups of six or seven animals) were challenged subcutaneously with 2.5 x 10⁶ Raji human lymphoma cells (Washington Biotechnology, Simpsonville, MD, U.S.). Tumor volume was determined using standard methods known in the art utilizing calipers. Day 0 was set at the point that mean tumor volume for each mouse reached approximately 160 mm³ (one mouse from each group had a tumor greater than 260 mm³ so it was excluded). On days 0-4 and 7-11 the groups received intravenous administration of the following by group: Group 1: PBS; Group 2: "αCD20-scFv fused with SLT-1A" version 2 at a dose of 2 mg/kg; Group 3: "αCD20-scFv fused with SLT-1A" version 1 at a dose of 2 mg/kg; Group 4: "αCD20-scFv fused with SLT-1A" version 1 at a dose of 4 mg/kg. Tumor volume was measured and graphed as a function of day of study. Figure 6 demonstrates how treatment with "αCD20-scFv fused with SLT-1A" (at both dosage levels) resulted in significantly reduced tumor volume compared to the PBS control through to Day 24. This is also reflected in the tumor free mouse number through day 54, reported in Table 6.

**Table 6. Elimination of Tumors by Exemplary Cytotoxic Proteins in a Subcutaneous-Tumor Mouse Model**

| **Group** | **Tumor Free Mice / Total Mice** |
|---|---|
| PBS | 0 / 7 |
| αCD20-scFv fused with SLT-1A version 1, 2 mg/kg | 5 / 6 |
| αCD20-scFv fused with SLT-1A version 1, 4 mg/kg | 6 / 7 |

### Determining In Vivo Effects of a Cytotoxic Protein in Non-Human Primates

The exemplary cytotoxic protein "αCD20-scFv fused with SLT-1A" version 1 was administered to non-human primates in order to test for *in vivo* effects. *In vivo* depletion of peripheral blood B lymphocytes in cynomolgus primates was observed after parenteral administration of different doses of "αCD20-scFv fused with SLT-1A" version 1.

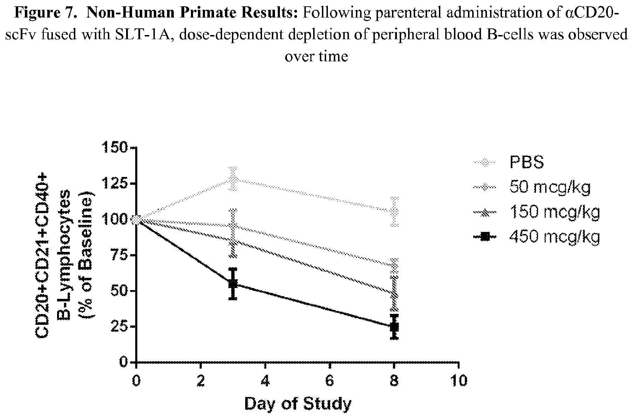

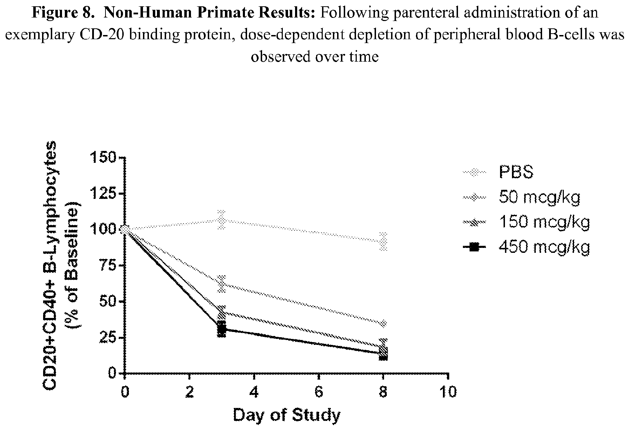

In one experiment, ten cynomolgus primates were intravenous injected with PBS or of "αCD20-scFv fused with SLT-1A" version 1 at different doses (50, 150, and 450 micrograms drug/kilogram body weight (mcg/kg)) on alternative days for 2 weeks. Then, peripheral blood samples collected prior to dosing on days 3 and 8 were analyzed for the percentage of B-lymphocytes which expressed CD20 (Figures 7 and 8). In cynomolgus monkeys, two distinct B-cell subsets have been described; CD21 negative, CD40 positive cells expressing a high level of CD20, and CD21 positive and CD40 positive expressing a lower level of CD20 than by flow-cytometry (Vugmeyster et al.., Cytometry 52: 101-9 (2003)). Dose dependent B-cell depletion as compared to baseline levels from blood samples collected prior to treatment was observed on day 3 (4, 14 and 45% decrease in animals dosed at 50, 150 and 450 mcg/kg) and day 8 (32, 52 and 75% decrease in animals dosed at 50, 150 and 450 mcg/kg) (Table 7). This experiment showed that "αCD20-scFv fused with SLT-1A" version 1 was capable of killing CD20 positive, primate B-cells *in vivo.*

**Table 7. Anti-CD20-scFv fused with SLT-1A Version 1 Protein Dose Dependent B-Cell Depletion in Non-Human Primates**

| | CD40+, CD20+ % Decrease | | | CD21+, CD40+, CD20+ % Decrease | | |
|---|---|---|---|---|---|---|
| Day | 50 mcg/kg | 150 mcg/kg | 450 mcg/kg | 50 mcg/kg | 150 mcg/kg | 450 mcg/kg |
| 3 | 38 | 57 | 69 | 4 | 14 | 45 |
| 8 | 65 | 81 | 86 | 32 | 52 | 75 |

### Example 4. A cytotoxic protein derived from the A Subunit of Shiga-like toxin-1 and the antibody aEpstein-Barr-antigen

In this example, the Shiga toxin effector region is derived from the A subunit of Shiga-like Toxin 1 (SLT-1A). An immunoglobulin-type binding region aEpstein-Barr-antigen is derived from a monoclonal antibody against an Epstein Barr antigen (Fang C et al., J Immunol Methods 287: 21-30 (2004)), which comprises an immunoglobulin-type binding region capable of binding a human cell infected by the Epstein-Barr virus or a transformed cell expressing an Epstein-Barr antigen. The Epstein-Barr antigen is expressed on multiple cell types, such as cells infected by an Epstein-Barr virus and cancer cells (*e.g.* lymphoma and naspharnygeal cancer cells). In addition, Epstein-Barr infection is associated with other diseases, *e.g*., multiple sclerosis.

### Construction, Production, and Purification of the Cytotoxic Protein SLT-1A::αEpsteinBarr

The immunoglobulin-type binding region aEpstein-Barr-antigen and Shiga toxin effector region are linked together to form a protein. For example, a fusion protein is produced by expressing a polynucleotide encoding the αEpstein-Barr-antigen-binding protein SLT-1A::αEpsteinBarr. Expression of the SLT-1A::αEpsteinBarr cytotoxic protein is accomplished using either bacterial and/or cell-free, protein translation systems as described in the previous examples.

### Determining the In Vitro Characteristics of the Cytotoxic Protein SLT-1A::αEpsteinBarr

The binding characteristics of the cytotoxic protein of this example for Epstein-Barr antigen positive cells and Epstein-Barr antigen negative cells is determined by a fluorescence-based, flow-cytometry assay as described above in the previous examples. The Bₘₐₓ for SLT-1A::αEpsteinBarr to Epstein-Barr antigen positive cells is measured to be approximately 50,000-200,000 MFI with a K_{D} of within the range of 0.01-100 nM, whereas there is no significant binding to Epstein-Barr antigen negative cells in this assay.

The ribosome inactivation abilities of the SLT-1A::αEpsteinBarr cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αEpsteinBarr on protein synthesis in this cell-free assay is approximately 0.1-100 pM.

### Determining the Cytotoxicity of the Cytotoxic Protein SLT-1A::αEpsteinBarr Using a Cell-Kill Assay

The cytotoxicity characteristics of SLT-1A::αEpsteinBarr are determined by the general cell-kill assay as described above in the previous examples using Epstein-Barr antigen positive cells. In addition, the selective cytotoxicity characteristics of SLT-1A::αEpsteinBarr are determined by the same general cell-kill assay using Epstein-Barr antigen negative cells as a comparison to the Epstein-Barr antigen positive cells. The CD₅₀ of the cytotoxic protein of this example is approximately 0.01-100 nM for Epstein-Barr antigen positive cells depending on the cell line. The CD₅₀ of the cytotoxic protein is approximately 10-10,000 fold greater (less cytotoxic) for cells not expressing the Epstein-Barr antigen on a cellular surface as compared to cells which do express the Epstein-Barr antigen on a cellular surface.

### Determining the In Vivo Effects of the Cytotoxic Protein SLT-1A::αEpsteinBarr using Animal Models

Animal models are used to determine the *in vivo* effects of the cytotoxic protein SLT-1A::αEpsteinBarr on neoplastic cells. Various mice strains are used to test the effect of the cytotoxic protein after intravenous administration on xenograft tumors in mice resulting from the injection into those mice of human neoplastic cells which express Epstein-Barr antigens on their cell surfaces.

### Example 5. A cytotoxic protein derived from the A Subunit of Shiga-like toxin-1 and the antibody αLeishmania-antigen

In this example, the Shiga toxin effector region is derived from the A subunit of Shiga-like Toxin 1 (SLT-1A). An immunoglobulin-type binding region αLeishmania-antigen is derived from an antibody generated, using techniques known in the art, to a cell-surface Leishmania antigen present on human cells harboring on intracellular trypanosomatid protozoa (*see* Silveira T et al., Int J Parasitol 31: 1451-8 (2001); Kenner J et al., J Cutan Pathol 26: 130-6 (1999); Berman J and Dwyer, Clin Exp Immunol 44: 342-348 (1981)).

### Construction, Production, and Purification of the Cytotoxic Protein SLT-1A::αLeishmania

The immunoglobulin-type binding region α-Leishmania-antigen and Shiga toxin effector region are linked together to form a protein. For example, a fusion protein is produced by expressing a polynucleotide encoding the Leishmania-antigen-binding protein SLT-1A::αLeishmania. Expression of the SLT-1A::αLeishmania cytotoxic protein is accomplished using either bacterial and/or cell-free, protein translation systems as described in the previous examples.

### Determining the In Vitro Characteristics of the Cytotoxic Protein SLT-1A::αLeishmania

The binding characteristics of the cytotoxic protein of this example for Leishmania antigen positive cells and Leishmania antigen negative cells is determined by a fluorescence-based, flow-cytometry assay as described above in the previous examples. The Bₘₐₓ for SLT-1A::αLeishmania to Leishmania antigen positive cells is measured to be approximately 50,000-200,000 MFI with a K_{D} of within the range of 0.01-100 nM, whereas there is no significant binding to Leishmania antigen negative cells in this assay.

The ribosome inactivation abilities of the SLT-1A::αLeishmania cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αLeishmania on protein synthesis in this cell-free assay is approximately 0.1-100 pM.

### Determining the Cytotoxicity of the Cytotoxic Protein SLT-1A::αLeishmania Using a Cell-Kill Assay

The cytotoxicity characteristics of SLT-1A::αLeishmania are determined by the general cell-kill assay as described above in the previous examples using Leishmania antigen positive cells. In addition, the selective cytotoxicity characteristics of SLT-1A::αLeishmania are determined by the same general cell-kill assay using Leishmania antigen negative cells as a comparison to the Leishmania antigen positive cells. The CD₅₀ of the cytotoxic protein of this example is approximately 0.01-100 nM for Leishmania antigen positive cells depending on the cell line. The CD₅₀ of the cytotoxic protein is approximately 10-10,000 fold greater (less cytotoxic) for cells not expressing the Leishmania antigen on a cellular surface as compared to cells which do express the Leishmania antigen on a cellular surface.

### Example 6. A cytotoxic protein derived from the A Subunit of Shiga-like toxin-1 and immunoglobulin-type binding region αNeurotensin-Receptor

In this example, the Shiga toxin effector region is derived from the A subunit of Shiga-like Toxin 1 (SLT-1A). An immunoglobulin-type binding region αNeurotensin-Receptor is derived from the DARPin™ (GenBank Accession: 2P2C_R) or a monoclonal antibody (Ovigne J et al., Neuropeptides 32: 247-56 (1998)) which binds the human neurotensin receptor. The neurotensin receptor is expressed by various cancer cells, such as breast cancer, colon cancer, lung cancer, melanoma, and pancreatic cancer cells.

### Construction, Production, and Purification of the Cytotoxic Protein SLT-1A::αNeurotensinR

The immunoglobulin-type binding region αNeurotensinR and Shiga toxin effector region are linked together to form a protein. For example, a fusion protein is produced by expressing a polynucleotide encoding the neurotensin-receptor-binding protein SLT-1A::αNeurotensinR. Expression of the SLT-1A::αNeurotensinR cytotoxic protein is accomplished using either bacterial and/or cell-free, protein translation systems as described in the previous examples.

### Determining the In Vitro Characteristics of the Cytotoxic Protein SLT-1A::αNeurotensinR

The binding characteristics of the cytotoxic protein of this example for neurotensin receptor positive cells and neurotensin receptor negative cells is determined by a fluorescence-based, flow-cytometry assay as described above in the previous examples. The Bₘₐₓ for SLT-1A::αNeurotensinR to neurotensin receptor positive cells is measured to be approximately 50,000-200,000 MFI with a K_{D} of within the range of 0.01-100 nM, whereas there is no significant binding to neurotensin receptor negative cells in this assay.

The ribosome inactivation abilities of the SLT-1A::αNeurotensinR cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αNeurotensinR on protein synthesis in this cell-free assay is approximately 0.1-100 pM.

### Determining the Cytotoxicity of the Cytotoxic Protein SLT-1A::αNeurotensinR Using a Cell-Kill Assay

The cytotoxicity characteristics of SLT-1A::αNeurotensinR are determined by the general cell-kill assay as described above in the previous examples using neurotensin receptor positive cells. In addition, the selective cytotoxicity characteristics of SLT-1A::αNeurotensinR are determined by the same general cell-kill assay using neurotensin receptor negative cells as a comparison to the neurotensin receptor positive cells. The CD₅₀ of the cytotoxic protein of this example is approximately 0.01-100 nM for neurotensin receptor positive cells depending on the cell line. The CD₅₀ of the cytotoxic protein is approximately 10-10,000 fold greater (less cytotoxic) for cells not expressing neurotensin receptor on a cellular surface as compared to cells which do express neurotensin receptor on a cellular surface.

### Determining the In Vivo Effects of the Cytotoxic Protein SLT-1A::αNeurotensinR using Animal Models

Animal models are used to determine the *in vivo* effects of the cytotoxic protein SLT-1A::αNeurotensinR on neoplastic cells. Various mice strains are used to test the effect of the cytotoxic protein after intravenous administration on xenograft tumors in mice resulting from the injection into those mice of human neoplastic cells which express neurotensin receptors on their cell surfaces.

### Example 7. A cytotoxic protein derived from the A Subunit of Shiga-like toxin-1 and an immunoglobulin-type binding region αEGFR

In this example, the Shiga toxin effector region is derived from the A subunit of Shiga-like Toxin 1 (SLT-1A). An immunoglobulin-type binding region αEGFR is derived from the AdNectin™ (GenBank Accession: 3QWQ_B), the Affibody™ (GenBank Accession: 2KZI_A; U.S. patent 8,598,113), or an antibody, all of which bind one or more human epidermal growth factor receptors. The expression of epidermal growth factor receptors are associated with human cancer cells, such as, *e.g*., lung cancer cells, breast cancer cells, and colon cancer cells.

### Construction, Production, and Purification of the Cytotoxic Protein SLT-1A::αEGFR

The immunoglobulin-type binding region αEGFR and Shiga toxin effector region are linked together to form a protein. For example, a fusion protein is produced by expressing a polynucleotide encoding the EGFR binding protein SLT-1A::αEGFR. Expression of the SLT-1A::αEGFR cytotoxic protein is accomplished using either bacterial and/or cell-free, protein translation systems as described in the previous examples.

### Determining the In Vitro Characteristics of the Cytotoxic Protein SLT-1A::αEGFR

The binding characteristics of the cytotoxic protein of this example for EGFR+ cells and EGFR- cells is determined by a fluorescence-based, flow-cytometry assay as described above in the previous examples. The Bₘₐₓ for SLT-1A::αEGFR to EGFR+ cells is measured to be approximately 50,000-200,000 MFI with a K_{D} of within the range of 0.01-100 nM, whereas there is no significant binding to EGFR- cells in this assay.

The ribosome inactivation abilities of the SLT-1A::αEGFR cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αEGFR on protein synthesis in this cell-free assay is approximately 0.1-100 pM.

### Determining the Cytotoxicity of the Cytotoxic Protein SLT-1A::αEGFR Using a Cell-Kill Assay

The cytotoxicity characteristics of SLT-1A::αEGFR are determined by the general cell-kill assay as described above in the previous examples using EGFR+ cells. In addition, the selective cytotoxicity characteristics of SLT-1A::αEGFR are determined by the same general cell-kill assay using EGFR-cells as a comparison to the Leishmania antigen positive cells. The CD₅₀ of the cytotoxic protein of this example is approximately 0.01-100 nM for EGFR+ cells depending on the cell line. The CD₅₀ of the cytotoxic protein is approximately 10-10,000 fold greater (less cytotoxic) for cells not expressing EGFR on a cellular surface as compared to cells which do express EGFR on a cellular surface.

### Determining the In Vivo Effects of the Cytotoxic Protein SLT-1A::αEGFR using Animal Models

Animal models are used to determine the *in vivo* effects of the cytotoxic protein SLT-1A::αEGFR on neoplastic cells. Various mice strains are used to test the effect of the cytotoxic protein after intravenous administration on xenograft tumors in mice resulting from the injection into those mice of human neoplastic cells which express EGFR(s) on their cell surfaces.

### Example 8. A cytotoxic protein derived from the A Subunit of Shiga-like toxin-1 and the antibody αCCR5

In this example, the Shiga toxin effector region is derived from the A subunit of Shiga-like Toxin 1 (SLT-1A). An immunoglobulin-type binding region αCCR5 is derived from a monoclonal antibody against human CCR5 (CD195) (Bernstone L et al., Hybridoma 31: 7-19 (2012)). CCR5 is predominantly expressed on T-cells, macrophages, dendritic cells, and microglia. In addition, CCR5 plays a role in the pathogenesis and spread of the Human Immunodeficiency Virus (HIV).

### Construction, Production, and Purification of the Cytotoxic Protein SLT-1A::αCCR5

The immunoglobulin-type binding region αCCR5 and Shiga toxin effector region are linked together to form a protein. For example, a fusion protein is produced by expressing a polynucleotide encoding the αCCR5-binding protein SLT-1A::αCCR5. Expression of the SLT-1A::αCCR5 cytotoxic protein is accomplished using either bacterial and/or cell-free, protein translation systems as described in the previous examples.

### Determining the In Vitro Characteristics of the Cytotoxic Protein SLT-1A::αCCR5

The binding characteristics of the cytotoxic protein of this example for CCR5+ cells and CCR5- cells is determined by a fluorescence-based, flow-cytometry assay as described above in the previous examples. The Bₘₐₓ for SLT-1A::αCCR5 to CCR5+ positive cells is measured to be approximately 50,000-200,000 MFI with a K_{D} of within the range of 0.01-100 nM, whereas there is no significant binding to CCR5- cells in this assay.

The ribosome inactivation abilities of the SLT-1A::αCCR5 cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αCCR5 on protein synthesis in this cell-free assay is approximately 0.1-100 pM.

### Determining the Cytotoxicity of the Cytotoxic Protein SLT-1A::αCCR5 Using a Cell-Kill Assay

The cytotoxicity characteristics of SLT-1A::αCCR5 are determined by the general cell-kill assay as described above in the previous examples using CCR5+ cells. In addition, the selective cytotoxicity characteristics of SLT-1A::αCCR5 are determined by the same general cell-kill assay using CCR5-cells as a comparison to the CCR5+ cells. The CD₅₀ of the cytotoxic protein of this example is approximately 0.01-100 nM for CCR5+ cells depending on the cell line. The CD₅₀ of the cytotoxic protein is approximately 10-10,000 fold greater (less cytotoxic) for cells not expressing CCR5 on a cellular surface as compared to cells which do express CCR5 on a cellular surface.

### Determining the In Vivo Effects of the Cytotoxic Protein SLT-1A::αCCR5 using Animal Models

Animal models are used to determine the *in vivo* effects of the cytotoxic protein SLT-1A::αCCR5 on depleting T-cells from donor materials (*see* Tsirigotis P et al., Immunotherapy 4: 407-24 (2012)). Non-human primates are used to determine *in vivo* effects of SLT-1A::αCCR5. Graft versus host disease is analyzed in rhesus macaques after kidney transplantation when the donated organs are pretreated with SLT-1A::αCCR5 (*see* Weaver T et al., Nat Med 15: 746-9 (2009)). *In vivo* depletion of peripheral blood T lymphocytes in cynomolgus primates is observed after parenteral administration of different doses of SLT-1A::αCCR5. The use of SLT-1A::αCCR5 to block HIV infection is tested by giving an acute dose of SLT-1A::αCCR5 to non-human primates in order to severely deplete circulating T-cells upon exposure to a simian immunodeficiency virus (SIV) (*see* Sellier P et al., PLoS One 5: e10570 (2010)).

### Example 9. A cytotoxic protein derived from the A Subunit of Shiga-like toxin-1 and the antibody αUL18

In this example, the Shiga toxin effector region is derived from the A subunit of Shiga-like Toxin 1 (SLT-1A). An immunoglobulin-type binding region αUL18 is derived from an antibody generated, using techniques known in the art, to the cell-surface cytomegalovirus protein UL18, which is present on human cells infected with cytomegalovirus (Yang Z, Bjorkman P, Proc Natl Acad Sci USA 105: 10095-100 (2008)). The human cytomegalovirus infection is associated with various cancers and inflammatory disorders.

### Construction, Production, and Purification of the Cytotoxic Protein SLT-1A::αUL18

The immunoglobulin-type binding region αUL18 and Shiga toxin effector region are linked together to form a protein. For example, a fusion protein is produced by expressing a polynucleotide encoding the αUL18-binding protein SLT-1A::αUL18. Expression of the SLT-1A::αUL18 cytotoxic protein is accomplished using either bacterial and/or cell-free, protein translation systems as described in the previous examples.

### Determining the In Vitro Characteristics of the Cytotoxic Protein SLT-1A::αUL18

The binding characteristics of the cytotoxic protein of this example for cytomegalovirus protein UL18 positive cells and cytomegalovirus protein UL18 negative cells is determined by a fluorescence-based, flow-cytometry assay as described above in the previous examples. The Bₘₐₓ for SLT-1A::αUL18 to cytomegalovirus protein UL18 positive cells is measured to be approximately 50,000-200,000 MFI with a K_{D} of within the range of 0.01-100 nM, whereas there is no significant binding to cytomegalovirus protein UL18 negative cells in this assay.

The ribosome inactivation abilities of the SLT-1A::αUL18 cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αUL18 on protein synthesis in this cell-free assay is approximately 0.1-100 pM.

### Determining the Cytotoxicity of the Cytotoxic Protein SLT-1A::αUL18 Using a Cell-Kill Assay

The cytotoxicity characteristics of SLT-1A::αUL18 are determined by the general cell-kill assay as described above in the previous examples using cytomegalovirus protein UL18 positive cells. In addition, the selective cytotoxicity characteristics of SLT-1A::αUL18 are determined by the same general cell-kill assay using cytomegalovirus protein UL18 negative cells as a comparison to the cytomegalovirus protein UL18 positive cells. The CD₅₀ of the cytotoxic protein of this example is approximately 0.01-100 nM for cytomegalovirus protein UL18 positive cells depending on the cell line. The CD₅₀ of the cytotoxic protein is approximately 10-10,000 fold greater (less cytotoxic) for cells not expressing the cytomegalovirus protein UL18 on a cellular surface as compared to cells which do express the cytomegalovirus protein UL18 on a cellular surface.

### Example 10. Various cytotoxic proteins targeting various cell types

In this example, the Shiga toxin effector region is derived from the A subunit of Shiga-like Toxin 1 (SLT-1A), Shiga toxin (StxA), and/or Shiga-like Toxin 2 (SLT-2A). An immunoglobulin-type binding region is derived from the immunoglobulin domain from the molecule chosen from column 1 of Table 8 and which binds the extracellular target biomolecule indicated in column 2 of Table 8. The exemplary cytotoxic proteins of this example are created and tested as described in the previous examples using cells expressing the appropriate extracellular target biomolecules. The exemplary proteins of this example may be used to diagnose and treat the diseases, conditions, or disorders indicated in column 3 of Table 8.

**Table 8. Various Immunoglobulin-Type Binding Regions for Cell Targeting of Cytotoxic Proteins**

| Source of binding region | Extracellular target | Application(s) |
|---|---|---|
| alemtuzumab | CD52 | B-cell cancers, such as lymphoma and leukemia, and B-cell related immune disorders, such as autoimmune disorders |
| basiliximab | CD25 | T-cell disorders, such as prevention of organ transplant rejections, and some B-cell lineage cancers |
| brentuximab vedotin | CD30 | hematological cancers, B-cell related immune disorders, and T-cell related immune disorders |
| catumaxomab | EpCAM | various cancers, such as ovarian cancer, malignant ascites, gastric cancer |
| cetuximab | EGFR | various cancers, such as colorectal cancer and head and neck cancer |
| daclizumab | CD25 | B-cell lineage cancers and T-cell disorders, such as rejection of organ transplants |
| dinutuximab | ganglioside GD2 | Various cancers, such as breast cancer, myeloid cancers, and neuroblastoma |
| efalizumab | LFA-1 (CD11a) | autoimmune disorders, such as psoriasis |
| gemtuzumab | CD33 | myeloid cancer or immune disorder |
| ibritumomab | CD20 | B-cell cancers, such as lymphoma and leukemia, and B-cell related immune disorders, such as autoimmune disorders |
| ipilimumab | CD152 | T-cell related disorders and various cancers, such as leukemia, melanoma |
| muromonab | CD3 | prevention of organ transplant rejections |
| natalizumab | integrin α4 | autoimmune disorders, such as multiple sclerosis and Crohn's disease |
| obinutuzumab | CD20 | B-cell cancers, such as lymphoma and leukemia, and B-cell related immune disorders, such as autoimmune disorders |
| ofatumumab | CD20 | B-cell cancers, such as lymphoma and leukemia, and B-cell related immune disorders, such as autoimmune disorders |
| palivizumab | F protein of respiratory syncytial virus | treat respiratory syncytial virus |
| panitumumab | EGFR | various cancers, such as colorectal cancer and head and neck cancer |
| pertuzumab | HER2/neu | various cancers and tumors, such as breast cancer and colorectal cancer |
| ramucirumab | VEGFR2 | various cancers and cancer related disorders, such as solid tumors |
| rituximab | CD20 | B-cell cancers, such as lymphoma and leukemia, and B-cell related immune disorders, such as autoimmune disorders |
| tocilizumab or atlizumab | IL-6 receptor | autoimmune disorders, such as rheumatoid arthritis |
| tositumomab | CD20 | B-cell cancers, such as lymphoma and leukemia, and B-cell related immune disorders, such as autoimmune disorders |
| vedolizumab | integrin α4β7 | autoimmune disorders, such as Crohn's disease and ulcerative colitis |
| CD20 binding scFv(s) Geng S et al., Cell Mol Immunol 3: 439-43 (2006); Olafesn T et al., Protein Eng Des Sel 23: 243-9 (2010) | CD20 | B-cell cancers, such as lymphoma and leukemia, and B-cell related immune disorders, such as autoimmune disorders |
| CD22 binding scFv(s) Kawas S et al., MAbs 3: 479-86 (2011) | CD22 | B-cell cancers or B-cell related immune disorders |
| CD25 binding scFv(s) Muramatsu H et al., Cancer Lett 225: 225-36 (2005) | CD25 | various cancers of the B-cell lineage and immune disorders related to T-cells |
| CD30 binding monoclonal antibody(s) Klimka A et al., Br J Cancer 83: 252-60 (2000) | CD30 | B-cell cancers or B-cell/T-cell related immune disorders |
| CD33 binding monoclonal antibody(s) Benedict C et al., J Immunol Methods 201: 223-31 (1997) | CD33 | myeloid cancer or immune disorder |
| CD40 binding scFv(s) Ellmark P et al., Immunology 106: 456-63 (2002) | CD40 | various cancers and immune disorders |
| CD52 binding monoclonal antibody(s) U.S. Patent 7,910,104 B2 | CD52 | B-cell cancers, such as lymphoma and leukemia, and B-cell related immune disorders, such as autoimmune disorders |
| CD56 binding monoclonal antibody(s) Shin J et al., Hybridoma 18: 521-7 (1999) | CD56 | immune disorders and various cancers, such as lung cancer, Merkel cell carcinoma, myeloma |
| CD79 binding monoclonal antibody(s) Zhang L et al., Ther Immunol 2: 191-202 (1995) | CD79 | B-cell cancers or B-cell related immune disorders |
| CD248 binding scFv(s) Zhao A et al., J Immunol Methods 363: 221-32 (2011) | CD248 | various cancers, such as inhibiting angiogenesis |
| EpCAM binding monoclonal antibody(s) Schanzer J et al., J Immunother 29: 477-88 (2006) | EpCAM | various cancers, such as ovarian cancer, malignant ascites, gastric cancer |
| PSMA binding monoclonal antibody(s) Frigerio B et al., Eur J Cancer 49: 2223-32 (2013) | PSMA | prostate cancer |
| Eph-B2 binding monoclonal antibody(s) Abéngozar M et al., Blood 119: 4565-76 (2012) | Eph-B2 | for various cancers such as colorectal cancer and prostate cancer |
| Endoglin binding monoclonal antibody(s) Völker T et al., Biocehm Biophys Res Commun 317: 512-21 (2004) | Endoglin | various cancers, such as breast cancer and colorectal cancers |
| FAP binding monoclonal antibody(s) Zhang J et al., FASEB J 27: 581-9 (2013) | FAP | various cancers, such as sarcomas and bone cancers |
| CEA binding monoclonal antibody(s) Zhu H et al., Cancer Biol Ther 8: 1034-44 (2009) | CEA | various cancers, such as gastrointestinal cancer, pancreatic cancer, lung cancer, and breast cancer |
| CD24 binding monoclonal antibody(s) Kristiansen G et al., Lab Invest 90: 1102-16 (2010) | CD24 | various cancers, such as bladder cancer |
| LewisY antigen binding scFv(s) Power B et al., Protein Sci 12: 734-47 (2003); monoclonal antibody BR96 Feridani A et al., Cytometry 71: 361-70 (2007) | Lewis Y antigens | various cancers, such as cervical cancer and uterine cancer |

| Sequence Listings | | |
|---|---|---|
| ID Number | Text Description | Biological Sequence |
| SEQ ID NO:1 | Shiga-like toxin 1 Subunit A (SLT-1A) | KEFTLDFSTAKTYVDSLNVIRSA IGTPLQTISSGGTSLLMIDSGSGD NLFAVDVRGIDPEEGRFNNLRLI VERNNLYVTGFVNRTNNVFYRF ADFSHVTFPGTTAVTLSGDSSYT TLQRVAGISRTGMQINRHSLTTS YLDLMSHSGTSLTQSVARAMLR FVTVTAEALRFRQIQRGFRTTLD DLSGRSYVMTAEDVDLTLNWG RLSSVLPDYHGQDSVRVGRISFG SINAILGSVALILNCHHHASRVA RMASDEFPSMCPADGRVRGITH NKILWDSSTLGAILMRRTISS |
| SEQ ID NO:2 | Shiga toxin Subunit A | KEFTLDFSTAKTYVDSLNVIRSA IGTPLQTISSGGTSLLMIDSGTGD NLFAVDVRGIDPEEGRFNNLRLI VERNNLYVTGFVNRTNNVFYRF ADFSHVTFPGTTAVTLSGDSSYT TLQRVAGISRTGMQINRHSLTTS YLDLMSHSGTSLTQSVARAMLR FVTVTAEALRFRQIQRGFRTTLD DLSGRSYVMTAEDVDLTLNWG RLSSVLPDYHGQDSVRVGRISFG SINAILGSVALILNCHHHASRVA RMASDEFPSMCPADGRVRGITH NKILWDSSTLGAILMRRTISS |
| SEQ ID NO:3 | Shiga-like toxin 2 Subunit A (SLT-2A) | DEFTVDFSSQKSYVDSLNSIRSAI STPLGNISQGGVSVSVINHVLGG NYISLNVRGLDPYSERFNHLRLI MERNNLYVAGFINTETNIFYRFS DFSHISVPDVITVSMTTDSSYSSL QRIADLERTGMQIGRHSLVGSY LDLMEFRGRSMTRASSRAMLRF VTVIAEALRFRQIQRGFRPALSE ASPLYTMTAQDVDLTLNWGRIS NVLPEYRGEEGVRIGRISFNSLS AILGSVAVILNCHSTGSYSVRSV SQKQKTECQIVGDRAAIKVNNV LWEANTIAALLNRKPQDLTEPN Q |
| SEQ ID NO:4 | anti-CD38-scFv fused with SLT-1A | MKEFTLDFSTAKTYVDSLNVIRS AIGTPLQTISSGGTSLLMIDSGSG DNLFAVDVRGIDPEEGRFNNLR LIVERNNLYVTGFVNRTNNVFY RFADFSHVTFPGTTAVTLSGDSS YTTLQRVAGISRTGMQINRHSLT TSYLDLMSHSGTSLTQSVARAM LRFVTVTAEALRFRQIQRGFRTT LDDLSGRSYVMTAEDVDLTLN |
| | | WGRLSSVLPDYHGQDSVRVGRI SFGSINAILGSVALILNCHHHASR VAREFPKPSTPPGSSGGAPDIELT QSPSSFSVSLGDRVTITCKASEDI YNRLAWYQQKPGNAPRLLISGA TSLETGVPSRFSGSGSGKDYTLSI TSLQTEDVATYYCQQYWSTPTF GGGTKLEIKGSTSGSGKPGSGEG SKVQLQESGPSLVQPSQRLSITC TVSGFSLISYGVHWVRQSPGKG LEWLGVIWRGGSTDYNAAFMS RLSITKDNSKSQVFFKMNSLQA DDTAIYFCAKTLITTGYAMDYW GQGTTVTVSS |
| SEQ ID NO 5: | anti-HER2-V_{HH} fused with SLT-1A | MEVQLVESGGGLVQAGGSLRLS CAASGITFSINTMGWYRQAPGK QRELVALISSIGDTYYADSVKGR FTISRDNAKNTVYLQMNSLKPE DTAVYYCKRFRTAAQGTDYWG QGTQVTVSSAHHSEDPSSKAPK APKEFTLDFSTAKTYVDSLNVIR SAIGTPLQTISSGGTSLLMIDSGS GDNLFAVDVRGIDPEEGRFNNL RLIVERNNLYVTGFVNRTNNVF YRFADFSHVTFPGTTAVTLSGDS SYTTLQRVAGISRTGMQINRHSL TTSYLDLMSHSGTSLTQSVARA MLRFVTVTAEALRFRQIQRGFR TTLDDLSGRSYVMTAEDVDLTL NWGRLSSVLPDYHGQDSVRVG RISFGSINAILGSVALILNCHHHA SRVAR |

## Claims

1. A cytotoxic protein comprising:
a) an immunoglobulin-type binding region:
(i) comprising one or more of: single-domain antibody fragment, single-chain variable fragment, antibody variable fragment, antigen-binding fragment, and Fd fragment; and
(ii) capable of specifically binding at least one extracellular target biomolecule;
and
b) a cytotoxic Shiga toxin A Subunit effector polypeptide comprising:
an amino acid sequence that is at least 85% identical to the amino acid sequence shown in SEQ ID NO: 1, SEQ ID NO: 2, or SEQ ID NO: 3;
or an amino acid sequence selected from:
(i) amino acids 75 to 251 of SEQ ID NO: 1, SEQ ID NO: 2, or SEQ ID NO: 3;
(ii) amino acids 1 to 241 of SEQ ID NO: 1, SEQ ID NO: 2, or SEQ ID NO: 3;
(iii) amino acids 1 to 251 of SEQ ID NO: 1, SEQ ID NO: 2, or SEQ ID NO: 3; and
(iv) amino acids 1 to 261 of SEQ ID NO: 1, SEQ ID NO: 2, or SEQ ID NO: 3;
whereby upon administration of the cytotoxic protein to a cell physically coupled with an extracellular target biomolecule of the immunoglobulin-type binding region, the cytotoxic protein is capable of causing death of the cell.

2. A cytotoxic protein according to claim 1, whereby upon administration of the cytotoxic protein to a first population of cells whose members are physically coupled to extracellular target biomolecules of the immunoglobulin-type binding region, and a second population of cells whose members are not physically coupled to any extracellular target biomolecule of the immunoglobulin-type binding region, a cytotoxic effect of the cytotoxic protein to members of said first population of cells relative to members of said second population of cells is at least 3-fold greater.

3. A cytotoxic protein according to claim 2, wherein said first population of cells is physically coupled with said extracellular target biomolecule by means of covalent and/or non-covalent intermolecular interactions that couple the extracellular target biomolecule, or a portion thereof, to the outside of a cell; or wherein said extracellular target biomolecule is an integral membrane protein or peripheral membrane protein expressed by the cell or first population of cells.

4. A cytotoxic protein according to any one of claims 1-3, wherein the immunoglobulin-type binding region is capable of binding to an extracellular target biomolecule selected from the group consisting of: CD20, CD22, CD40, CD79, CD25, CD30, HER2/neu/ErbB2, EGFR, EphB2, prostate-specific membrane antigen, Cripto, endoglin, fibroblast activation protein, Lewis-Y, CD19, CD21, CS1/ SLAMF7, CD33, CD52, EpCAM, gpA33, mucin, TAG-72, carbonic anhydrase IX, folate binding protein, ganglioside GD2, ganglioside GD3, ganglioside GM2, ganglioside Lewis-Y2, VEGFR, Alpha V beta3, Alpha5beta1, ErbB1/EGFR, Erb3, c-MET, IGF1R, EphA3, TRAIL-R1, TRAIL-R2, RANKL, tenascin, CD64, mesothelin, BRCA1, MART-1/MelanA, gp100, tyrosinase, TRP-1, TRP-2, MAGE-1, MAGE-3, GAGE-1/2, BAGE, RAGE, NY-ESO-1, CDK-4, beta-catenin, MUM-1, caspase-8, KIAA0205, HPVE6, SART-1, PRAME, carcinoembryonic antigen, prostate specific antigen, prostate stem cell antigen, human aspartyl (asparaginyl) beta-hydroxylase, EphA2, HER3/ErbB-3, MUC1, tyrosinase-associated antigen, HPV-E7, Epstein-Barr Virus antigen, Bcr-Abl, alpha-fetoprotein antigen, 17-A1, bladder tumor antigen, CD38, CD15, CD23, CD53, CD88, CD129, CD183, CD191, CD193, CD244, CD294, CD305, C3AR, FceRla, galectin-9, mrp-14, Siglec-8, Siglec-10, CD49d, CD13, CD44, CD54, CD63, CD69, CD123, TLR4, IgE, CD107a, CD203c, CD14, CD68, CD80, CD86, CD105, CD115, F4/80, ILT-3, galectin-3, CD11a-c, GITRL, MHC Class II, CD284-TLR4, CD107-Mac3, CD195-CCR5, HLA-DR, CD16/32, CD282-TLR2, and any immunogenic fragment of any of the foregoing.

5. A cytotoxic protein according to any one of claims 1-4, wherein the cytotoxic Shiga toxin effector polypeptide comprises an amino acid sequence selected from:
(i) amino acids 75 to 251 of SEQ ID NO:1, SEQ ID NO:2, or SEQ ID NO:3;
(ii) amino acids 1 to 241 of SEQ ID NO:1, SEQ ID NO:2, or SEQ ID NO:3;
(iii) amino acids 1 to 251 of SEQ ID NO:1, SEQ ID NO:2, or SEQ ID NO:3; and
(iv) amino acids 1 to 261 of SEQ ID NO:1, SEQ ID NO:2, or SEQ ID NO:3.

6. A cytotoxic protein according to claim 4, wherein the immunoglobulin-type binding region comprises the sequence of amino acids 269-508 of SEQ ID NO: 4;
or wherein the immunoglobulin-type binding region comprises the sequence of amino acids 1-119 of SEQ ID NO: 5, such as wherein the cytotoxic protein comprises the polypeptide sequence of SEQ ID NO: 5.

7. A cytotoxic protein according to any one of claims 1-6, wherein the cytotoxic Shiga toxin effector polypeptide comprises a mutation relative to a naturally occurring A Subunit of a member of the Shiga toxin family which changes the enzymatic activity of the Shiga toxin effector polypeptide while retaining a cytotoxic activity; wherein the mutation is selected from at least one amino acid residue deletion or substitution.

8. The cytotoxic protein of any one of claims 1-7, in the form of a homo-multimer or hetero-multimer.

9. The cytotoxic protein of any one of claims 1-8, in the form of a pharmaceutically acceptable salt or solvate.

10. A cytotoxic protein according to any one of claims 1-9, conjugated to a diagnostic agent.

11. A pharmaceutical composition comprising a cytotoxic protein according to any one of claims 1-10 and at least one pharmaceutically acceptable excipient or carrier.

12. A polynucleotide capable of encoding a cytotoxic protein according to any one of claims 1-7, or a complement thereof.

13. An expression vector comprising a polynucleotide according to claim 12.

14. A transformed host cell comprising a polynucleotide according to claim 12 or an expression vector according to claim 13.

15. An *in vitro* method of killing a cell, the method comprising the step of contacting the cell with a cytotoxic protein according to any one of claims 1-10.

16. An *in vitro* method of labelling the interior of a cancer cell or immune cell, the method comprising the step of contacting the cell with a cytotoxic protein according to claim 10.

17. A cytotoxic protein according to any one of claims 1-10 or a pharmaceutical composition according to claim 11 for use in the treatment of a disease, disorder, or condition in a patient.

18. The cytotoxic protein or pharmaceutical composition for use according to claim 17, wherein the treatment is of a disease, disorder, or condition selected from the group consisting of: cancer, tumor, immune disorder, and microbial infection;
such as wherein the cancer is selected from the group consisting of: bone cancer, breast cancer, central/peripheral nervous system cancer, gastrointestinal cancer, germ cell cancer, glandular cancer, head-neck cancer, hematological cancer, kidney-urinary tract cancer, liver cancer, lung/pleura cancer, prostate cancer, sarcoma, skin cancer, and uterine cancer;
or wherein the immune disorder is associated with a disease selected from the group consisting of: amyloidosis, ankylosing spondylitis, asthma, Crohn's disease, diabetes, graft rejection, graft versus host disease, Hashimoto's thyroiditis, hemolytic uremic syndrome, HIV-related disease, lupus erythmatosus, multiple sclerosis, polyarteritis, psoriasis, psoriatic arthritis, rheumatoid arthritis, scleroderma, septic shock, Sjögren's syndrome, ulcerative colitis, and vasculitis.

## Patentansprüche

1. Ein zytotoxisches Protein, das Folgendes beinhaltet:
a) eine immunglobulinartige Bindungsregion:
i) die eines oder mehrere der Folgenden beinhaltet: Einzeldomänen-Antikörperfragment, variables Einzelkettenfragment, variables Antikörperfragment, Antigenbindungsfragment und Fd-Fragment; und
ii) die in der Lage ist, mindestens ein extrazelluläres Zielbiomolekül spezifisch zu binden;
und
b) ein zytotoxisches Shigatoxin-A-Untereinheit-Effektorpolypeptid, das Folgendes beinhaltet:
eine Aminosäuresequenz, die zu mindestens 85% mit der in SEQ ID NO: 1, SEQ ID NO: 2 oder SEQ ID NO: 3 gezeigten Aminosäuresequenz identisch ist;
oder eine Aminosäuresequenz, ausgewählt aus:
i) Aminosäuren 75 bis 251 von SEQ ID NO: 1, SEQ ID NO: 2 oder SEQ ID NO: 3;
ii) Aminosäuren 1 bis 241 von SEQ ID NO: 1, SEQ ID NO: 2 oder SEQ ID NO: 3;
iii) Aminosäuren 1 bis 251 von SEQ ID NO: 1, SEQ ID NO: 2 oder SEQ ID NO: 3; und
iv) Aminosäuren 1 bis 261 von SEQ ID NO: 1, SEQ ID NO: 2 oder SEQ ID NO: 3;
wobei nach Verabreichung des zytotoxischen Proteins an eine Zelle, die physikalisch mit einem extrazellulären Zielbiomolekül der immunglobulinartigen Bindungsregion gekoppelt ist, das zytotoxische Protein in der Lage ist, den Tod der Zelle zu verursachen.

2. Zytotoxisches Protein nach Anspruch 1, wobei nach Verabreichung des zytotoxischen Proteins an eine erste Population von Zellen, deren Mitglieder physikalisch mit den extrazellulären Zielbiomolekülen der immunglobulinartigen Bindungsregion gekoppelt sind, und eine zweite Population von Zellen, deren Mitglieder nicht physikalisch mit einem extrazellulären Zielbiomolekül der immunglobulinartigen Bindungsregion gekoppelt sind, ein zytotoxischer Effekt des zytotoxischen Proteins auf Mitglieder der genannten ersten Population von Zellen im Verhältnis zu Mitgliedern der genannten zweiten Population von Zellen mindestens um das 3-Fache größer ist.

3. Zytotoxisches Protein nach Anspruch 2, wobei die genannte erste Population von Zellen mittels kovalenter und/oder nicht kovalenter intermolekularer Interaktionen physikalisch mit dem genannten extrazellulären Zielbiomolekül gekoppelt ist, welche das genannte extrazelluläre Zielbiomolekül oder einen Teil davon mit der Außenseite einer Zelle koppeln; oder wobei das genannte extrazelluläre Zielbiomolekül ein integriertes Membranprotein oder peripheres Membranprotein ist, das von der Zelle oder ersten Population von Zellen exprimiert wird.

4. Zytotoxisches Protein nach einem der Ansprüche 1-3, wobei die immunglobulinartige Bindungsregion in der Lage ist, an ein extrazelluläres Zielbiomolekül zu binden, ausgewählt aus der Gruppe, bestehend aus: CD20, CD22, CD40, CD79, CD25, CD30, HER2/neu/ErbB2, EGFR, EphB2, prostataspezifischem Membranantigen, Cripto, Endoglin, fibroblastenaktiviertem Protein, Lewis-Y, CD19, CD21, CS1/ SLAMF7, CD33, CD52, EpCAM, gpA33, Mucin, TAG-72, Carboanhydrase IX, Folatbindungsprotein, Gangliosid GD2, Gangliosid GD3, Gangliosid GM2, Gangliosid Lewis-Y2, VEGFR, Alpha V beta3, Alpha5beta1, ErbB1/EGFR, Erb3, c-MET, IGF1R, EphA3, TRAIL-R1, TRAIL-R2, RANKL, Tenascin, CD64, Mesothelin, BRCA1, MART-1/MelanA, gp100, Tyrosinase, TRP-1, TRP-2, MAGE-1, MAGE-3, GAGE-1/2, BAGE, RAGE, NY-ESO-1, CDK-4, Beta-Catenin, MUM-1, Caspase-8, KIAA0205, HPVE6, SART-1, PRAME, Carcinoembryo-Antigen, prostataspezifischem Antigen, Prostatastammzellenantigen, humaner Aspartyl-(Asparaginyl)-beta-hydroxylase, EphA2, HER3/ErbB-3, MUC1, tyrosinaseassoziiertem Antigen, HPV-E7, Epstein-Barr-Virus-Antigen, Bcr-Abl, Alpha-Fetoprotein-Antigen, 17-A1, Blasentumorantigen, CD38, CD15, CD23, CD53, CD88, CD129, CD183, CD191, CD193, CD244, CD294, CD305, C3AR, FceRla, Galectin-9, mrp-14, Siglec-8, Siglec-10, CD49d, CD13, CD44, CD54, CD63, CD69, CD123, TLR4, IgE, CD107a, CD203c, CD14, CD68, CD80, CD86, CD105, CD115, F4/80, ILT-3, Galectin-3, CD11a-c, GITRL, MHC Class II, CD284-TLR4, CD107-Mac3, CD195-CCR5, HLA-DR, CD16/32, CD282-TLR2 und einem beliebigen immunogenen Fragment eines der zuvor genannten.

5. Zytotoxisches Protein nach einem der Ansprüche 1-4, wobei das zytotoxische Shigatoxin-Effektorpolypeptid eine Aminosäuresequenz beinhaltet, ausgewählt aus:
i) Aminosäuren 75 bis 251 von SEQ ID NO: 1, SEQ ID NO: 2 oder SEQ ID NO: 3;
ii) Aminosäuren 1 bis 241 von SEQ ID NO: 1, SEQ ID NO: 2 oder SEQ ID NO: 3;
iii) Aminosäuren 1 bis 251 von SEQ ID NO: 1, SEQ ID NO: 2 oder SEQ ID NO: 3; und
iv) Aminosäuren 1 bis 261 von SEQ ID NO: 1, SEQ ID NO: 2 oder SEQ ID NO: 3.

6. Zytotoxisches Protein nach Anspruch 4, wobei die immunglobulinartige Bindungsregion die Sequenz von Aminosäuren 269-508 von SEQ ID NO: 4 beinhaltet;
oder wobei die immunglobulinartige Bindungsregion die Sequenz von Aminosäuren 1-119 von SEQ ID NO: 5 beinhaltet, wobei beispielsweise das zytotoxische Protein die Polypeptidsequenz von SEQ ID NO: 5 beinhaltet.

7. Zytotoxisches Protein nach einem der Ansprüche 1-6, wobei das zytotoxische Shigatoxin-Effektorpolypeptid eine Mutation im Verhältnis zu einer natürlich vorkommenden A-Untereinheit eines Mitglieds der Shigatoxin-Familie beinhaltet, welche die enzymatische Aktivität des Shigatoxin-Effektorpolypeptids verändert, während eine zytotoxische Aktivität beibehalten wird; wobei die Mutation aus mindestens einer Aminosäurerestdeletion oder -substitution ausgewählt ist.

8. Zytotoxisches Protein nach einem der Ansprüche 1-7 in der Form eines Homomultimers oder Heteromultimers.

9. Zytotoxisches Protein nach einem der Ansprüche 1-8 in der Form eines pharmazeutisch akzeptablen Salzes oder Solvats.

10. Zytotoxisches Protein nach einem der Ansprüche 1-9, das mit einem Diagnostikum konjugiert ist.

11. Eine pharmazeutische Zusammensetzung, die das zytotoxische Protein nach einem der Ansprüche 1-10 und mindestens einen pharmazeutisch akzeptablen Hilfsstoff oder Träger beinhaltet.

12. Ein Polynukleotid, das in der Lage ist, für das zytotoxische Protein nach einem der Ansprüche 1-7 oder ein Komplement davon zu kodieren.

13. Ein Expressionsvektor, der ein Polynukleotid nach Anspruch 12 beinhaltet.

14. Eine transformierte Wirtszelle, die ein Polynukleotid nach Anspruch 12 oder einen Expressionsvektor nach Anspruch 13 beinhaltet.

15. Ein In-vitro-Verfahren zum Abtöten einer Zelle, wobei das Verfahren den Schritt des In-Kontakt-Bringens der Zelle mit dem zytotoxischen Protein nach einem der Ansprüche 1-10 beinhaltet.

16. Ein In-vitro-Verfahren zum Markieren des Inneren einer Krebszelle oder Immunzelle, wobei das Verfahren den Schritt des In-Kontakt-Bringens der Zelle mit dem zytotoxischen Protein nach Anspruch 10 beinhaltet.

17. Zytotoxisches Protein nach einem der Ansprüche 1-10 oder eine pharmazeutische Zusammensetzung nach Anspruch 11 zur Verwendung bei der Behandlung einer Erkrankung, einer Störung oder eines Krankheitszustands bei einem Patienten.

18. Zytotoxisches Protein oder pharmazeutische Zusammensetzung zur Verwendung nach Anspruch 17, wobei die Behandlung für eine Erkrankung, eine Störung oder einen Krankheitszustand ist, ausgewählt aus der Gruppe, bestehend aus: Krebs, Tumor, Immunstörung oder mikrobieller Infektion;
wobei der Krebs beispielsweise ausgewählt ist aus der Gruppe, bestehend aus: Knochenkrebs, Brustkrebs, Krebs des Zentralnervensystems/peripheren Nervensystems, Krebs des Magen-Darm-Trakts, Keimzellenkrebs, Drüsenkrebs, Kopf-Hals-Krebs, Blutkrebs, Krebs der Nieren/Harnwege, Leberkrebs, Lungenkrebs/Pleurakrebs, Prostatakrebs, Sarkom, Hautkrebs und Gebärmutterkrebs;
oder wobei die Immunstörung mit einer Erkrankung assoziiert ist, ausgewählt aus der Gruppe, bestehend aus: Amyloidose, Spondylitis ankylosans, Asthma, Morbus Crohn, Diabetes, Transplantatabstoßung, Graft-versus-Host-Krankheit, Hashimoto-Thyreoiditis, hämolytischem urämischem Syndrom, mit HIV verbundener Erkrankung, Lupus erythematodes, multipler Sklerose, Polyarthritis, Psoriasis, Psoriasis-Arthritis, rheumatoider Arthritis, Sklerodermie, septischem Schock, Sjögren-Syndrom, Colitis ulcerosa und Vaskulitis.

## Revendications

1. Protéine cytotoxique comprenant :
a) une région de liaison de type immunoglobuline :
i) comprenant un ou plusieurs d'un fragment d'anticorps à un seul domaine, d'un fragment variable monocaténaire, d'un fragment variable d'anticorps, d'un fragment se liant à un antigène, et d'un fragment Fd ; et
ii) capable de se lier spécifiquement à au moins une biomolécule cible extracellulaire,
et
b) un polypeptide effecteur de sous-unité A de shiga-toxine cytotoxique comprenant :
une séquence d'acides aminés qui est au moins 85 % identique à la séquence d'acides aminés énoncée dans la SÉQ. ID n° 1, la SÉQ. ID n° 2, ou la SÉQ. ID n° 3 ;
ou une séquence d'acides aminés sélectionnée parmi :
i) les acides aminés 75 à 251 de la SÉQ. ID n° 1, de la SÉQ. ID n° 2, ou de la SÉQ. ID n° 3 ;
ii) les acides aminés 1 à 241 de la SÉQ. ID n° 1, de la SÉQ. ID n° 2, ou de la SÉQ. ID n° 3 ;
iii) les acides aminés 1 à 251 de la SÉQ. ID n° 1, de la SÉQ. ID n° 2, ou de la SÉQ. ID n° 3 ; et
iv) les acides aminés 1 à 261 de la SÉQ. ID n° 1, de la SÉQ. ID n° 2, ou de la SÉQ. ID n° 3 ;
**caractérisée en ce que**, lorsque la protéine cytotoxique est administrée à une cellule physiquement couplée à une biomolécule cible extracellulaire de la région de liaison de type immunoglobuline, la protéine cytotoxique est capable de causer la mort de la cellule.

2. Protéine cytotoxique selon la revendication 1, **caractérisée en ce que**, lorsque la protéine cytotoxique est administrée à une première population de cellules dont les membres sont physiquement couplés à des biomolécules cibles extracellulaires de la région de liaison de type immunoglobuline, et à une deuxième population de cellules dont les membres ne sont physiquement couplés à aucune biomolécule cible extracellulaire de la région de liaison de type immunoglobuline, un effet cytotoxique de la protéine cytotoxique sur les membres de ladite première population de cellules relativement aux membres de ladite deuxième population de cellules est au moins 3 fois supérieur.

3. Protéine cytotoxique selon la revendication 2, ladite première population de cellules étant couplée physiquement à ladite biomolécule cible extracellulaire au moyen d'interactions intermoléculaires covalentes et/ou non covalentes qui couplent la biomolécule cible extracellulaire, ou une partie de celle-ci, à l'extérieur d'une cellule ; ou ladite biomolécule cible extracellulaire étant une protéine membranaire intégrale ou une protéine membranaire périphérique exprimée par la cellule ou la première population de cellules.

4. Protéine cytotoxique selon l'une quelconque des revendications 1 à 3, dans laquelle la région de liaison de type immunoglobuline est capable de se lier à une biomolécule cible extracellulaire sélectionnée dans le groupe constitué de : CD20, CD22, CD40, CD79, CD25, CD30, HER2/neu/ERbB2, EGFR, EphB2, antigène membranaire spécifique de la prostate, Cripto, endogline, protéine d'activation des fibroblastes, Lewis-Y, CD19, CD21, CS1/SLAMF7, CD33, CD52, EpCAM, gpA33, mucine, tag-72, anhydrase carbonique IX, protéine liant les folates, ganglioside GD2, ganglioside GD3, ganglioside GM2, ganglioside Lewis-Y2, VEGFR, alphaV bêta3, AlphaV bêta1, ErbB1/EGFR, Erb3, c-MET, IGF1R, EphA3, TRAIL-R1, TRAIL-R2, RANKL, ténascine, CD64, mésothéline, BRCA1, MART-1/MelanA, gp100, tyrosinase, TRP-1, TRP-2, MAGE-1, MAGE-3, GAGE-1/2, BAGE, RAGE, NY-ESO-1, CDK-4, bêta-caténine, MUM-1, caspase-8, KIAA0205, HPVE6, SART-1, PRAME, antigène carcinoembryonnaire, antigène spécifique de la prostate, antigène des cellules souches prostatiques, aspartyl(asparaginyl)bêta-hydroxylase humaine, EphA2, HER3/ErbB-3, MUC1, antigène associé à la tyrosinase, HPV-E7, antigène du virus d'Epstein-Barr, Bcr-Abl, antigène de l'alpha-fœtoprotéine, 17-A1, antigène tumoral de la vessie, CD38, CD15, CD23, CD53, CD88, CD129, CD183, CD191, CD193, CD244, CD294, CD305, C3AR, FceRla, galectine-9, mrp-14, Siglec-8, Siglec-10, CD49d, CD13, CD44, CD54, CD63, CD69, CD123, TLR4, IgE, CD107a, CD203c, CD14, CD68, CD80, CD86, CD105, CD115, F4/80, ILT-3, galectine-3, CD11a-c, GITRL, MHC Classe II, CD284-TLR4, CD107-Mac3, CD195-CCR5, HLA-DR, CD16/32, CD282-TLR2, et tout fragment immunogène de l'une quelconque des molécules susmentionnées.

5. Protéine cytotoxique selon l'une quelconque des revendications 1 à 4, dans laquelle le polypeptide effecteur de shiga-toxine cytotoxique comprend une séquence d'acides aminés sélectionnée parmi :
i) les acides aminés 75 à 251 de la SÉQ. ID n° 1, de la SÉQ. ID n° 2, ou de la SÉQ. ID n° 3 ;
ii) les acides aminés 1 à 241 de la SÉQ. ID n° 1, de la SÉQ. ID n° 2, ou de la SÉQ. ID n° 3 ;
iii) les acides aminés 1 à 251 de la SÉQ. ID n° 1, de la SÉQ. ID n° 2, ou de la SÉQ. ID n° 3 ; et
iv) les acides aminés 1 à 261 de la SÉQ. ID n° 1, de la SÉQ. ID n° 2, ou de la SÉQ. ID n° 3.

6. Protéine cytotoxique selon la revendication 4, dans laquelle la région de liaison de type immunoglobuline comprend la séquence d'acides aminés 269-508 de la SÉQ. ID n° 4 ;
ou dans laquelle la région de liaison de type immunoglobuline comprend la séquence d'acides aminés 1-119 de la SÉQ. ID n° 5, par exemple la protéine cytotoxique comprenant la séquence polypeptidique de la SÉQ. ID n° 5.

7. Protéine cytotoxique selon l'une quelconque des revendications 1 à 6, dans laquelle le polypeptide effecteur de shiga-toxine cytotoxique comprend une mutation par rapport à une sous-unité A naturelle d'un membre de la famille des shiga-toxines qui modifie l'activité enzymatique du polypeptide effecteur de shiga-toxine tout en conservant une activité cytotoxique ; la mutation étant sélectionnée parmi au moins une délétion ou substitution de résidu acide aminé.

8. Protéine cytotoxique selon l'une quelconque des revendications 1 à 7, sous la forme d'un homomultimère ou d'un hétéromultimère.

9. Protéine cytotoxique selon l'une quelconque des revendications 1 à 8, sous la forme d'un sel ou d'un solvate pharmaceutiquement acceptable.

10. Protéine cytotoxique selon l'une quelconque des revendications 1 à 9, conjuguée à un agent diagnostique.

11. Composition pharmaceutique comprenant une protéine cytotoxique selon l'une quelconque des revendications 1 à 10 et au moins un excipient ou un support pharmaceutiquement acceptable.

12. Polynucléotide capable de coder une protéine cytotoxique selon l'une quelconque des revendications 1 à 7, ou complément de celui-ci.

13. Vecteur d'expression comprenant un polynucléotide selon la revendication 12.

14. Cellule hôte transformée comprenant un polynucléotide selon la revendication 12 ou un vecteur d'expression selon la revendication 13.

15. Procédé *in vitro* de destruction d'une cellule, le procédé comprenant l'étape de mise en contact de la cellule avec une protéine cytotoxique selon l'une quelconque des revendications 1 à 10.

16. Procédé *in vitro* de marquage de l'intérieur d'une cellule cancéreuse ou d'une cellule immunitaire, le procédé comprenant l'étape de mise en contact de la cellule avec une protéine cytotoxique selon la revendication 10.

17. Protéine cytotoxique selon l'une quelconque des revendications 1 à 10 ou composition pharmaceutique selon la revendication 11, destinée à une utilisation dans le traitement d'une maladie, d'une affection ou d'une pathologie chez un patient.

18. Protéine cytotoxique ou composition pharmaceutique destinée à une utilisation selon la revendication 17, le traitement étant le traitement d'une maladie, d'une affection ou d'une pathologie sélectionnée dans le groupe constitué d'un cancer, d'une tumeur, d'un trouble immunitaire, et d'une infection microbienne ;
le cancer étant par exemple sélectionné dans le groupe constitué d'un cancer des os, d'un cancer du sein, d'un cancer du système nerveux central/périphérique, d'un cancer gastro-intestinal, d'un cancer des cellules germinales, d'un cancer glandulaire, d'un cancer de la tête et du cou, d'un cancer hématologique, d'un cancer du rein/des voies urinaires, d'un cancer du foie, d'un cancer du poumon/pleural, d'un cancer de la prostate, d'un sarcome, d'un cancer de la peau et d'un cancer utérin ;
ou le trouble immunitaire étant associé à une maladie sélectionnée dans le groupe constitué de l'amyloïdose, de la spondylarthrite ankylosante, de l'asthme, de la maladie de Crohn, d'un diabète, d'un rejet de greffe, de la maladie du greffon contre l'hôte, de la thyroïdite de Hashimoto, du syndrome hémolytique et urémique, d'une maladie liée au VIH, du lupus érythémateux, de la sclérose en plaques, de la polyartérite, du psoriasis, de l'arthrite psoriasique, de la polyarthrite rhumatoïde, de la sclérodermie, du choc septique, du syndrome de Sjögren, de la colite ulcéreuse, et de la vascularite.
